Europäisches Patentamt

European Patent Office

Office européen des brevets

(19)

(11) EP 1 066 380 B1

(12) EUROPEAN PATENT SPECIFICATION

(45) Date of publication and mention
of the grant of the patent:
**14.11.2001 Bulletin 2001/46**

(51) Int Cl.[7]: **C12N 15/12**, C07K 14/725,
C07K 19/00, C07K 14/82

(21) Application number: **99922360.5**

(22) Date of filing: **19.05.1999**

(86) International application number:
**PCT/GB99/01588**

(87) International publication number:
**WO 99/60120 (25.11.1999 Gazette 1999/47)**

(54) **SOLUBLE T CELL RECEPTOR**

LÖSLICHER T-ZELL REZEPTOR

RECEPTEUR DE LYMPHOCYTE T SOLUBLE

(84) Designated Contracting States:
**AT BE CH CY DE DK ES FI FR GB GR IE IT LI LU
MC NL PT SE**
Designated Extension States:
**AL LT LV MK RO SI**

(30) Priority: **19.05.1998 GB 9810759
29.09.1998 GB 9821129**

(43) Date of publication of application:
**10.01.2001 Bulletin 2001/02**

(73) Proprietor: **Avidex Ltd
Abingdon, Oxon OX14 4RX (GB)**

(72) Inventors:
• **JAKOBSEN, Bent Karsten Avidex Limited
Oxon, OX14 4RX (GB)**
• **BELL, John, Irving MRC Human Immu. Unit
Oxford, Oxfordshire OX3 9DS (GB)**
• **GAO, George, Fu
Lab.of Molecular Medicine,H.Hughes
Boston, Ma 02115 (US)**
• **WILLCOX,Benjamin Ernest
WellcomeTrustTravelling
Pasadena, Ca 91125 (US)**
• **BOULTER, Jonathan Michael Avidex Limited,
Oxon, OX14 4RX (GB)**

(74) Representative: **Lee, Nicholas John et al
Kilburn & Strode,
20 Red Lion Street
London WC1R 4PJ (GB)**

(56) References cited:
WO-A-96/21028          WO-A-97/35991
WO-A-98/06749          US-A- 5 328 985

• CHANG H.-C. ET AL.: "A general method for
facilitating heterodimeric pairing between two
proteins: application to expression of alpha and
beta T-cell receptor extracellular segments"
PROCEEDINGS OF THE NATIONAL ACADEMY
OF SCIENCES OF USA, US, NATIONAL
ACADEMY OF SCIENCE, WASHINGTON, vol. 91,
no. 24, November 1994 (1994-11), pages
11408-11412, XP002008362 ISSN: 0027-8424
cited in the application
• DAVODEAU F. ET AL.: "Secretion of
disulfide-linked human T-cell receptor
gammadelta heterodimers" JOURNAL OF
BIOLOGICAL CHEMISTRY, US, AMERICAN
SOCIETY OF BIOLOGICAL CHEMISTS,
BALTIMORE, MD, vol. 268, no. 21, 25 July 1993
(1993-07-25), page 15455-15460 XP002008366
ISSN: 0021-9258
• GOLDEN A. ET AL.: "High-level production of a
secreted, heterodimeric alpha-beta murine
T-cell receptor in Escherichia coli" JOURNAL OF
IMMUNOLOGICAL METHODS, NL, ELSEVIER
SCIENCE PUBLISHERS B.V., AMSTERDAM, vol.
206, no. 1-2, 1997, pages 163-169, XP004093129
ISSN: 0022-1759 cited in the application

**(Cont. next page)**

- JAKOBSEN B.K. ET AL.: "Expression of soluble T-cell receptor -- leucine zipper fusions" IMMUNOTECHNOLOGY, NL, ELSEVIER SCIENCE PUBLISHERS BV, vol. 2, no. 4, November 1996 (1996-11), page 309 XP004063271 ISSN: 1380-2933
- PECORARI F. ET AL.: "Folding, heterodimeric association and specific peptide recognition of a murine alphabeta T-cell receptor expressed in Escherichia coli" JOURNAL OF MOLECULAR BIOLOGY, vol. 285, no. 4, 29 January 1999 (1999-01-29), pages 1831-1843, XP002123748
- KALANDADZE A. ET AL.: "Expression of recombinant HLA-DR2 molecules" JOURNAL OF BIOLOGICAL CHEMISTRY, US, AMERICAN SOCIETY OF BIOLOGICAL CHEMISTS, BALTIMORE, MD, vol. 271, no. 33, 16 August 1996 (1996-08-16), pages 20156-20162, XP002053288 ISSN: 0021-9258
- WÜLFING C. ET AL.: "Soluble T-cell receptor fragments - guidance of folding and assembly" THE IMMUNOLOGIST, CA, HOGREFE AND HUBER PUBLISHERS, TORONTO, vol. 3, no. 2, 1995, pages 59-66, XP000575900 ISSN: 1192-5612
- GARBOCZI D.N. ET AL.: "Assembly, specific binding, and crystallization of a human TCR-alphabeta with an antigenic Tax peptide from human T lymphotropic virus type 1 and the Class I MHC molecule HLA-A2" THE JOURNAL OF IMMUNOLOGY, vol. 157, 1996, pages 5403-5410, XP002123312 cited in the application
- HILYARD K.L. ET AL.: "Binding of soluble natural ligands to a soluble human T-cell receptor fragment produced in Escherichia coli" PROCEEDINGS OF THE NATIONAL ACADEMY OF SCIENCES OF USA, vol. 91, September 1994 (1994-09), pages 9057-9061, XP002123164 NATIONAL ACADEMY OF SCIENCE WASHINGTON, US ISSN: 0027-8424
- PALIWAL V. ET AL.: "Recombinant soluble alphabeta T cell receptors protect T cells from immune suppression: requirement for aggregated multimeric, disulfide-linked alphabeta heterodimers" THE JOURNAL OF IMMUNOLOGY, vol. 159, no. 4, 1997, pages 1718-1727, XP002093583 ISSN: 0022-1767
- FREMONT D.H. ET AL.: "Biophysical studies of T-cell receptors and their ligands" CURRENT OPINION IN IMMUNOLOGY, vol. 8, no. 1, February 1996 (1996-02), pages 93-100, XP002123165
- WARD E. SALLY: "Production and manipulation of antibodies and T-cell receptors using recombinant DNA technology" THERAPEUTIC IMMUNOLOGY. ED: AUSTEN KARL F. PUB: BLACKWELL, BOSTON, MA,1996, pages 335-346, XP000856686

**Description**

This invention relates to non-membrane bound recombinant T cell receptors (TCRs) and to methods and reagents for producing them.

**General background**

**1. Antigen presentation on the cell surface**

MHC molecules are specialised protein complexes which present short protein fragments, peptide antigens, for recognition on the cell surface by the cellular arm of the adaptive immune system.

Class I MHC is a dimeric protein complex consisting of a variable heavy chain and a constant light chain, β2microglobulin. Class I MHC presents peptides which are processed intracellularly, loaded into a binding cleft in the MHC, and transported to the cell surface where the complex is anchored in the membrane by the MHC heavy chain. Peptides are usually 8-11 amino acids in length, depending on the degree of arching introduced in the peptide when bound in the MHC. The binding cleft, which is formed by the membrane distal α1 and α2 domains of the MHC heavy chain, has "closed" ends, imposing quite tight restrictions on the length of peptide which can be bound.

Class II MHC is also a dimeric protein consisting of an α (heavy) and a β (light) chain, both of which are variable glycoproteins and anchored in the cell by transmembrane domains. Like Class I MHC, the Class II molecule forms a binding cleft in which longer peptides of 12-24 amino acids are inserted. Peptides are taken up from the extracellular environment by endocytosis and processed before loading into the Class II complex, which is then transported to the cell surface.

Each cell presents peptides in up to six different Class I molecules and a similar number of Class II molecules, the total number of MHC complexes presented being in the region $10^5$-$10^6$ per cell. The diversity of peptides presented in Class I molecules is typically estimated to be between 1,000-10,000, with 90% of these being present in 100-1,000 copies per cell (Hunt, Michel et al. 1992; Chicz, Urban et al. 1993; Engelhard, Appella et al. 1993; Huczko, Bodnar et al. 1993). The most abundant peptides are thought to constitute between 0.4-5% of the total peptide presented, which means that up to 20,000 identical complexes could be present. However, an average number for the most abundant single peptide complexes is likely to be in the region of 2,000-4,000 per cell, and typical presentation levels of recognisable T cell epitopes are in the region of 100-500 complexes per cell (for review see (Engelhard 1994)).

**2. Recognition of antigen presenting cells**

A wide spectrum of cells can present antigen, as MHC-peptide, and the cells that have that property are known as antigen presenting cells (APC). The type of cell which presents a particular antigen depends upon how and where the antigen first encounters cells of the immune system. APCs include the interdigitating dendritic cells found in the T cell areas of the lymph nodes and spleen in large numbers; Langerhans cells in the skin; follicular dendritic cells in B cell areas of the lymphoid tissue; monocytes, macrophages and other cells of the monocyte/macrophage lineage; B cells and T cells; and a variety of other cells such as endothelial cells and fibroblasts which are not classical APCs but can act in the manner of an APC.

APCs are recognised by a subgroup of lymphocytes which mature in the thymus (T cells), where they undergo a selection procedure designed to ensure that T cells which respond to self-peptides are eradicated (negative selection). In addition, T cells which do not have the ability to recognise the MHC variants which are presented (in man, the HLA haplotypes) fail to mature (positive selection).

Recognition of specific MHC-peptide complexes by T cells is mediated by the T cell receptor (TCR) which consists of an α- and a β-chain, both of which are anchored in the membrane. In a recombination process similar to that observed for antibody genes, the TCR α and β genes rearrange from Variable, Joining, Diversity and Constant elements creating enormous diversity in the extracellular antigen binding domains ($10^{13}$ to $10^{15}$ different possibilities). TCRs also exist in a different form with y and δ chains, but these are only present on about 5% of T cells.

Antibodies and TCRs are the only two types of molecules which recognise antigens in a specific manner, and thus the TCR is the only receptor for specific for particular peptide antigens presented in MHC, the alien peptide often being the only sign of an abnormality within a cell.

TCRs are expressed in enormous diversity, each TCR being specific for one or a few MHC-peptide complexes. Contacts between TCR and MHC-peptide ligands are extremely short-lived, usually with a half-life of less than a second. Adhesion between T cells and target cells, presumably TCR/MHC-peptide, relies on the employment of multiple TCR/MHC-peptide contacts as well as a number coreceptor-ligand contacts.

T cell recognition occurs when a T-cell and an antigen presenting cell (APC) are in direct physical contact and is initiated by ligation of antigen-specific TCRs with pMHC complexes. The TCR is a heterodimeric cell surface protein of the immunoglobulin superfamily which is associated with invariant proteins of the CD3 complex involved in mediating signal transduction. TCRs exist in αβ and γδ forms, which are structurally similar but have quite distinct anatomical locations and probably functions. The extracellular portion of the receptor consists of two membrane-proximal constant domains, and two membrane-distal variable domains bearing highly polymorphic loops analogous to the complementarity determining regions (CDRs) of antibodies. It is these loops which form the MHC-binding site of the TCR molecule and determine peptide specificity. The MHC class I and class II ligands are also immunoglobulin superfamily proteins but are specialised for antigen presentation, with a highly polymorphic peptide binding site which enables them to present a diverse array of short peptide fragments at the APC cell surface.

Recently, examples of these interactions have been characterised structurally (Garboczi, Ghosh et al. 1996; Garcia, Degano et al. 1996; Ding, Smith et al. 1998). Crystallographic structures of murine and human Class I pMHC-TCR complexes indicate a diagonal orientation of the TCR over its pMHC ligand and show poor shape complementarity in the interface. CDR3 loops contact exclusively peptide residues. Comparisons of liganded and unliganded TCR structures also suggest that there is a degree of flexibility in the TCR CDR loops (Garboczi and Biddison 1999).

T cell activation models attempt to explain how such protein-protein interactions at an interface between T cell and antigen presenting cell (APC) initiate responses such as killing of a virally infected target cell. The physical properties of TCR-pMHC interactions are included as critical parameters in many of these models. For instance, quantitative changes in TCR dissociation rates have been found to translate into qualitative differences in the biological outcome of receptor engagement, such as full or partial T cell activation, or antagonism (Matsui, Boniface et al. 1994; Rabinowitz, Beeson et al. 1996; Davis, Boniface et al. 1998).

TCR-pMHC interactions have been shown to have low affinities and relatively slow kinetics. Many studies have used biosensor technology, such as Biacore™ (Willcox, Gao et al. 1999; Wyer, Willcox et al. 1999), which exploits surface plasmon resonance (SPR) and enables direct affinity and real-time kinetic measurements of protein-protein interactions (Garcia, Scott et al. 1996; Davis, Boniface et al. 1998). However, the receptors studied are either alloreactive TCRs or those which have been raised in response to an artificial immunogen.

### 3. TCR and CD8 interactions with MHC-peptide complexes

The vast majority of T cells restricted by Class I MHC-peptide complexes also require the engagement of the coreceptor CD8 for activation, while T cells restricted by Class II MHC require the engagement of CD4. The exact function of the coreceptors in T cell activation is not yet entirely clarified. Neither are the critical mechanisms and parameters controlling activation. However, both CD8 and CD4 have cytoplasmic domains which are associated with the kinase p56[lck] which is involved in the very earliest tyrosine phosphorylation event which characterises T cell activation. CD8 is a dimeric receptor, expressed either in an αα form or, more commonly, in an αβ form. CD4 is a monomer. In the CD8 receptor, only the α-chain is associated with p56[lck].

Recent determinations of the physical parameters controlling binding of TCR and CD8 to MHC, using soluble versions of the receptors, has shown that binding by TCR dominates the recognition event. TCR has significantly higher affinity for MHC than the coreceptors (Willcox, Gao et al. 1999; Wyer, Willcox et al. 1999).

The individual interactions of the receptors with MHC are very short-lived at physiological temperature, i.e. 37°C. An approximate figure for the half-life of a TCR-MHC/peptide interaction, measured with a human TCR specific for the influenza virus "matrix" peptide presented by HLA-A*0201 (HLA-A2), is 0.7 seconds. The half-life of the CD8αα interaction with this MHC/peptide complex is less than 0.01 seconds, i.e. at least 18 times faster.

### 4. Production of soluble MHC-peptide complexes

Soluble MHC-peptide complexes were first obtained by cleaving the molecules of the surface of antigen presenting cells with papain (Bjorkman, Strominger et al. 1985). Although this approach provided material for crystallisation, it has, for Class I molecules, in recent years been replaced by individual expression of heavy and light chain in *E.coli* followed by refolding in the presence of synthetic peptide (Garboczi, Hung et al. 1992; Madden, Garboczi et al. 1993; Garboczi, Madden et al. 1994; Reid, McAdam et al. 1996; Reid, Smith et al. 1996; Smith, Reid et al. 1996; Smith, Reid et al. 1996; Gao, Tormo et al. 1997; Gao, Gerth et al. 1998). This approach has several advantages over previous methods in that a better yield is obtained at a lower cost, peptide identity can be controlled very accurately, and the final product is more homogeneous. Furthermore, expression of modified heavy or light chain, for instance fused to a protein tag, can be easily performed.

### 5. Soluble TCR

At present, there are no published data on human TCR-pMHC interactions, and no studies artalysing naturally selected TCRs specific for natural (e.g. viral) epitopes. This may reflect difficulties in obtaining protein which is suitable for SPR i.e. protein which is homogenous, monomeric, correctly folded, available in milligram quantities and stable over a range of concentration.

It would be an advantage to be able to produce a recombinant TCR in non-membrane bound (or soluble) form, not only for the purpose of investigating specific TCR-pMHC interactions, but also as a diagnostic tool to detect infection or to detect autoimmune disease markers, or to detect the efficacy of T cell vaccines. Soluble TCR would also have applications in staining, for example to stain cells for the presence of a particular viral antigen presented in the context of the MHC. Similarly, the soluble TCR could be used to deliver a therapeutic agent, for example a cytotoxic compound or an immunostimulating compound, to cells presenting a particular antigen.

Proteins which are made up of more than one polypeptide subunit and which have a transmembrane domain can be difficult to produce in soluble form because in many cases the protein is stabilised by its transmembrane region. This is the case for the TCR.

Production of soluble TCR has only recently been described by a number of groups. In general, all methods describe truncated forms of TCR, containing either only extracellular domains or extracellular and cytoplasmic domains. Thus, in all cases, the transmembrane domains have been deleted from the expressed protein. Although many reports show that TCR produced according to their methods can be recognised by TCR-specific antibodies (indicating that the part of the recombinant TCR recognised by the antibody has correctly folded), none has been able to produce a soluble TCR at a good yield which is stable at low concentrations and which can recognise MHC-peptide complexes

The first approach to yield crystallisable material made use of expression in eukaryotic cells but the material is extremely expensive to produce (Garcia, Degano et al. 1996; Garcia, Scott et al. 1996). Another approach which has produced crystallisable material made use of an *E.coli* expression system similar to what has previously been used for MHC-peptide complexes (Garboczi, Ghosh et al. 1996; Garboczi, Utz et al. 1996). The latter method, which expresses the extracellular portions of the TCR chains truncated immediately before the cysteine residues involved in forming the interchain disulphide bridge, followed by refolding *in vitro*, has turned out not to be generally applicable. Most heterodimeric TCRs appear to be unstable when produced in this fashion due to low affinity between the $\alpha$ and $\beta$ chains.

In addition, a number of other descriptions of engineered production of soluble TCR exist Some of these describe only the expression of either the $\alpha$ or $\beta$ chain of the TCR, thus yielding protein which does not retain MHC-peptide specific binding (Calaman, Carson et al. 1993; Ishii, Nakano et al. 1995). $\beta$ chain crystals have been obtained without $\alpha$ chain, either alone or bound to superantigen (Boulot, Bentley et al. 1994; Bentley, Boulot et al. 1995; Fields, Malchiodi et al. 1996).

Other reports describe methods for expression of heterodimeric $\gamma/\delta$ or $\alpha/\beta$ TCR (Gregoire, Rebai et al. 1991; Necker, Rebai et al. 1991; Eilat, Kikuchi et al. 1992; Weber, Traunecker et al. 1992; Corr, Slanetz et al. 1994; Ishii, Nakano et al. 1995; Gregoire, Malissen et al. 1996; Romagne, Peyrat et al. 1996). In some cases, the TCR has been expressed as a single chain fusion protein (Brocker, Peter et al. 1993; Gregoire, Malissen et al. 1996; Schlueter, Schodin et al. 1996). Another strategy has been to express the TCR chains as chimeric proteins fused to lg hinge and constant domains (Eilat, Kikuchi et al. 1992; Weber, Traunecker et al. 1992). Other chimaeric TCR proteins have been expressed with designed sequences which form coiled-coils which have high affinity and specificity for each other, thus stabilising TCR $\alpha$-$\beta$ contacts and increasing solubility. This approach was taken by Chang, Bao et al. (1994) who replaced the transmembrane region of the protein with a leucine zipper protein consisting of two synthetic peptide sequences, an acid peptide and a base peptide, that specifically interact to create a heterodimeric coiled coil. The authors employed a bacculovirus expression system in eukaryotic cells to secrete heterodimeric TCR protein. The artificial leucine zipper peptides assist heterodimerisation of the TCR $\alpha$ and $\beta$ chains, which are also linked by an interchain disulphide bond just above the fusion point with the zipper peptides. However, these techniques have not since proved successful and there is no evidence that the soluble TCR described can recognise a TCR ligand. Similarly, Golden, Khandekar *et al* (1997) described the production of heterodimeric T cell receptors as leucine zipper fusion proteins. The soluble TCR was expressed in *E. coli* as a secreted heterodimer with the $\alpha$-$\beta$ interchain disulphide bond as in Chang *et al.* Again, there is no evidence that this ready-folded TCR heterodimer is capable of interacting with its ligand.

There is therefore a need for a soluble version of the membrane bound TCR, which is correctly folded so that it is capable of recognising its native ligand. A soluble form of a TCR which is stable over a period of time, and a method for producing it in reasonable quantities, would also be useful. The present invention aims to meet some or all of these requirements.

**The Invention**

The invention provides in one aspect a refolded recombinant T cell receptor (TCR) which comprises:

    i) a recombinant TCR $\alpha$ or $\gamma$ chain extracellular domain having a first heterologous C-terminal dimerisation peptide; and

ii) a recombinant TCR β or δ chain extracellular domain having a second C-terminal dimerisation peptide which is specifically heterodimerised with the first dimerisation peptide to form a heterodimerisation domain, wherein a disulphide bond present in native TCRs between the α and β or γ and δ chains adjacent to the cytoplasmic domain is absent.

The TCR according to the invention, which is refolded after being expressed, rather than secreted as a heterodimer, is conformationally superior to soluble TCR previously available. This is indicated by its ability to recognise MHC-peptide complexes. It is also stable at relatively low concentrations. It may be stable at concentrations below 1 mg/ml and preferably about 10 μg/ml.

In a further aspect, the invention provides a biologically-active recombinant T cell receptor (TCR) which comprises:

i) a recombinant TCR α or γ chain extracellular domain having a first heterologous C-terminal dimerisation peptide; and

ii) a recombinant TCR β or δ chain extracellular domain having a second C-terminal dimerisation peptide which is specifically heterodimerised with the first dimerisation peptide to form a heterodimerisation domain, wherein a disulphide bond present in native TCRs between the α and β or γ and δ chains adjacent to the cytoplasmic domain is absent. Such a TCR will bind specifically to MHC-peptide complexes, preferably in a manner similar to the native TCR from which it is derived.

In another aspect, the invention provides recombinant nucleic acid sequences encoding the recombinant TCR chains as described herein. Such nucleic acid sequences may be isolated from T-cell clones. Alternatively, they may be produced synthetically, for example by inserting a heterologous nucleic acid sequence in a nucleic acid sequence encoding a TCR chain, or by mutating (by insertion, deletion or substitution) a nucleic acid sequence encoding a TCR chain. Thus, the invention includes within its scope synthetic peptides which have the activity and/or binding specificity of native TCRs.

In yet another aspect, the invention provides a method of making a recombinant non membrane bound T cell receptor, which method comprises:

expressing (i) a recombinant TCR α or γ chain extracellular domain having a first heterologous C-terminal dimerisation peptide, and (ii) a recombinant TCR β or δ chain extracellular domain having a second C-terminal dimerisation peptide which specifically heterodimerises with the first dimerisation peptide to form a heterodimerisation domain; and

refolding the chains together *in vitro* to produce a TCR heterodimer.

Recombinant TCRs in accordance with the invention may be for recognising Class I MHC-peptide complexes and Class II MHC-peptide complexes.

The heterodimerisation domain of the recombinant TCR according to the invention is preferably a so-called "coiled coil" or "leucine zipper". These terms are used to describe pairs of helical peptides which interact with each other in a specific fashion to form a heterodimer. The interaction occurs because there are complementary hydrophobic residues along one side of each zipper peptide. The nature of the peptides is such that the formation of heterodimers is very much more favourable than the formation of homodimers of the helices. Leucine zippers may be synthetic or naturally occurring. Synthetic leucines can be designed to have a much higher binding affinity than naturally occurring leucine zippers, which is not necessarily an advantage. In fact, preferred leucine zippers for use in the invention are naturally occurring leucine zippers or leucine zippers with a similar binding affinity. Leucine zippers from the c-jun and c-fos protein are an example of leucine zippers with a suitable binding affinity. Other suitable leucine zippers include those from the myc and max proteins (Amati, Dalton, et al 1992). Other leucine zippers with suitable properties could easily be designed (O'Shea *et al* 1993).

It is preferred that the soluble TCRs in accordance with the invention have approximately 40 amino acid leucine zipper fusions corresponding to the heterodimerisation domains from c-jun (αchain) and c-fos (βchain) (O'Shea, Rutkowski et al 1989, O'Shea, Rutkowski et al, 1992, Glover and Harrison, 1995). Longer leucine zippers may be used. Since heterodimerisation specificity appears to be retained even in quite short fragments of some leucine zipper domains (O'Shea, Rutkowski et al, 1992), it is possible that a similar benefit could be obtained with shorter c-jun and c-fos fragments. Such shorter fragments could have as few as 8 amino acids for example. Thus, the leucine zipper domains may be in the range of 8 to 60 amino acids long.

The molecular principles of specificity in leucine zipper pairing is well characterised (Landschulz, Johnson et al, 1988; McKnight, 1991) and leucine zippers can be designed and engineered by those skilled in the art to form homodimers, heterodimers or trimeric complexes (Lumb and Kim, 1995; Nautiyal, Woolfson et al, 1995; Boice, Dieckmann et al, 1996, Chao, Houston et al, 1996). Designed leucine zippers, or other heterodimerisation domains, of higher affinity than the c-jun and c-fos leucine zippers may be beneficial for the expression of soluble TCRs in some systems. However, as mentioned in more detail below, when soluble TCR is folded *in vitro*, a solubilising agent is preferably included in the folding buffer to reduce the formation of unproductive protein aggregates. One interpretation of this phenomenon is that the kinetics of folding of the leucine zipper domains are faster than for the TCR chains, leading to dimerisation of unfolded TCR $\alpha$ and $\beta$ chain, in turn causing protein aggregation. By slowing the folding process and inhibiting aggregation by inclusion of solubilising agent, the protein can be maintained in solution until folding of both fusion domains is completed. Therefore, heterodimerisation domains of higher affinity than the c-fos and c-jun leucine zippers may require higher concentrations of solubilising agent to achieve a yield of soluble TCRs comparable to that for c-jun and c-fos.

Different biological systems use a variety of methods to form stable homo- and hetero protein dimers, and each of these methods in principle provide an option for engineering dimerisation domains into genetically modified proteins. Leucine zippers (Kouzarides and Ziff 1989) are probably the most popular dimerisation modules and have been widely used for production of genetically designed dimeric proteins. Thus, the leucine zipper of GCN4, a transcriptional activator protein from the yeast *Saccharomyces cerevisiae*, has been used to direct homodimerisation of a number of heterologous proteins (Hu, Newell et al. 1993; Greenfield, Montelione et al. 1998). The preferred strategy is to use zippers that direct formation of heterodimeric complexes such as the Jun/Fos leucine zipper pair (de Kruif and Logtenberg 1996; Riley, Ralston et al. 1996).

The heterodimerisation domain of the recombinant TCR according to the present invention is not limited to leucine zippers. Thus, it may be provided by disulphide bridge-forming elements. Alternatively, it may be provided by the SH3 domains and hydrophobic/proline rich counterdomains, which are responsible for the protein-protein interactions seen among proteins involved in signal transduction (reviewed by Schlessinger, (Schlessinger 1994). Other natural protein-protein interactions found among proteins participating in signal transduction cascades rely on associations between post-translationally modified amino acids and protein modules that specifically recognise such modified residues. Such post-translationally modified amino acids and protein modules may form the heterodimerisation domain of the recombinant TCR in accordance with the invention. An example of a protein pair of this type is provided by tyrosine phosphorylated receptors such as Epidermal Growth Factor Receptor or Platelet Derived Growth Factor Receptor and the SH2 domain of GRB2 (Lowenstein, Daly et al. 1992; Buday and Downward 1993). As in all fields of science, new dimerisation modules are being actively sought (Chevray and Nathans 1992) and methods for engineering completely artificial modules have now successfully been developed (Zhang, Murphy et al. 1999).

In the recombinant TCR according to the invention, an interchain disulphide bond which forms between two cysteine residues in the native $\alpha$ and $\beta$ TCR chains and between the native $\gamma$ and $\delta$ TCR chains, is absent. This may be achieved for example by fusing the dimerisation domains to the TCR receptor chains above the cysteine residues so that these residues are excluded from the recombinant protein. In an alternative example, one or more of the cysteine residues is replaced by another amino acid residue which is not involved in disulphide bond formation. These cysteine residues may not be incorporated because they may be detrimental to *in vitro* folding of functional TCR.

Refolding of the $\alpha$ and $\beta$ chains or $\gamma$ and $\delta$ chains of the refolded recombinant TCR according to the invention takes place *in vitro* under suitable refolding conditions. In a particular embodiment, a recombinant TCR with correct conformation is achieved by refolding solubilised TCR chains in a refolding buffer comprising a solubilising agent, for example urea. Advantageously, the urea may be present at a concentration of at least 0.1M or at least 1M or at least 2.5M, or about 5M. An alternative solubilising agent which may be used is guanidine, at a concentration of between 0.1M and 8M, preferably at least 1M or at least 2.5M. Prior to refolding, a reducing agent is preferably employed to ensure complete reduction of cysteine residues. Further denaturing agents such as DTT and guanidine may be used as necessary. Different denaturants and reducing agents may be used prior to the refolding step (e.g. urea, $\beta$-mercaptoethanol). Alternative redox couples may be used during refolding, such as a cystaminelcysteamine redox couple, DTT or $\beta$-mercaptoethanol/atmospheric oxygen, and cysteine in reduced and oxidised forms.

The monomeric TCR described herein may be labelled with a detectable label, for example a label which is suitable for diagnostic purposes. Thus, the invention provides a method for detecting MHC-peptide complexes which method comprises contacting the MHC-peptide complexes with a TCR in accordance with the invention which is specific for the MHC-peptide complex; and detecting binding of the TCR to the MHC-peptide complex. In tetrameric TCR formed using biotinylated heterodimers, fluorescent streptavidin (commercially available) can be used to provide a detectable label. A fluorescently labelled tetramer will be suitable for use in FACS analysis, for example to detect antigen presenting cells carrying the peptide for which the TCR is specific.

Another manner in which the soluble TCRs may be detected is by the use of TCR-specific antibodies, in particular monoclonal antibodies. There are many commercially available anti-TCR antibodies, such as $\beta$FI and $\alpha$FI, which recognise the constant regions of the $\beta$ and $\alpha$ chain, respectively.

The TCR may alternatively or additionally be linked to a therapeutic agent which may be for example a toxic moiety for example for use in cell killing, or an immunostimulating agent such as an interleukin or a cytokine. Thus, the invention provides a method for delivering a therapeutic agent to a target cell, which method comprises contacting potential target cells with a TCR in accordance with the invention under conditions to allow attachment of the TCR to the target cell, said TCR being specific for the MHC-peptide complexes and having the therapeutic agent associated therewith. In particular, the soluble TCR could be used to deliver therapeutic agents to the location of cells presenting a particular antigen. For instance, a toxin could be delivered to a tumour thereby helping to eradicate it. This would be useful in many situations and in particular against tumours because not all cells in the tumour present antigens and therefore not all tumour cells are detected by the immune system. With the soluble TCR, a compound could be delivered such that it would exercise its effect locally but not only on the cell it binds to. Thus, one particular strategy envisages anti-tumour molecules linked to T cell receptors specific for tumour antigens.

Many toxins could be employed for this use, for instance radioactive compounds, enzymes (perforin for example) or chemotherapeutic agents (cis-platin for example). To ensure that toxic effects are exercised in the desired location the toxin could be inside a liposome linked to streptavidin so that the compound is released slowly. This will prevent damaging effects during the transport in the body and ensure that the toxin has maximum effect after binding of the TCR to the relevant antigen presenting cells.

An alternative way to label or attach another moiety such as a toxic moiety to the soluble TCR is by including the label or other moiety in a mixed molecule multimer. An example of such a multimeric molecule is a tetramer containing three TCR molecules and one peroxidase molecule. This could be achieved by mixing the TCR and the enzyme at a molar ratio of 3:1 to generate tetrameric complexes and isolating the desired complex from any complexes not containing the correct ratio of molecules. Mixed molecules could contain any combination of molecules, provided that steric hindrance does not compromise or does not significantly compromise the desired function of the molecules. The positioning of the binding sites on the streptavidin molecule is suitable for mixed tetramers since steric hindrance is not likely to occur.

Preferably, the recombinant TCR chains according to the invention have a flexible linker located between the TCR domain and the dimerisation peptide. Suitable flexible linkers include standard peptide linkers containing glycine, for example linkers containing glycine and serine. C-terminal truncations close to the cysteine residues forming the interchain disulphide bond are believed to be advantageous because the α and β chains are in dose proximity through these residues in cellular TCRs. Therefore only relatively short linker sequences may be required to supply a nondistortive transition from the TCR chains to the heterodimerisation domain. It is preferred that the linker sequences Pro-Gly-Gly or Gly-Gly are used. However, the linker sequence could be varied. For instance, the linker could be omitted completely, or reduced to a single residue, the preferred choice in this case being a single Glycine residue. Longer linkers variations are also likely to be tolerated in the soluble TCR, provided that they could be protected from protease attack which would lead to segregation of the dimerisation peptides from the extracellular domains of the TCR with ensuing loss of α-β chain stability.

The soluble TCR according to the invention is not necessarily α-βTCR. Molecules such as γ-δ, α-δ and γ-βTCR molecules, as well as TCR molecules (pre-TCR) containing invariant alpha chains which are only expressed early in development are also included. Pre-TCR specifies the cell lineage which will express α-β T cell receptor, as opposed to those cells which will express γ-δ T cell receptor (for reviews, see (Aifantis, Azogui et al. 1998; von Boehmer, Aifantis et al. 1998; Wurch, Biro et al. 1998)). The Pre-TCR is expressed with the TCR β chain pairing with an invariant Pre-TCR α chain (Saint Ruf, Ungewiss et al. 1994; Wilson and MacDonald 1995) which appears to commit the cell to the α-β T cell lineage. The role of the Pre-TCR is therefore thought to be important during thymus development (Ramiro, Trigueros et al. 1996).

Standard modifications to the recombinant proteins according to the invention may be made as appropriate. These include for example altering an unpaired cysteine residue in the constant region of the β chain to avoid incorrect intrachain or interchain pairing.

The signal peptide may be omitted since it does not serve any purpose in the mature receptor or for its ligand binding ability, and may in fact prevent the TCR from being able to recognise ligand. In most cases, the cleavage site at which the signal peptide is removed from the mature TCR chains is predicted but not experimentally determined. Engineering the expressed TCR chains such that they are a few, i.e. up to about 10 for example, amino acids longer or shorter at the n-terminal end will have no significance for the functionality of the soluble TCR. Certain additions which are not present in the original protein sequence could be added. For example, a short tag sequence which can aid in purification of the TCR chains could be added provided that it does not interfere with the correct structure and folding of the antigen binding site of the TCR.

For expression in *E.coli*, a methionine residue may be engineered onto the N-terminal starting point of the predicted mature protein sequence in order to enable initiation of translation.

Far from all residues in the variable domains of TCR chains are essential for antigen specificity and functionality. Thus, a significant number of mutations can be introduced in this region without affecting antigen specificity and functionality.

By contrast, certain residues involved in forming contacts to the peptide antigen or the HLA heavy chain polypeptide, i.e. the residues constituting the CDR regions of the TCR chains, may be substituted for residues that would enhance the affinity of the TCR for the ligand. Such substitutions, given the low affinity of most TCRs for peptide-MHC ligands, could be useful for enhancing the specificity and functional potential of soluble TCRs. In the examples herein, the affinities of soluble TCRs for peptide-MHC ligands are determined. Such measurements can be used to assay the effects of mutations introduced in the TCR and thus also for the identification of TCRs containing substitutions which enhance the activity of the TCR.

Far from all residues in the constant domains of TCR chains are essential for antigen specificity and functionality. Thus, a significant number of mutations can be introduced in this region affecting antigen specificity. In Example 14 below, we have shown that two amino acid substitutions in the constant domain of a TCR β chain had no detectable consequences for the ability of the TCR to bind a HLA-peptide ligand.

The TCR β chain contains a cysteine residue which is unpaired in the cellular or native TCR. Mutation of this residue enhances the efficiency of *in vitro* refolding of soluble TCR. Substitutions of this cysteine residue for serine or alanine has a significant positive effect on refolding efficiencies *in vitro*. Similar positive effects, or even better effects, may be obtained with substitutions for other amino acids.

Because the constant domains are not directly involved in contacts with the peptide-MHC ligands, the C-terminal truncation point may be altered substantially without loss of functionality. For instance, it should be possible to produce functional soluble TCRs excluding the entire constant domain. In principle, it would be simpler to express and fold soluble TCRs comprising only the variable regions or the variable regions and only a short fragment of the constant regions, because the polypeptides would be shorter. However, this strategy is not preferred. This is because the provision of additional stability of the α-β chain pairing through a heterodimerisation domain would be complicated because the engineered C-termini of the two chains would be some distance apart, necessitating long linker sequences. The advantage of fusing heterodimerisation domains just prior to the position of the cysteines forming the interchain disulphide bond, as is preferred, is that the α and β chains are held in close proximity in the cellular receptor. Therefore, fusion at this point is less likely to impose distortion on the TCR structure.

It is possible that functional soluble TCR could be produced with a larger fragment of the constant domains present than is preferred herein, i.e. they constant domains need not be truncated just prior to the cysteines forming the interchain disulphide bond. For instance, the entire constant domain except the transmembrane domain could be included. It would be advantageous in this case to mutate the cysteine residues forming the interchain disulphide bond in the cellular TCR.

In addition to aiding interchain stability through a heterodimerisation domain, incorporation of cysteine residues which could form an interchain disulphide bond could be used. Cysteine residues could be engineered in as substitutions at amino acid positions where the folding of the two chains would bring the residues in close proximity, suitable for disulphide bond formation.

Purification of the TCR may be achieved by many different means. Alternative modes of ion exchange may be employed or other modes of protein purification may be used such as gel filtration chromatography or affinity chromatography.

In the method of producing a recombinant TCR in accordance with the invention, folding efficiency may also be increased by the addition of certain other protein components, for example chaperone proteins, to the refolding mixture. Improved refolding has been achieved by passing protein through columns with immobilised mini-chaperones (Altamirano, Golbik et al. 1997; Altamirano, Garcia et al. 1999).

In addition to the methods described in the examples, alternative means of biotinylating the TCR may be possible. For example, chemical biotinylation may be used. Alternative biotinylation tags may be used, although certain amino acids in the biotin tag sequence are essential (Schatz et al, 1993). The mixture used for biotinylation may also be varied. The enzyme requires Mg-ATP and low ionic strength although both of these conditions may be varied e.g. it may be possible to use a higher ionic strength and a longer reaction time. It may be possible to use a molecule other than avidin or streptavidin to form multimers of the TCR. Any molecule which binds biotin in a multivalent manner would be suitable. Alternatively, an entirely different linkage could be devised (such as poly-histidine tag to chelated nickel ion (Quiagen Product Guide 1999, Chapter 3 "Protein Expression, Purification, Detection and Assay" p. 35-37). Preferably, the tag is located towards the C-terminus of the protein so as to minimise the amount of steric hindrance in the interaction with potential peptide-MHC complexes.

Examples of suitable MHC-peptide targets for the TCR according to the invention include, but are not limited to, viral epitopes such as HTLV-1 epitopes (e.g. the Tax peptide restricted by HLA-A2; HTLV-1 is associated with leukaemia), HIV epitopes, EBV epitopes, CMV epitopes; melanoma epitopes and other cancer-specific epitopes; and epitopes associated with autoimmune disorders, such as rheumatoid arthritis.

A multitude of disease treatments can potentially be enhanced by localising the drug through the specificity of soluble TCRs.

Viral diseases for which drugs exist, e.g. HIV, SIV, EBV, CMV, would benefit from the drug being released in the near vicinity of infected cells. For cancer, the localisation in the vicinity of tumours or metastasis would enhance the effect of toxins or immunostimulants. In autoimmune diseases, immunosuppressive drugs could be released slowly, having more local effect over a longer time-span while minimally affecting the overall immuno-capacity of the subject. In the prevention of graft rejection, the effect of immunosuppressive drugs could be optimised in the same way. For vaccine delivery, the vaccine antigen could be localised in the vicinity of antigen presenting cells, thus enhancing the efficacy of the antigen. The method can also be applied for imaging purposes.

Preferred features of each aspect of the invention are as for each of the other aspects *mutatis mutandis*. The prior art documents mentioned herein are incorporated to the fullest extent permitted by law.

The invention is further described in the following examples, which do not limit the scope of the invention in any way.

Reference is made in the following to the accompanying drawings in which:

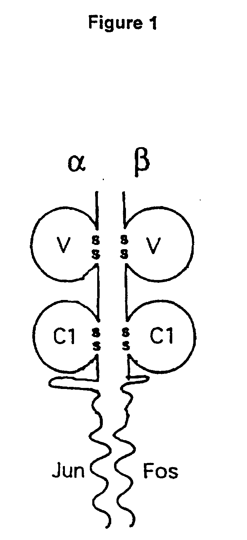

Figure 1 is a schematic view of a T-cell Receptor-leucine zipper fusion protein. Each chain consists of two immunoglobulin superfamily domains, one variable (V) and one constant (C). The constant domains are truncated immediately n-terminal of the interchain cysteine residues, and fused to a leucine zipper heterodimerisation motif from c-Jun ($\alpha$) or c-Fos ($\beta$) of around 40 amino acids at the C-terminal via a short linker. The $\alpha$-Jun and $\beta$-Fos each contain two intrachain disulphide bonds and pair solely by non-covalent contacts. The alpha chain is shorter than the beta chain due to a smaller constant domain.

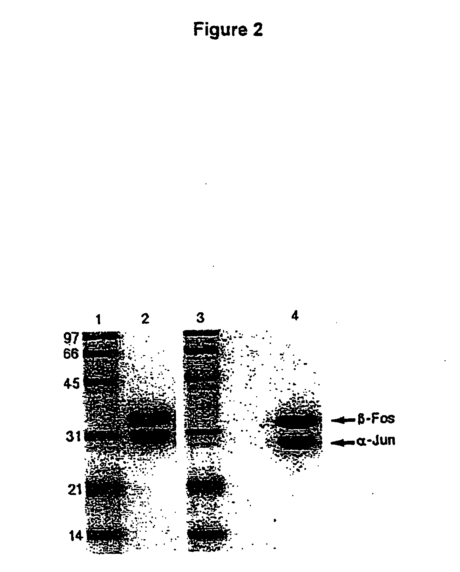

Figure 2 is a photograph of a reducing/non-reducing gel analysis of heterodimeric JM22zip receptor. Identical samples of purified TCR-zipper were loaded onto a 15% acrylamide SDS gel, either under reducing conditions (lane 2) and non-reducing conditions (lane 4). Marker proteins are shown in lanes 1 and 3. Molecular weights are shown in kilodaltons. Under both sets of conditions, the non-covalently associated heterodimer is dissociated into alpha and beta chains. In lane 4, each chain runs with a higher mobility and as a single band, indicating a single species of intrachain disulphide bonding is present. This is compatible with correct disulphide bond formation.

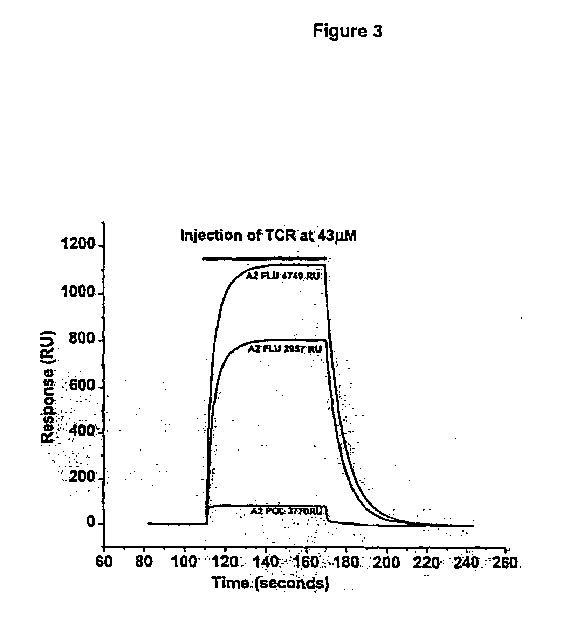

Figure 3 is a graph showing the specific binding of JM22zip TCR to HLA-A2 Flu matrix (M58-66) complexes. HLA-A2 complexes, refolded around single peptides and biotinylated on $\beta$2-microglobulin have been immobilised onto three streptavidin-coated flow cells: 3770 Resonance Units (RU) of HLA-A2 POL control onto flow cell (FC) 3, and two different levels of HLA-A2 M58-66 FLU (2970 RU on FC1 and 4960 RU on FC2). JM22zip has been injected in the soluble phase sequentially over all three flow cells at a concentration of 43 $\mu$M for 60 seconds. During the injection, an above-background increase in the response of both HLA-A2 FLU-coated flow cells is seen, with approximately 1000 RU and 700 RU of specific binding of JM22zip to flow cells 1 and 2 respectively.

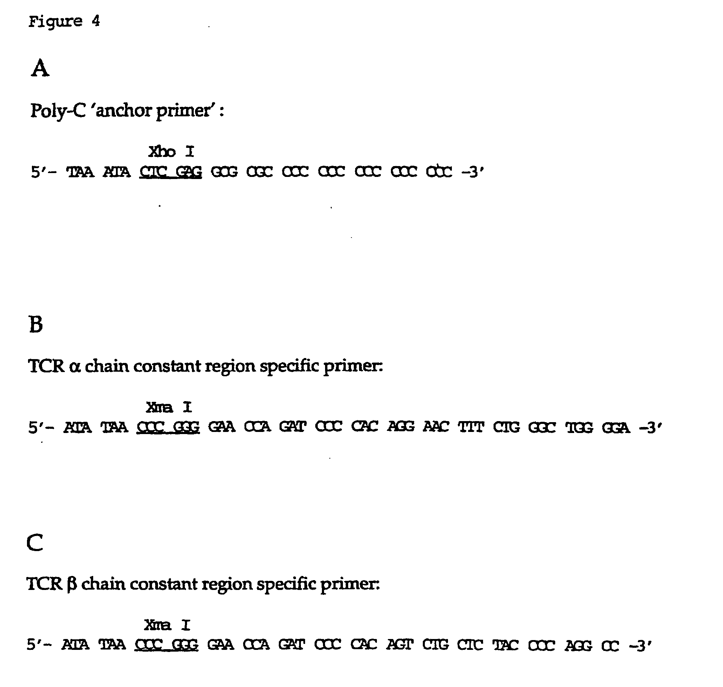

Figure 4 shows the sequences of synthetic DNA primers used for "anchor amplification of TCR genes. Recognition sites for DNA restriction enzymes used for cloning are underlined. A: poly-C "anchor primer". B: TCR $\alpha$ chain constant region specific primer. C: TCR $\beta$ chain constant region specific primer.

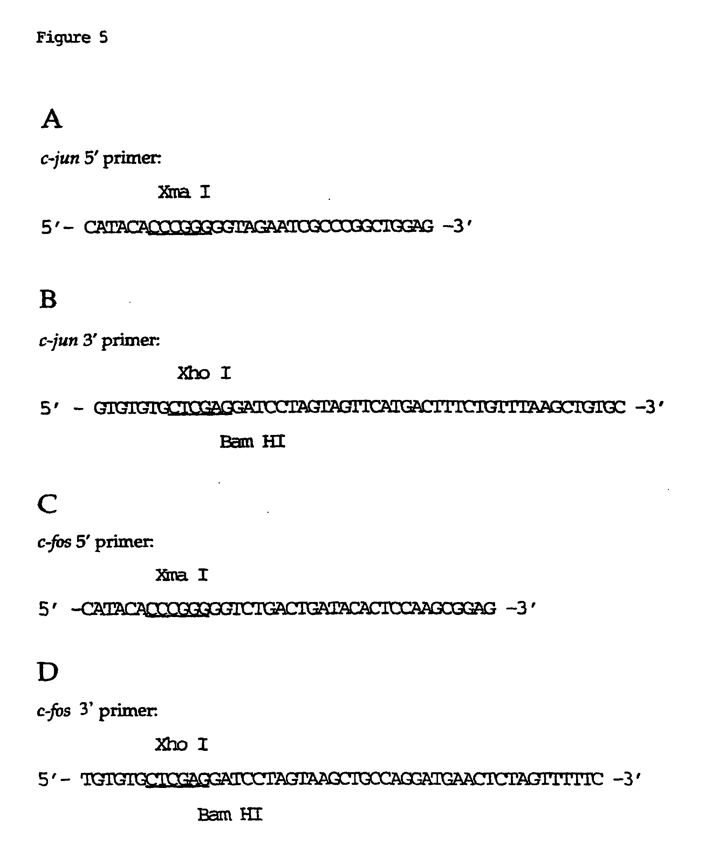

Figure 5 shows the sequences of synthetic DNA primers used for PCR amplification of DNA fragments encoding the 40 amino acid coiled-coil ("leucine zipper") regions of c-jun and c-fos. Recognition sites for DNA restriction enzymes used for cloning are underlined. A: *c-jun* 5' primer. B: *c-jun* 3'primer. C: *c-fos* 5' primer. D: *c-fos* 3' primer.

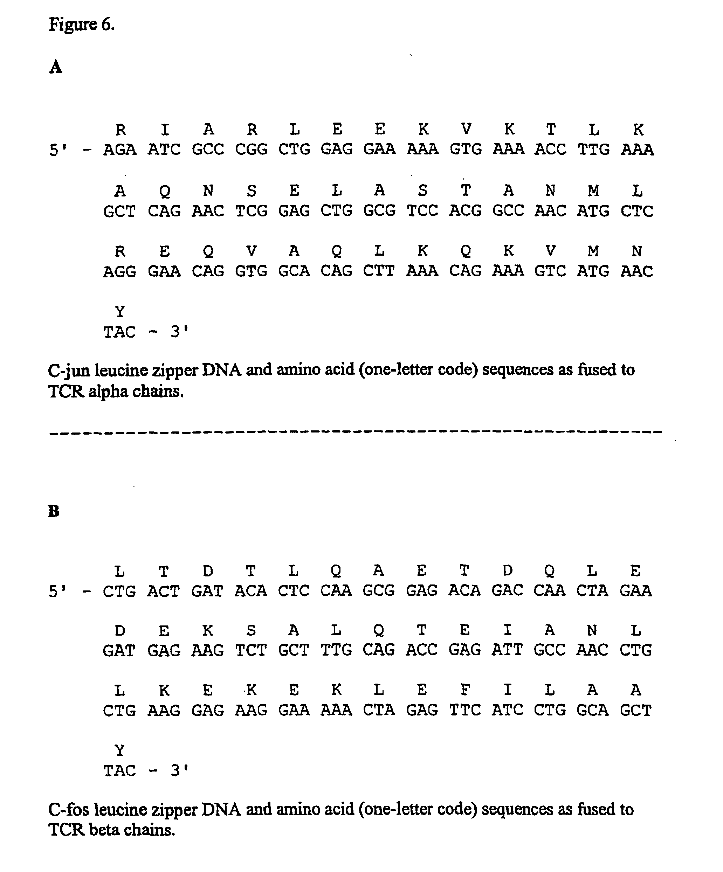

Figure 6 shows the respective DNA and amino acid (one letter code) sequences of c-fos and c-jun fragments as fused to TCRs (inserts in pBJ107 and pBJ108). A: c-jun leucine zipper as fused to TCR $\alpha$ chains. B: c-fos leucine zipper as fused to TCR $\beta$ chains.

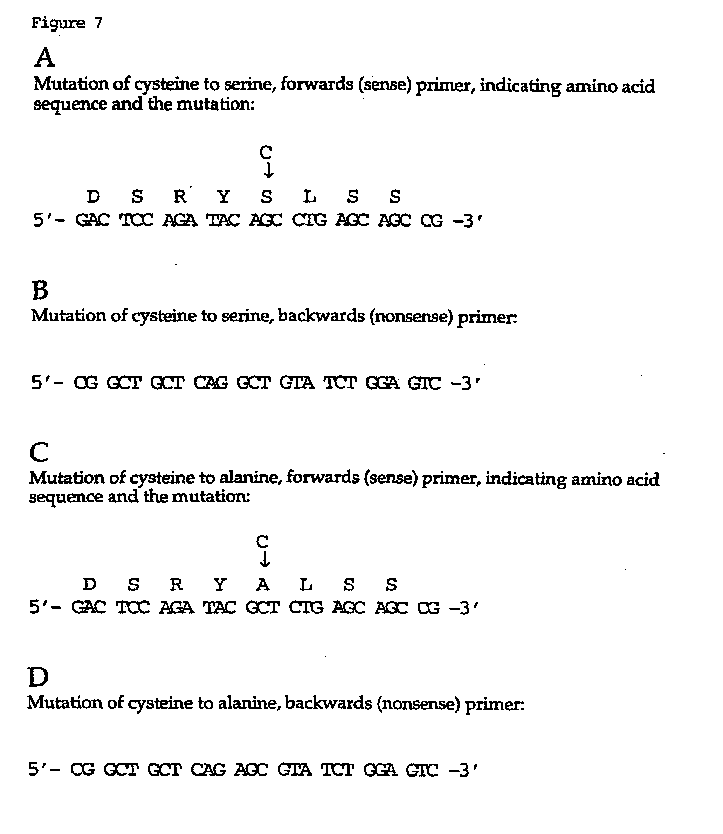

Figure 7 shows the sequences of the synthetic DNA primers used for mutating the unpaired cysteine residue in TCR $\beta$ chains. The primers were designed for used with the "Quickchange™" method for mutagenesis (Stratagene). A: Mutation of cysteine to serine, forwards (sense) primer, indicating amino acid sequence and the mutation. B: mutation of cysteine to serine, backwards (nonsense) primer. C: mutation of cysteine to alanine, forwards (sense) primer, indicating amino acid sequence and the mutation. D: mutation of cysteine to alanine, backwards (nonsense) primer.

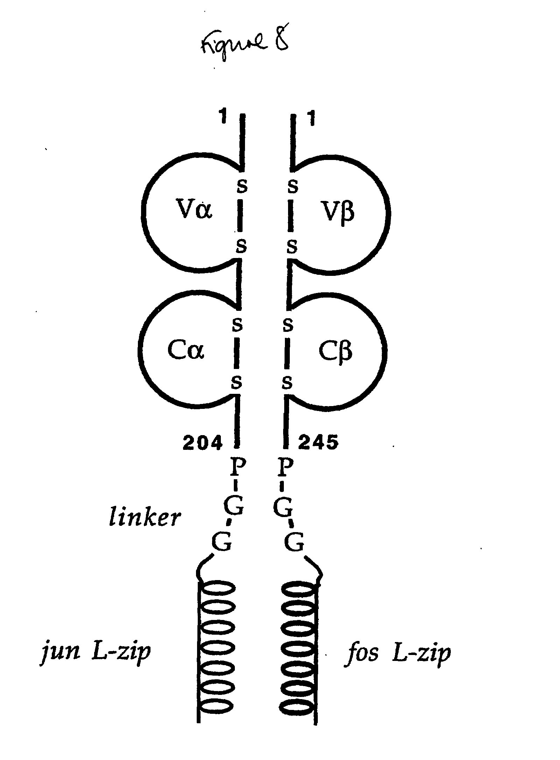

Figure 8 is a schematic representation of a TCR-zipper fusion protein. The four immunoglobulin domains are indicated as domes, with the intrachain disulphide bridges between matching pairs of cysteine residues shown. The numbers indicate amino acid positions in the mature T-cell receptor chains; due to slight variation in chain length after recombination, the lengths of the chains can vary slightly between different TCRs. The residues introduced in the linker sequences are indicated in the one-letter code.

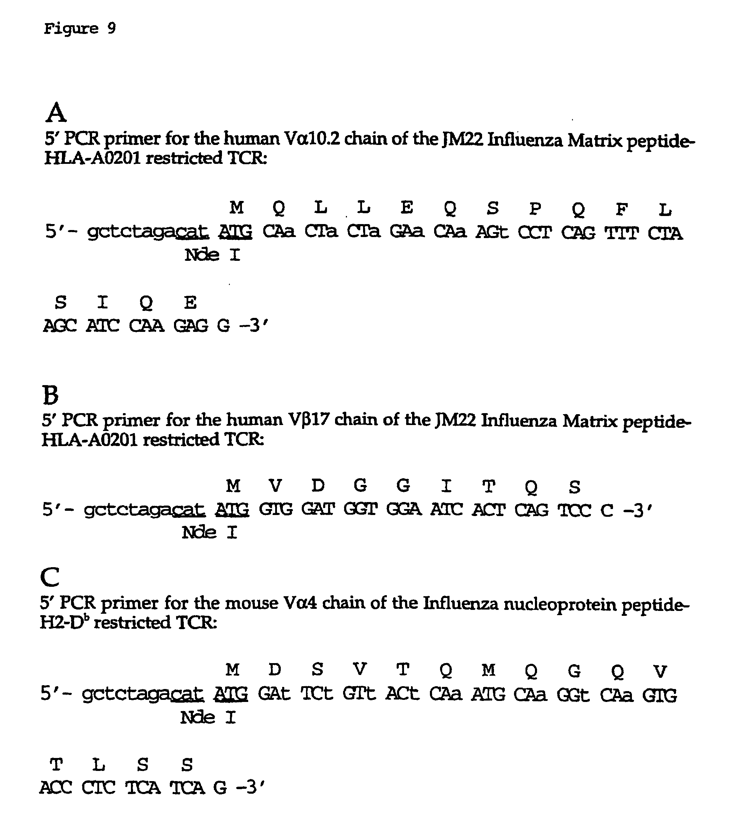

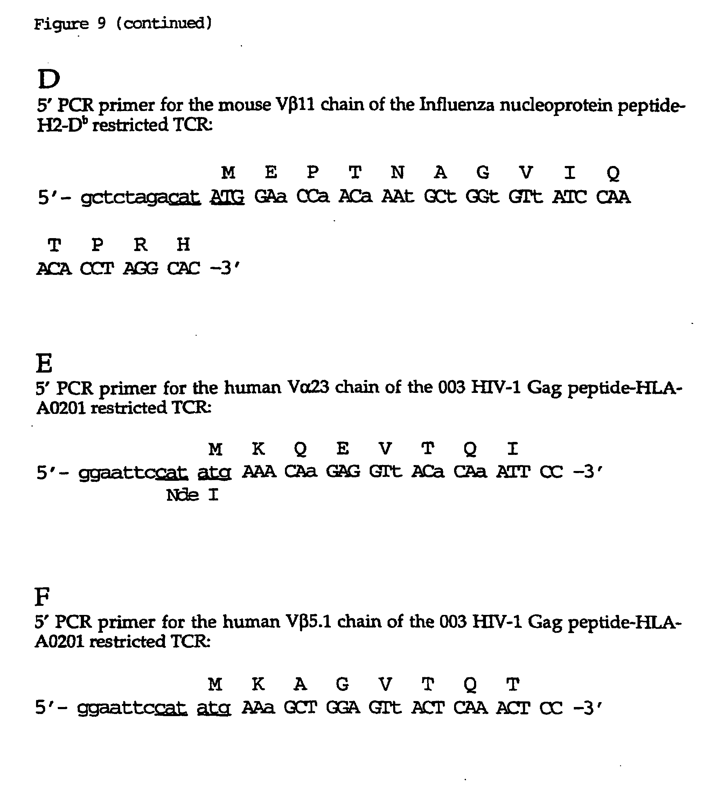

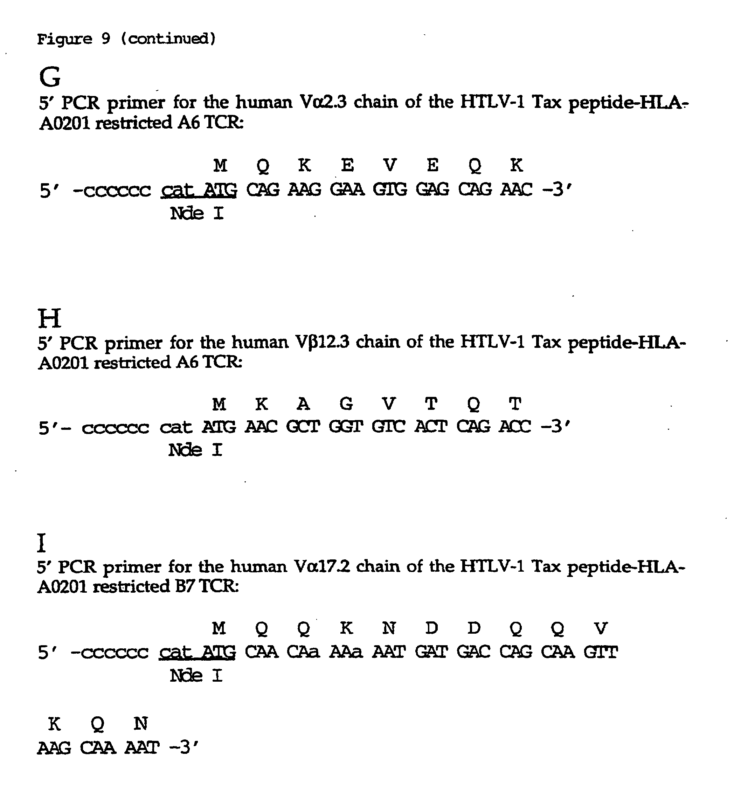

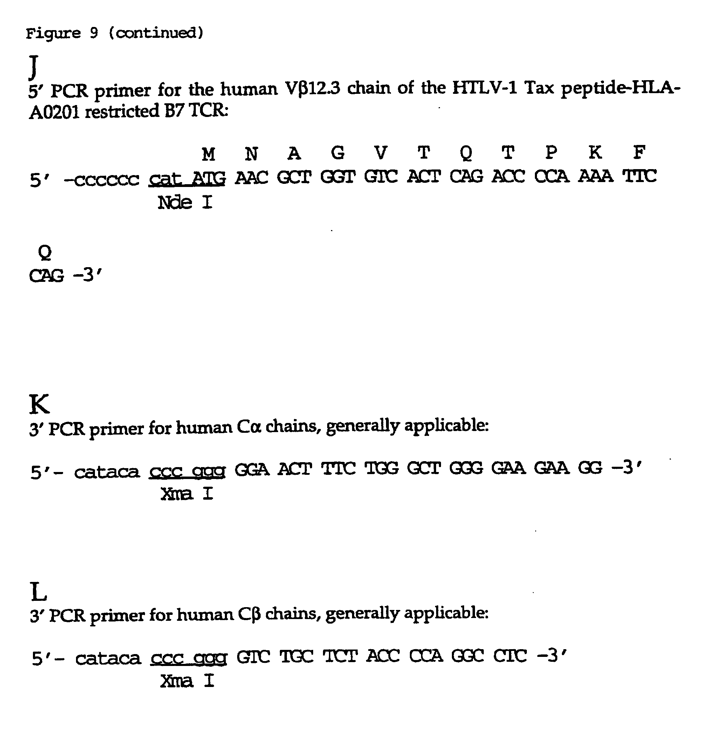

Figure 9 shows the sequences of the synthetic DNA primers used for PCR amplification of TCR $\alpha$ and $\beta$ chains. Recognition sites for DNA restriction enzymes are underlined and the amino acid sequences corresponding to the respective TCR chains are indicated over the forward primer sequences. Silent DNA mutations relative to the TCR gene sequences and other DNA sequences which do not correspond to the TCR genes are shown in lower case letters. A: 5' PCR primer for the human V$\alpha$10.2 chain of the JM22 Influenza Matrix virus peptide-HLA-A0201 restricted TCR. B: 5' PCR primer for the human V$\beta$17 chain of the JM22 Influenza Matrix virus peptide-HLA-A0201 restricted TCR. C: 5' PCR primer for the mouse V$\alpha$4 chain of the Influenza nucleoprotein peptide-H2-D$^b$ restricted TCR. D: 5' PCR primer for the mouse V$\beta$11 chain of the Influenza nucleoprotein peptide-H2-D$^b$ restricted TCR. E: 5' PCR primer of the human V$\alpha$23 chain of the 003 HIV-1 Gag peptide-HLA-A0201 restricted TCR. F: 5' PCR primer of the human V$\beta$5.1 chain of the 003 HIV-1 Gag peptide-HLA-A0201 restricted TCR. G: 5' PCR primer of the human V$\alpha$2.3 chain of

the HTLV-1 Tax peptide-HLA-A0201 restricted A6 TCR. H: 5' PCR primer of the human Vβ12.3 chain of the HTLV-1 Tax peptide-HLA-A0201 restricted A6 TCR. I: 5' PCR primer of the human Vα17.2 chain of the HTLV-1 Tax peptide-HLA-A0201 restricted B7 TCR. J: 5' PCR primer of the human Vβ12.3 chain of the HTLV-1 Tax peptide-HLA-A0201 restricted B7 TCR. K: 3' PCR primer for human Cα chains, generally applicable. L: 3' PCR primer for human Cβ chains, generally applicable.

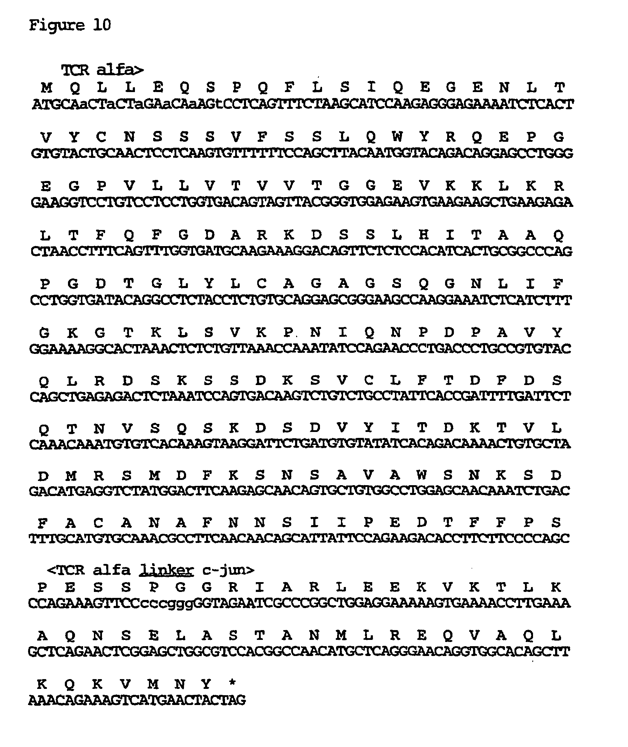

Figure 10 shows the predicted protein sequence (one letter code, top) and DNA sequence (bottom) of the soluble HLA-A2/flu matrix restricted TCR α chain from JM22, as fused to the "leucine zipper" domain of c-jun. Mutations introduced into the 5' end of the DNA sequence to enhance expression of the gene in *E. coli* are indicated in small letters, as is the linker sequence between the TCR and c-jun sequences

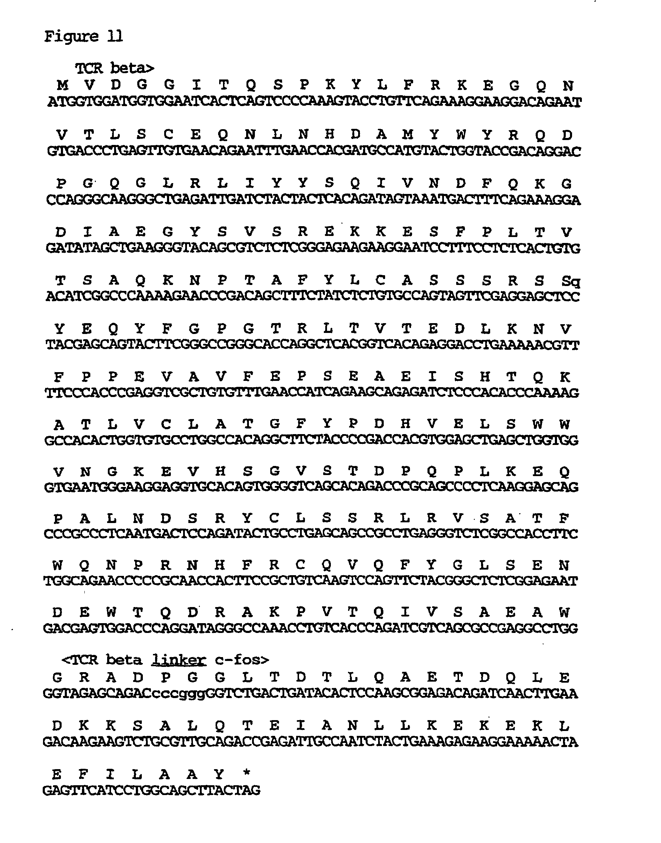

Figure 11 shows the predicted protein sequence (one letter code, top) and DNA sequence (bottom) of the soluble HLA-A2/flu matrix restricted TCR β chain from JM22, as fused to the "leucine zipper" domain of c-fos. The linker sequence between the TCR and c-fos sequences is indicated in small letters.

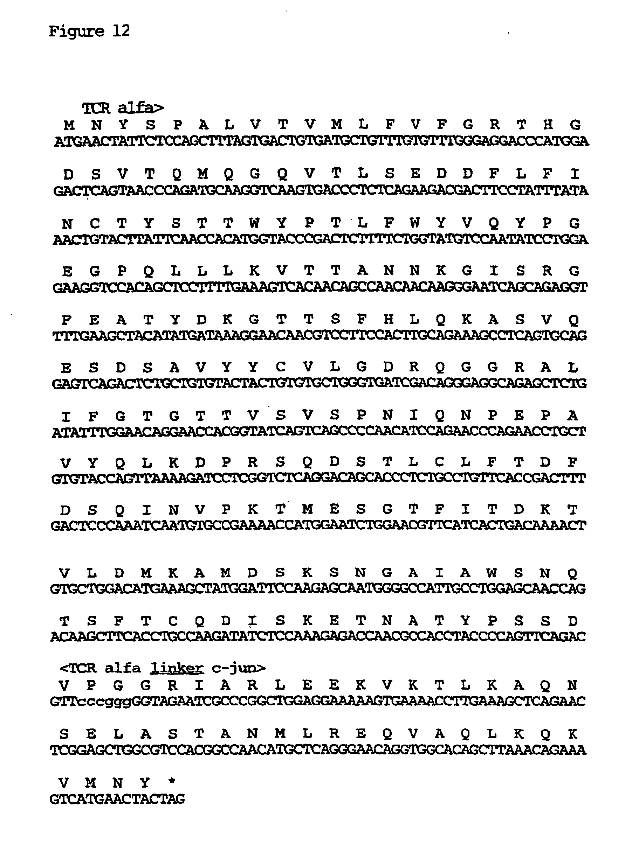

Figure 12 shows the predicted protein sequence (one letter code, top) and DNA sequence (bottom) of the soluble H2-D^b/Influenza virus nucleoprotein restricted TCR α chain from murine F5 receptor, as fused to the "leucine zipper" domain of c-jun. Mutations introduced into the 5' end of the DNA sequence to enhance expression of the gene in *E. coli* are indicated in small letters, as is the linker sequence between the TCR and c-jun sequences.

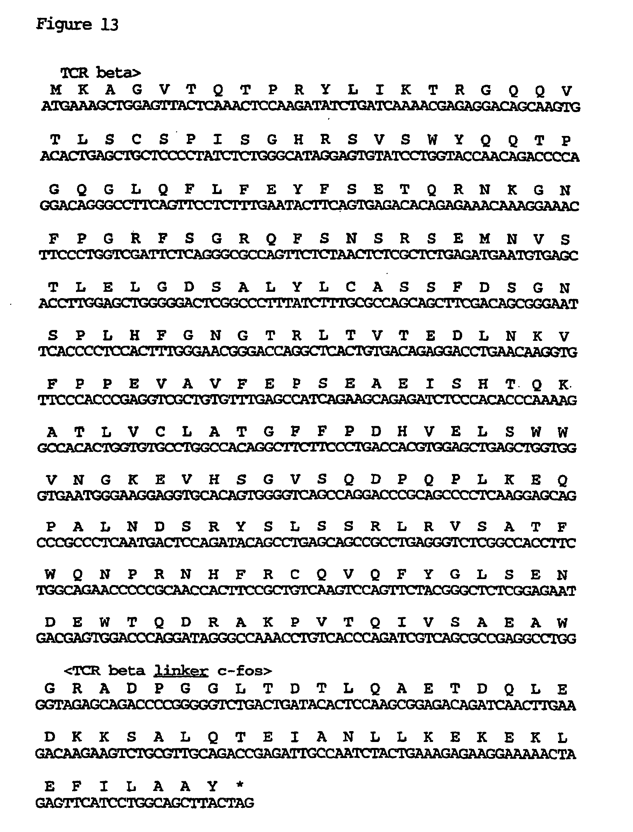

Figure 13 shows the predicted protein sequence (one letter code, top) and DNA sequence (bottom) of the soluble H2-D^b/Influenza virus nucleoprotein restricted TCR β chain from murine F5 receptor, as fused to the "leucine zipper" domain of c-fos. The linker sequence between the TCR and c-fos sequences is indicated in small letters.

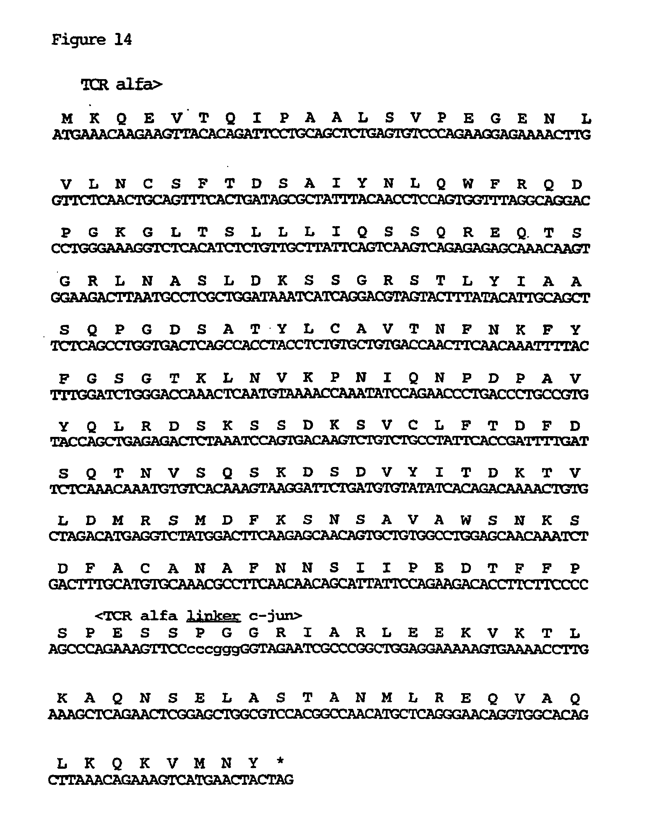

Figure 14 shows the predicted protein sequence (one letter code, top) and DNA sequence (bottom) of the soluble HLA-A2/HIV-1 Gag restricted TCR α chain from patient 003, as fused to the "leucine zipper" domain of c-jun. Mutations introduced into the 5' end of the DNA sequence to enhance expression of the gene in *E. coli* are indicated in small letters, as is the linker sequence between the TCR and c-jun sequences.

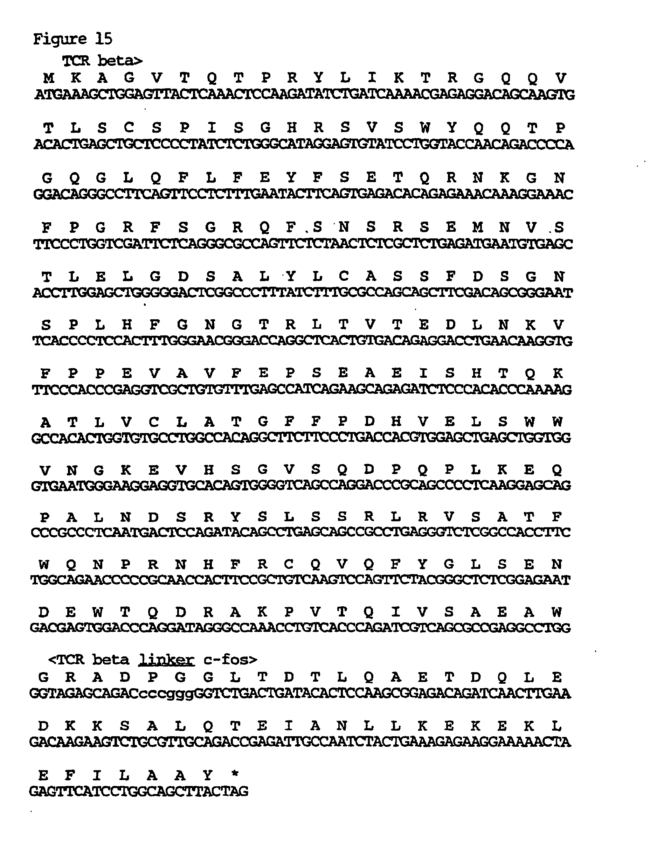

Figure 15 shows the predicted protein sequence (one letter code, top) and DNA sequence (bottom) of the soluble HLA-A2/HIV-1 Gag restricted TCR β chain from patient 003, as fused to the "leucine zipper" domain of c-fos. The linker sequence between the TCR and c-fos sequences is indicated in small letters.

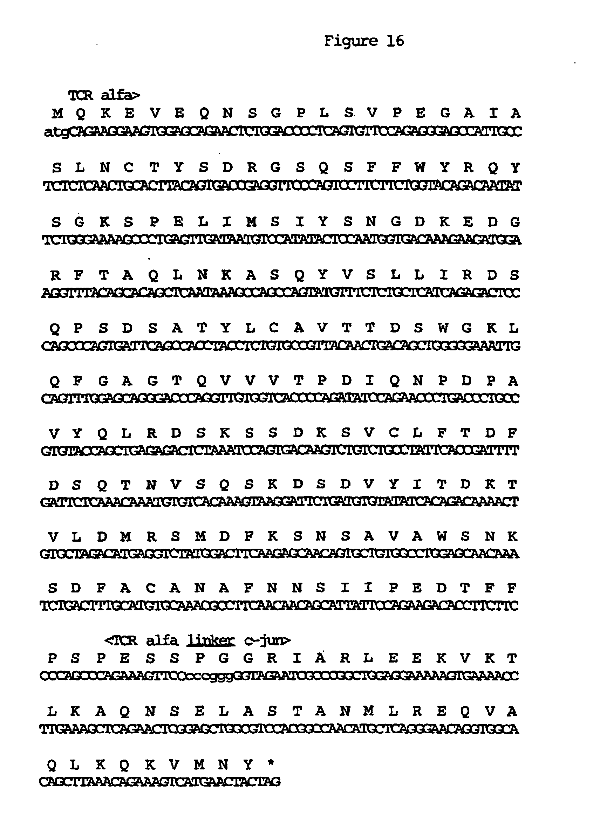

Figure 16 shows the predicted protein sequence (one letter code, top) and DNA sequence (bottom) of the soluble HTLV-1 Tax/HLA-A2 restricted TCR α chain clone A6 (Garboczi, Utz et al, 1996; Garboczi, Ghosh et al, 1996), as fused to the "leucine zipper" domain of c-jun. Mutations introduced into the 5' end of the DNA sequence to enhance expression of the gene in *E. coli* are indicated in small letters, as is the linker sequence between the TCR and c-jun sequences.

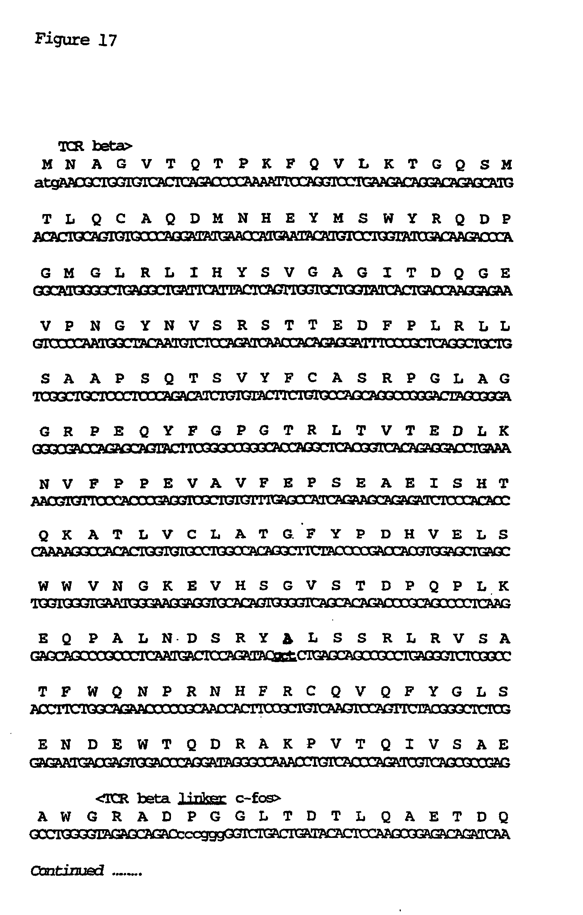

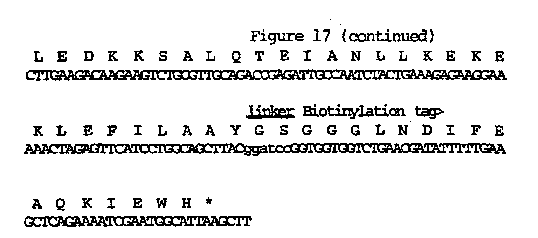

Figure 17 shows the predicted protein sequence (one letter code, top) and DNA sequence (bottom) of the soluble HTLV-1 Tax/HLA-A2 restricted TCR β chain from clone A6 (Garboczi, Utz et al, 1996; Garboczi, Ghosh et al, 1996), as fused to the "leucine zipper" domain of c-fos and the biotinylation tag which acts as a substitute for BirA (Barker and Campbell, 1981; Barker and Campbell, 1981; Howard, Shaw et al, 1985; Schatz, 1993; O'Callaghan, Byford, 1999). The linker sequence between the TCR and c-fos sequences is indicated in small letters. Mutation of the DNA sequence which substitutes a cysteine residue for an alanine residue is indicated in bold and underlined.

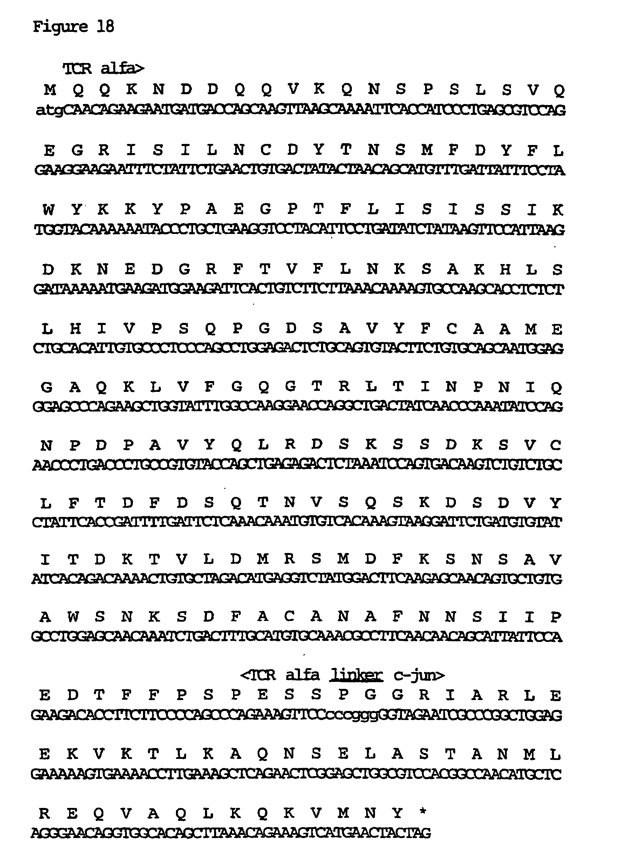

Figure 18 shows the predicted protein sequence (one letter code, top) and DNA sequence (bottom) of the soluble HTLV-1 Tax/HLA-A2 restricted TCR α chain from clone M10B7/D3 (Ding et al, 1998), as fused to the "leucine zipper" domain of c-jun. The linker sequence between the TCR and c-jun sequences is indicated in small letters.

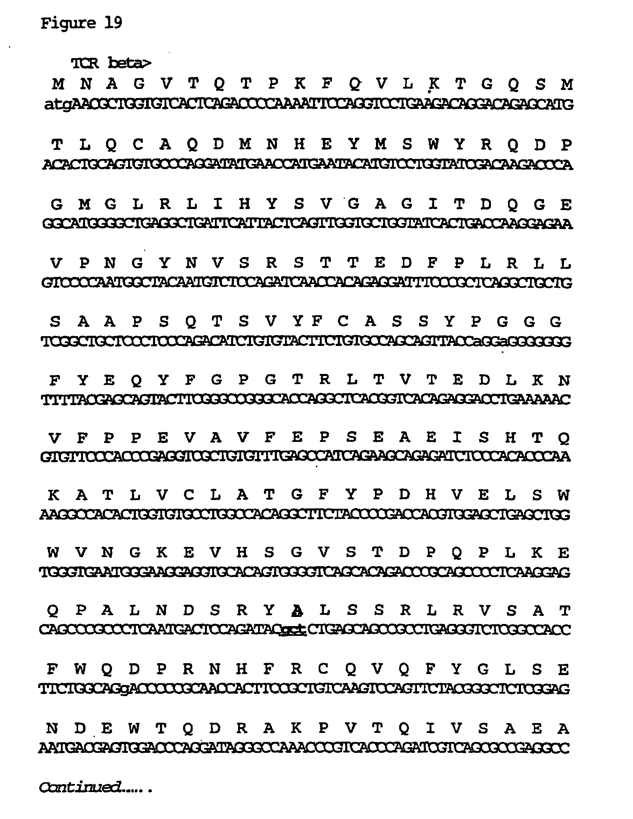

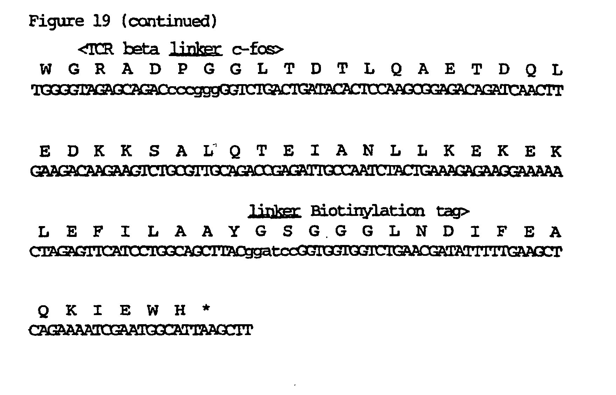

Figure 19 shows the predicted protein sequence (one letter code, top) and DNA sequence (bottom) of the soluble HTLV-1 Tax/HLA-A2 restricted TCR β chain from clone M10B7/D3 (Ding et al, 1998), as fused to the "leucine zipper" domain of c-fos and the biotinylation tag which acts as a substitute for BirA. The linker sequence between the TCR and c-fos sequences is indicated in small letters. Mutation of the DNA sequence which substitutes an alanine for a cysteine residue is indicated in bold and underlined. Two silent mutations (P-G codons) introduced for cloning purposes and to remove a Xmal restriction site are also indicated in small letters.

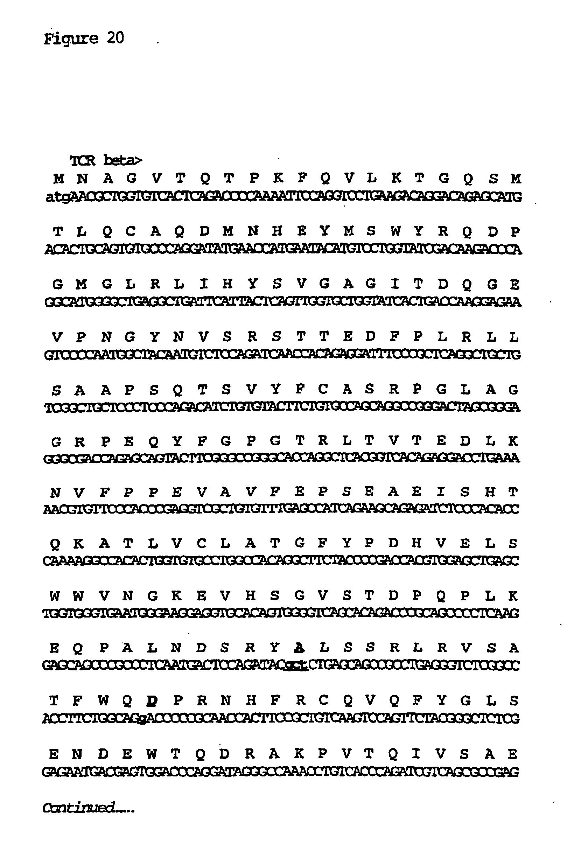

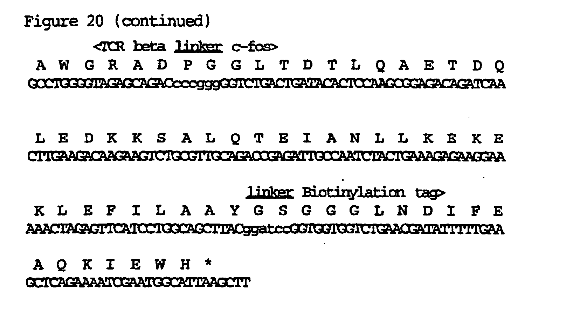

Figure 20 shows the predicted protein sequence (one letter code, top) and DNA sequence (bottom) of mutated soluble HTLV-1 Tax/HLA-A2 restricted TCR β chain from clone A6 (Garboczi, Utz et al, 1996; Garboczi, Ghosh et al, 1996), as fused to the "leucine zipper" domain of c-fos and the biotinylation tag which acts as a substitute for BirA (Barker and Campbell, 1981; Barker and Campbell, 1981; Howard, Shaw, 1985; Schatz, 1993; O'Callaghan, Byford, 1999). The linker sequence between the TCR and c-fos sequences is indicated in small letters. Mutation of the DNA sequence which substitutes a cysteine residue for an alanine residue is indicated in bold and underlined. Also indicated in bold and underlined is a substitution of an asparagine residue for an aspartic acid, a mutation in the constant region which had no detectable functional effect on the soluble TCR.

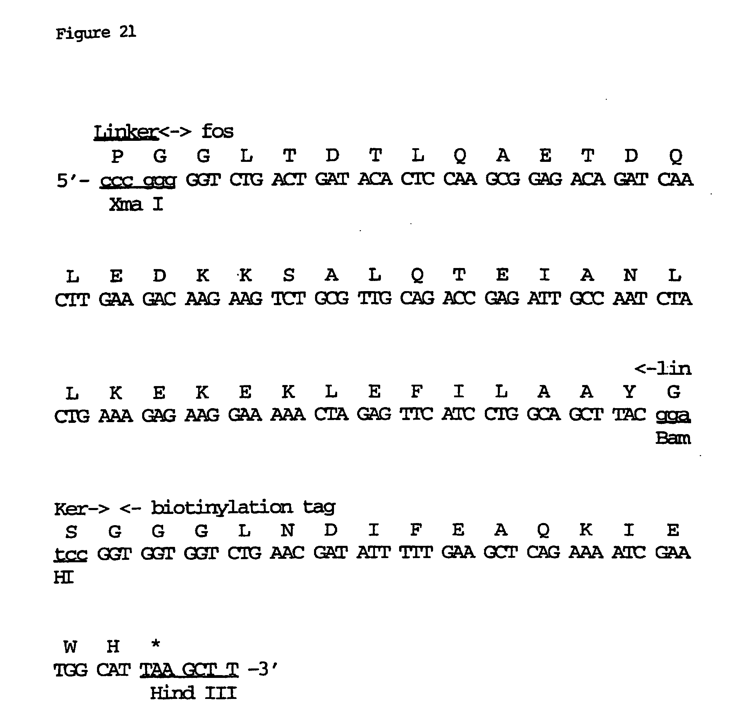

Figure 21 shows the predicted protein sequence (one letter code, top) and DNA sequence (bottom) of the *c-fos* - biotinylation fusion partner used for TCR β chains. Recognition sites for DNA restriction enzymes are underlined and the borders of the two fusion domains are indicated. Linker sequences are shown in lower case letters.

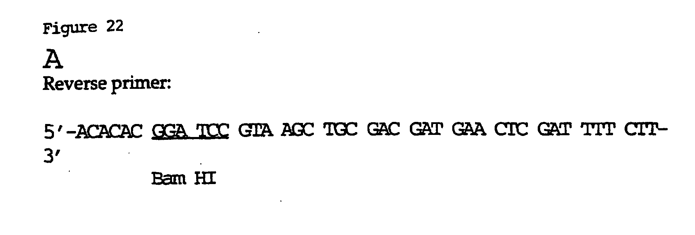

Figure 22 shows the sequence of a synthetic DNA primer used for PCR amplification of the Vβ-*c-fos* leucine zipper fragment of the human JM22 Influenza Matrix peptide-HLA-A0201.

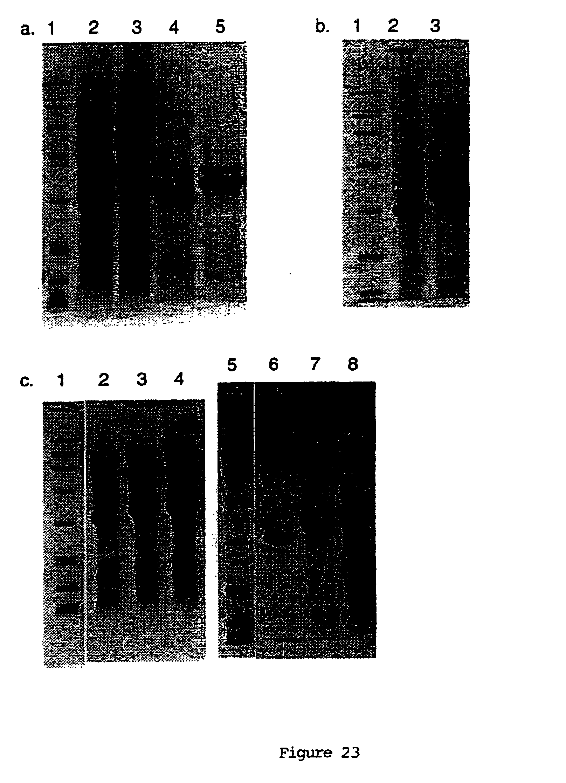

Figure 23 is a set of photographs of gels. <u>a.</u> Preparation of denatured protein for the TCR specific for the 003 HIV gag peptide - HLA-A2 complex analysed by SDS-PAGE. Lane 1: broad-range molecular weight markers (Bio-Rad), lanes 2 & 3: bacteria after induction of protein expression with 0.5 mM IPTG, lanes 4 & 5: purified inclusion bodies solubilised in 6M guanidine buffer. <u>b.</u> Preparation of denatured protein for the biotin-tagged TCR specific for the influenza matrix peptide - HLA-A2 complex analysed by SDS-PAGE. Lane 1: broad-range molecular weight markers (Bio-Rad), lanes 2 & 3: $\alpha$- & $\beta$-chain purified inclusion bodies solubilised in 6M guanidine buffer. <u>c.</u> Preparation of denatured protein for the biotin-tagged TCR specific for the HTLV tax peptide - HLA-A2 complex analysed by SDS-PAGE. Lanes 1 & 5: broad-range molecular weight markers (Bio-Rad), lanes 2, 3 & 4: $\alpha$-, $\beta$- & mutant $\beta$-chain expression in bacteria after induction of protein expression with 0.5 mM IPTG, lanes 6, 7 & 8: $\alpha$-, $\beta$- & mutant $\beta$-chain purified inclusion bodies solubilised in 6M guanidine buffer.

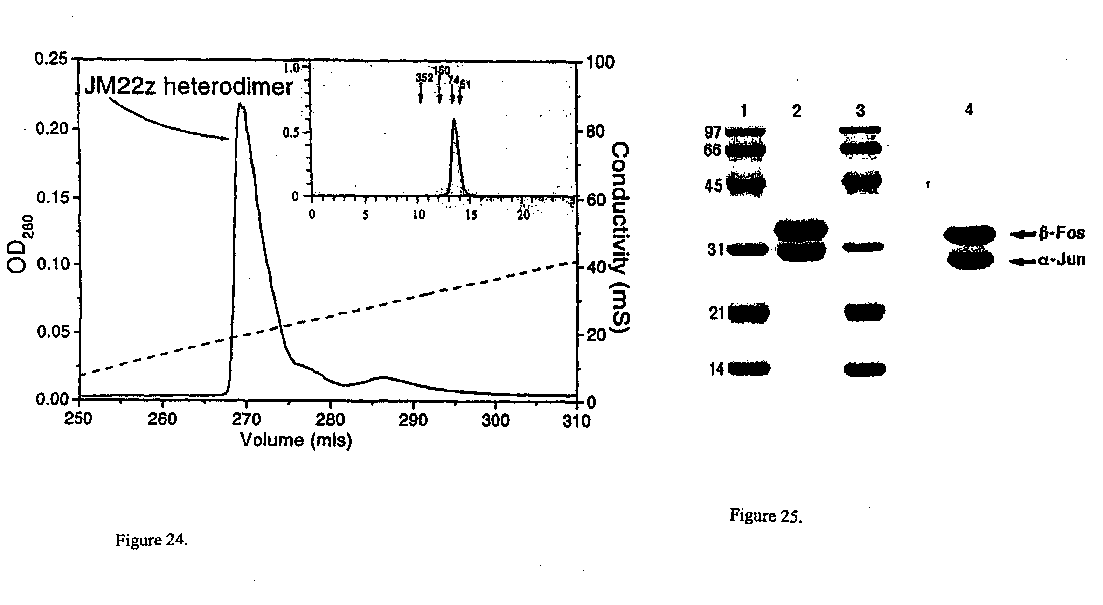

Figure 24 is a chromatogram showing the elution of the JM22z heterodimer from a POROS 10HQ anion exchange column. Dashed line shows the conductivity which is indicative of a sodium chloride concentration, the solid line shows optical density at 280 nm which is indicative of protein concentration of the eluate. Peak protein containing fractions were pooled for further analysis. Insert shows a chromatogram of elution of purified JM22z from a Superdex 200 HR column. Arrows indicate the calibration of the column with proteins of known molecular weight By comparison with these proteins, the refolded JM22z protein has a molecular weight of approximately 74 kDA which is compatible with a heterodimeric protein.

Figure 25 is a photograph showing an SDS-polyacrylamide gel electrophoresis (Coomassie-stained) of the purified JM22z protein. Lanes 1 & 3: standard proteins of known molecular weight (as indicated), lane 2: JM22z protein treated with SDS-sample buffer containing reducing agent (DTT) prior to sample loading, lane 4: JM22z protein treated with SDS-sample buffer in the absence of reducing agents.

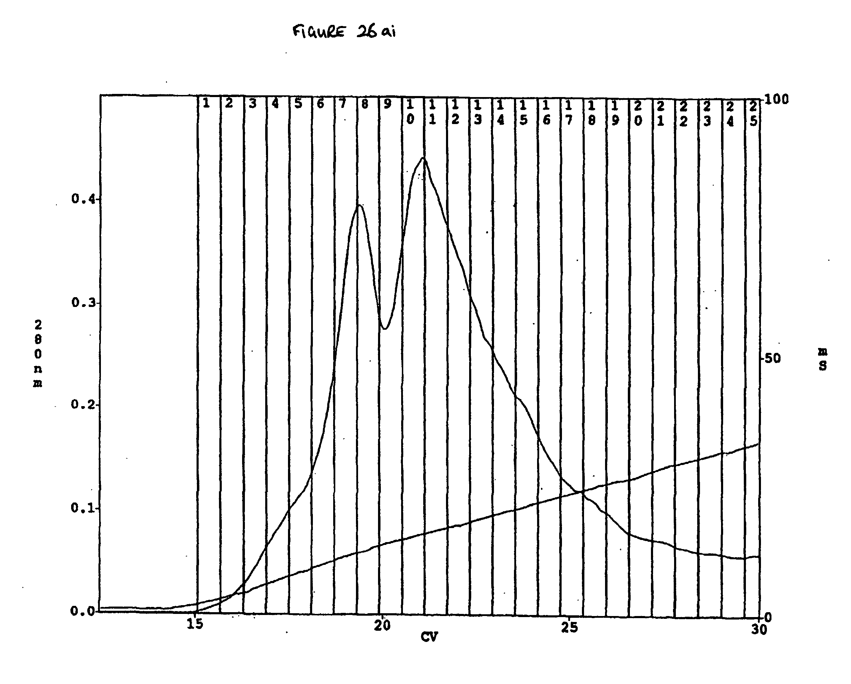

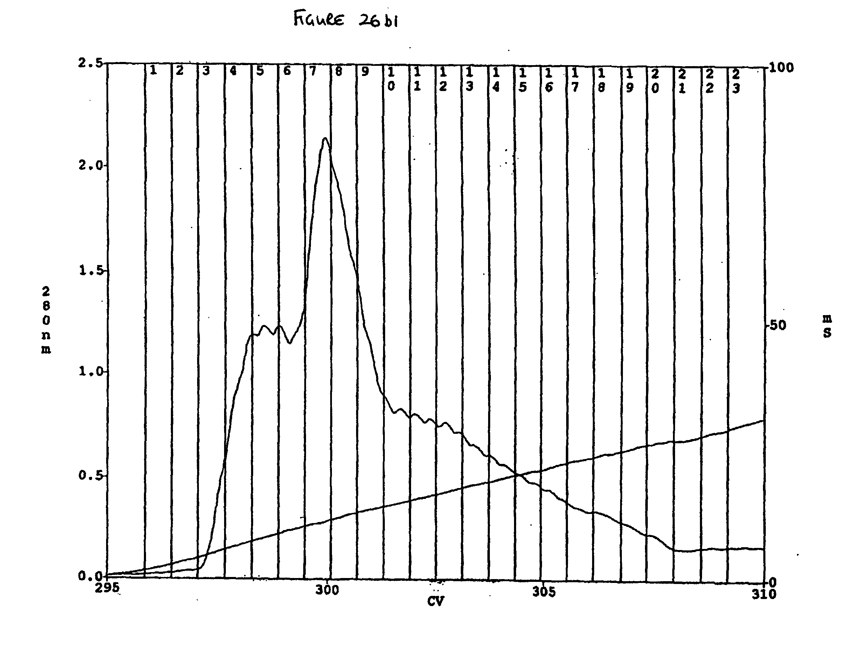

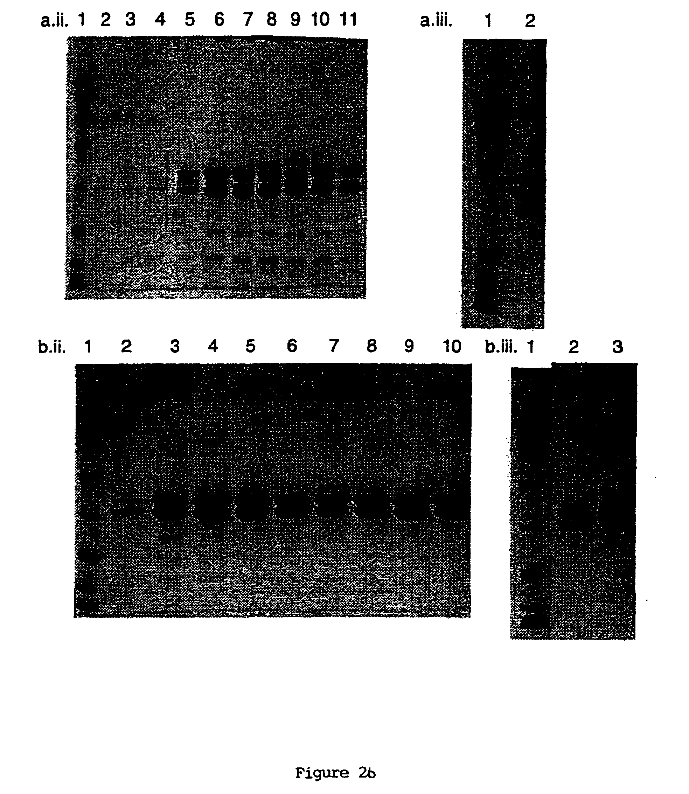

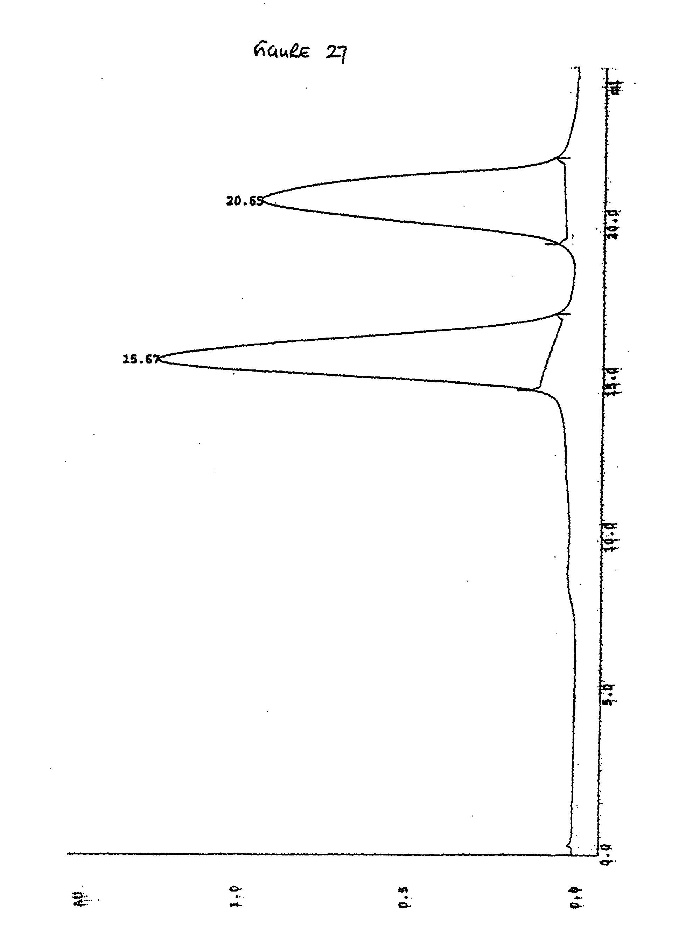

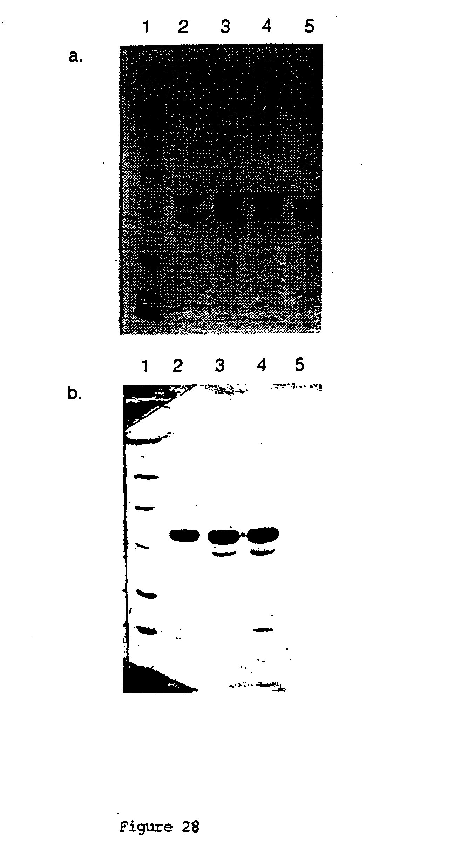

Figure 26. <u>a.</u> Purification of the refolded biotin-tagged TCR specific for the influenza matrix peptide - HLA-A2 complex. i. Chromatogram of the elution of the protein from a POROS 10HQ column. Line x indicates absorbance at 280 nm and line y indicates conductivity (a measure of sodium chloride gradient used to elute the protein). Fraction numbers are indicated by the vertical lines ii. SDS-PAGE of the fractions eluting off the column as in i. Lane 1 contains broad-range molecular weight markers (Bio-Rad) and lanes 2-13 contain 5 µl of fractions 6-15 respectively. iii. SDS-PAGE analysis of pooled fractions from i. containing biotin-tagged flu-TCR. Lane 1: broad-range molecular weight markers (Bio-Rad), lane 2: biotin-tagged flu-TCR protein. <u>b.</u> Purification of the refolded biotin-tagged TCR specific for the HTLV-tax peptide - HLA-A2 complex. i. Chromatogram of the elution of the protein from a POROS 10HQ column.

Line x indicates absorbance at 280 nm and line y indicates conductivity (a measure of sodium chloride gradient used to elute the protein). Fraction numbers are indicated in by the vertical lines. ii. SDS-PAGE of the fractions eluting off the column as in i. Lane 1 contains broad-range molecular weight markers (Bio-Rad) and lanes 2 - 10 contain 5µl of fractions 3 - 11 respectively. iii. SDS-PAGE analysis of pooled fractions from i. of biotin-tagged tax-TCR. Lane 1: broad-range molecular weight markers (Bio-Rad), lane 2: biotin-tagged tax-TCR protein, lane 3: mutant biotin-tagged tax-TCR protein.

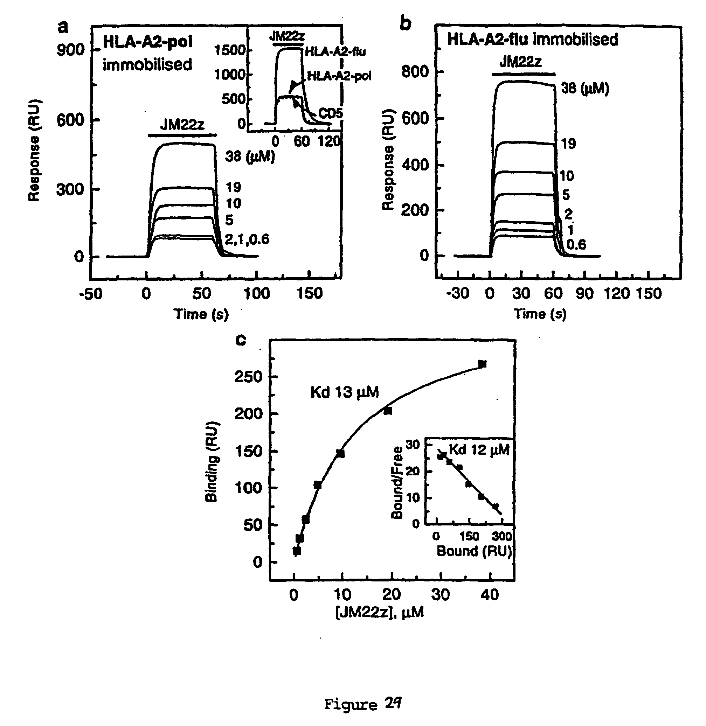

Figure 27 is a chromatogram showing elution of biotin-tagged soluble TCR after biotinylation with BirA enzyme from a Superdex 200 HR column equilibrated in PBS. The biotinylated TCR elutes at around 15-16 minutes and the free biotin elutes at around 21 minutes. Fractions containing biotinylated soluble TCR are pooled for future use.

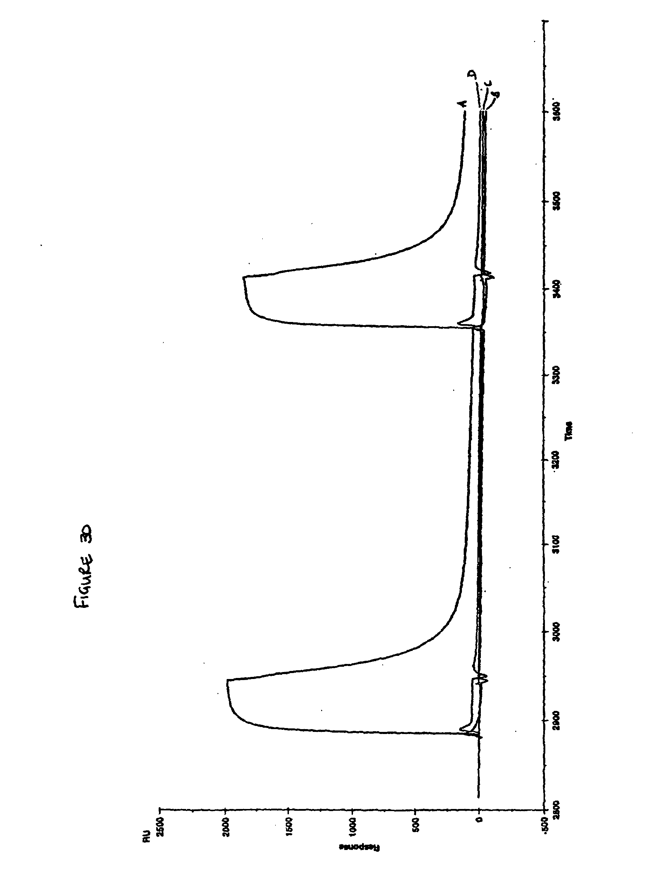

Figure 28 is a set of photographs of gels. Assessment of biotinylation of the biotinylated TCRs. a. SDS-PAGE of refolded TCRs and inclusion body preparations. Lane 1: broad-range molecular weight markers (Bio-Rad), lane 2: Biotinylated flu-TCR, lane 3: Biotinylated tax-TCR, lane 4: Biotinylated mutant tax-TCR, lane 5: HIV gag-TCR, (not biotin-tagged); b. Western blot of a gel identical to a. except that the broad-range markers were biotin labelled (Bio-Rad). Staining was with avidin-HRP conjugate to show biotinylated proteins and visualisation was with Opti-4CN (Bio-Rad).

Figure 29 illustrates JM22z binding to different HLA-A2-peptide complexes. (a inset) The specificity of the interaction between JM22z and HLA-A2-flu is demonstrated by comparing the SPR response from passing the TCR over a flow cell coated with 1900 RU of HLA-A2-flu to the responses from passing the TCR over two other flow cells one coated with 4200 RU of HLA-A2-pol, the other coated with 4300 RU of CD5. Background responses at different JM22z concentrations were measured on 1700 RU of HLA-A2-pol (a). The background value was subtracted from the specific response measured on 1900 RU of HLA-A2-flu (b) and plotted against concentration (c). The Kd of 13 µM, estimated by non-linear curve fitting was in accordance with the Kd of 12 µM calculated on basis of a Scatchard plot of the same data.

Figure 30 is a graph showing the result of Biacore 2000™ analysis of wild-type and mutant soluble biotinylated tax TCR. 5 µl of wild-type tax TCR at a concentration of 2.2 mg/ml and then mutant tax TCR at a concentration of 2.4 mg/ml was flowed over four flow cells with the following proteins attached to the surface: A: tax-pMHC complex, B/C: flu-pMHC complex, D: OX68 control protein. Both wild-type and mutant proteins bind similarly to the specific pMHC complex.

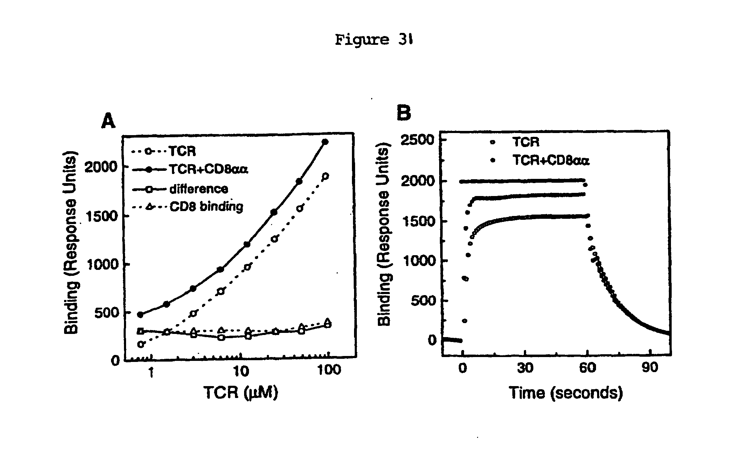

Figure 31 shows the effect of soluble CD8aa binding on soluble TCR binding to the same HLA-A2-flu complex. (A) TCR or TCR plus 120 µM soluble CD8 were injected into a control flow cell coated with 4100 RU of an irrelevant protein (CD5) and a probe flow cell coated with 4700 RU of HLA-A2-flu. After subtraction of the background, the calculated equilibrium response values at different concentrations of TCR alone (open circles) or in combination with 120 µM soluble CD8 (closed circles) is shown. Also shown is the value of CD8 alone (open triangles) and the calculated difference between TCR + CD8 and TCR alone (open squares). (B) The time-dependence of the responses on 4700 RU of immobilised HLA-A2-flu of 49µM TCR alone (open circles) or in combination with 120 µM CD8 (closed circles) at 25° C and a flow rate of 5 µl/min is shown (The values are corrected for background contributions measured on 4100 RU of immobilised CD5); the off-rate of TCR is not affected by the simultaneous CD8 binding.

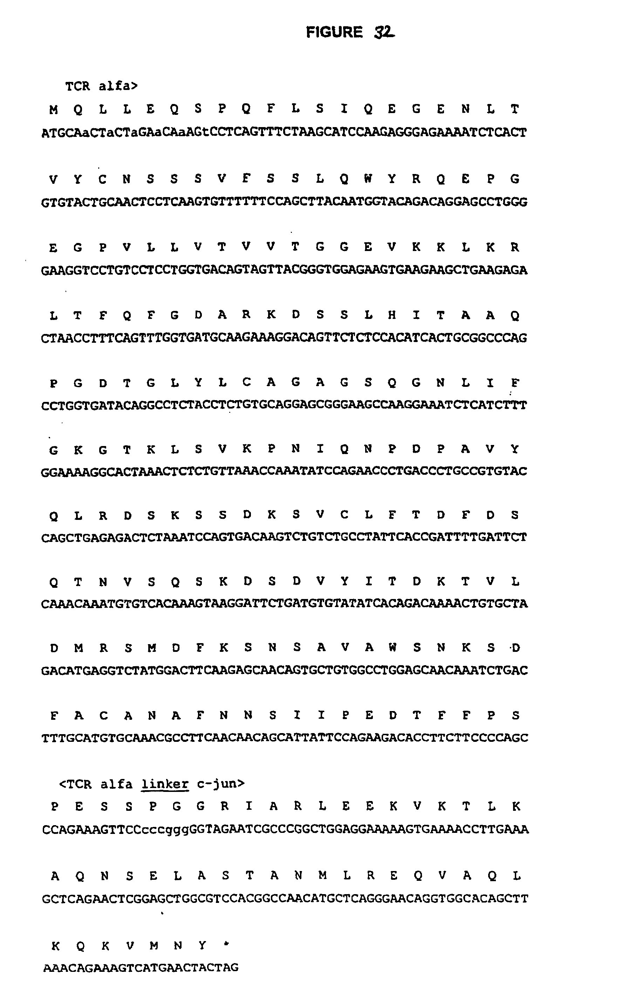

Figure 32 shows the protein sequence (one-letter code, top) and DNA sequence (bottom) of the soluble, HLA-A2/flu matrix restricted TCR alfa chain from JM22, as fused to the "leucine zipper" domain of c-jun. Mutations introduced in the 5' end of the DNA sequence to enhance expression of the gene in *E.coli* are indicated in small letters as is the linker sequence between the TCR and c-jun sequences.

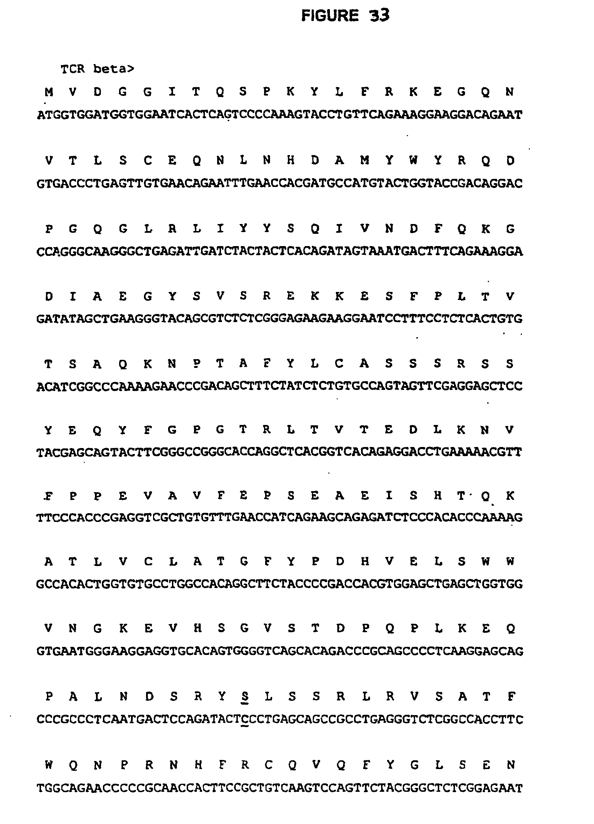

Figure 33 show the protein sequence (one-letter code, top) and DNA sequence (bottom) of the soluble, HLA-A2/flu matrix restricted TCR beta chain from JM22, as fused to the "leucine zipper" domain of c-fos. The linker sequence between the TCR and c-fos sequences is indicated in small letters. Mutation of the DNA sequence which substitutes a Serine residue for a Cysteine residue is indicated in bold and underlined. This mutation increases the folding efficiency of the TCR.

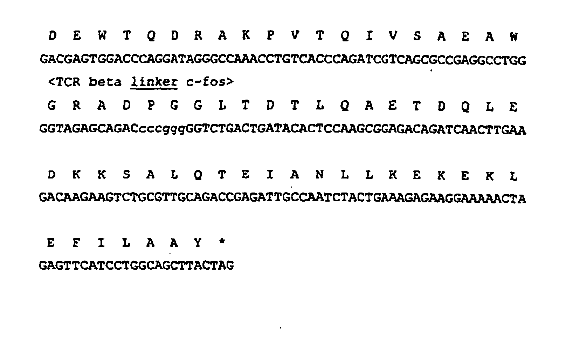

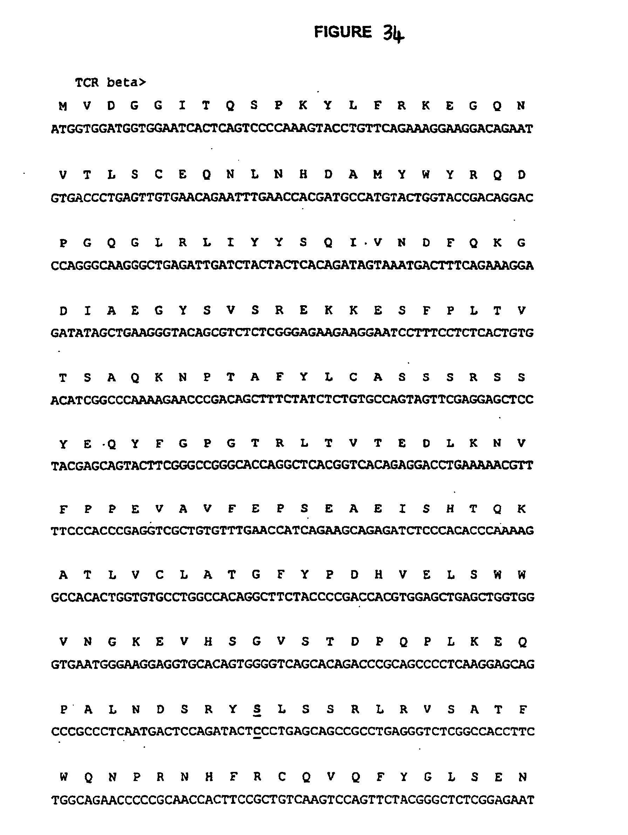

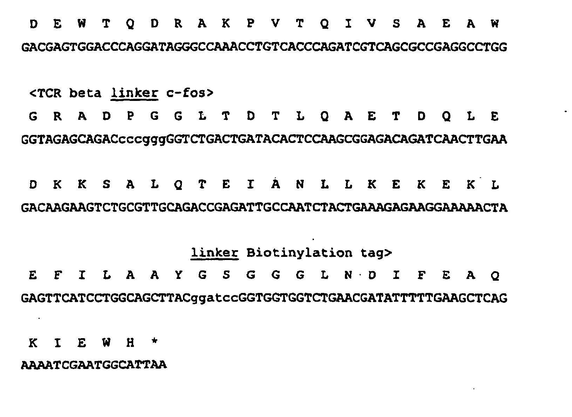

Figure 34 shows the protein sequence (one-letter code, top) and DNA sequence (bottom) of the soluble, HLA-A2/flu matrix restricted TCR beta chain from JM22, as fused to the "leucine zipper" domain of c-fos and the biotinylation tag which acts as a substrate for BirA. The linker sequence between the TCR and c-fos sequences, and between c-fos and the biotinylation tag, are indicated in small letters. Mutation of the DNA sequence which substitutes a Serine residue for a Cysteine residue is indicated in bold and underlined. This mutation increases the folding efficiency of the TCR.

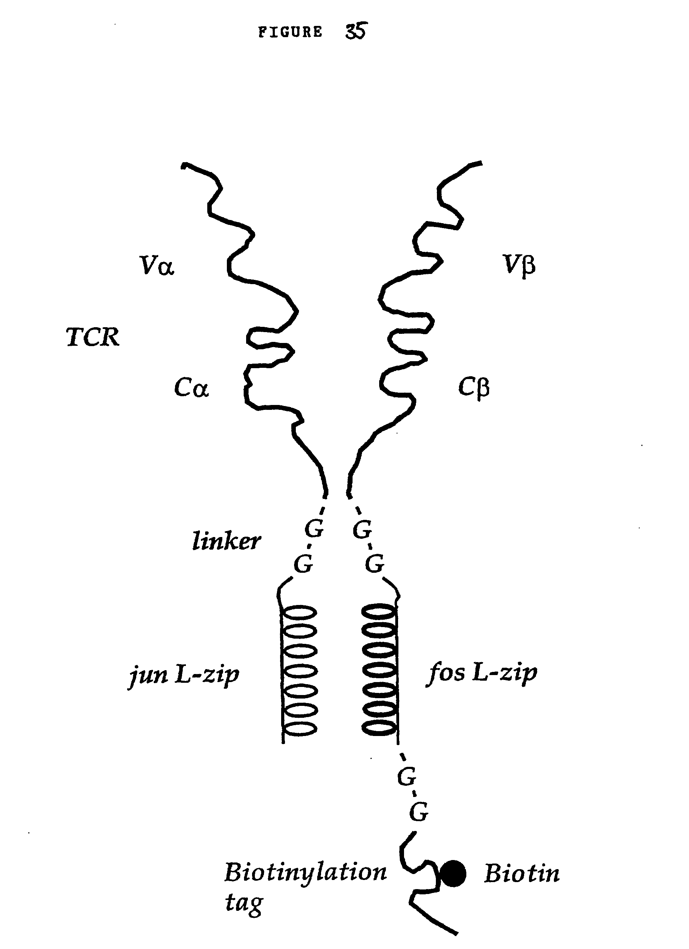

Figure 35 is a schematic diagram of TCR-zipper-biotinylation tag fusion protein

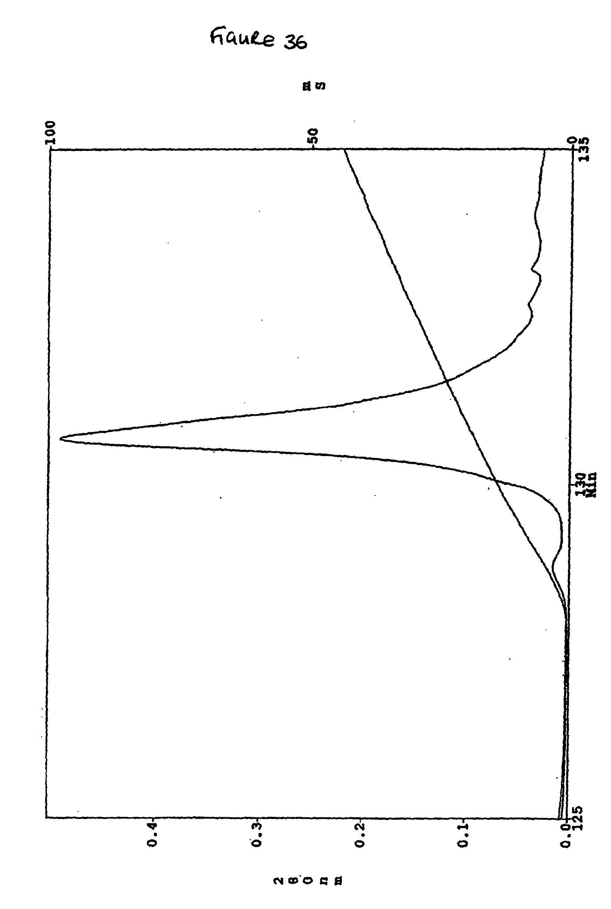

Figure 36. Elution of refolded TCR from POROS 10HQ column with a gradient of sodium chloride. TCR elutes as a single peak at approximately 100 mM NaCl. Fractions containing protein with an OD(280 nm) of more than 0.1 were pooled and concentrated for biotinylation.

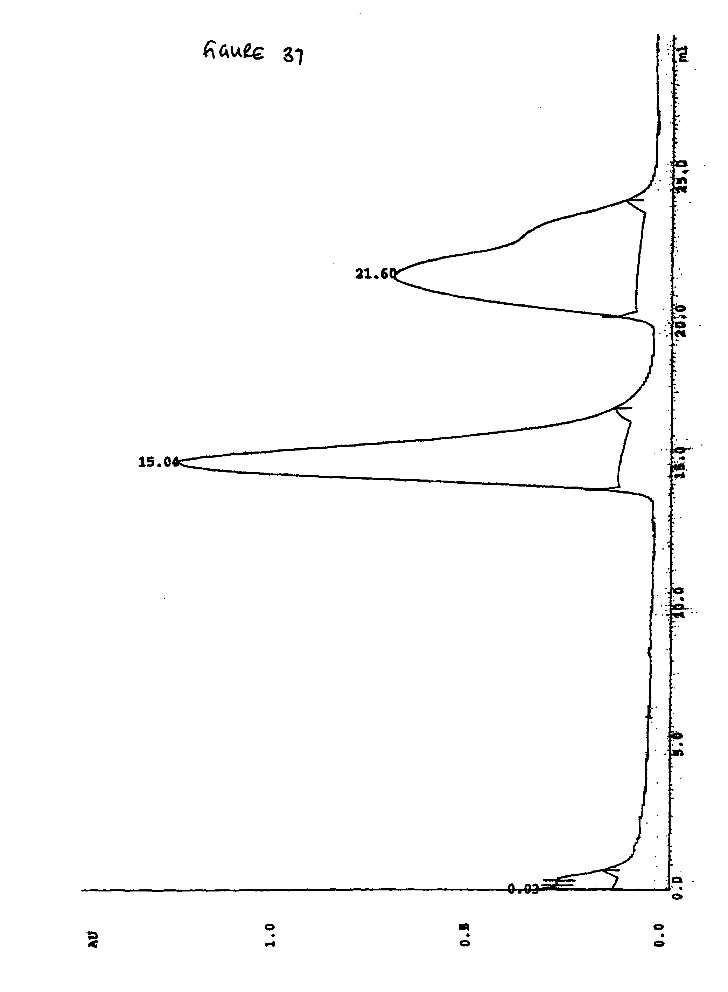

Figure 37. Separation of biotinylated TCR from free biotin by gel filtration on a Superdex 200HR 10/30 column (Pharmacia). TCR-biotin elutes at around 15 ml, corresponding to a molecular weight of 69 kDa. (Standard proteins and their elution volumes: Thyroglobulin (669 kDa) 10.14 ml, Apoferritin (443 kDa) 11.36 ml, beta-amylase (200 kDa) 12.72 ml, BSA dimer (134 kDa) 13.12 ml, BSA monomer (67 kDa) 14.93 ml, ovalbumin (43 kDa) 15.00 ml, chymotrypsinogen A (25 kDa) 18.09 ml, RNase A (13.7 kDa) 18.91 ml).

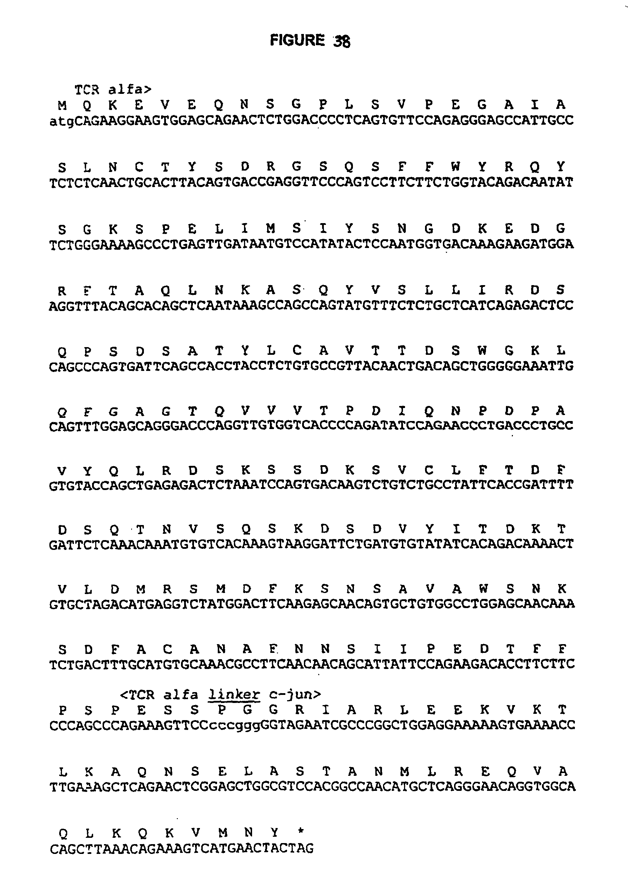

Figure 38. Protein sequence (one-letter code, top) and DNA sequence (bottom) of the soluble, HTLV-1 Tax/HLA-A2 restricted TCR alfa chain from clone A6 (Garboczi et al., 1996; Garboczi et al., 1996), as fused to the "leucine zipper" domain of c-jun. Mutations introduced in the 5' end of the DNA sequence to enhance expression of the gene in *E.coli* are indicated in small letters as is the linker sequence between the TCR and c-jun sequences.

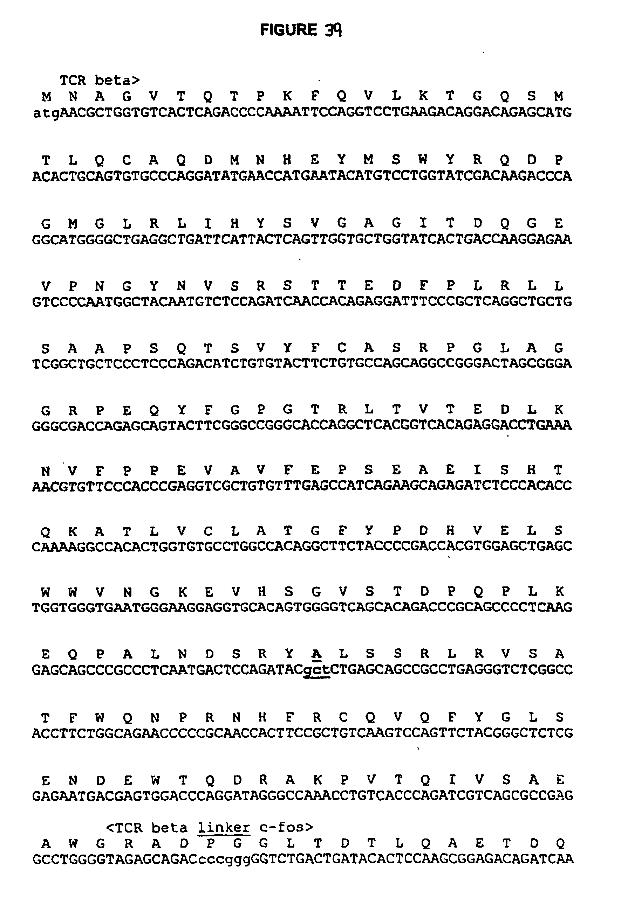

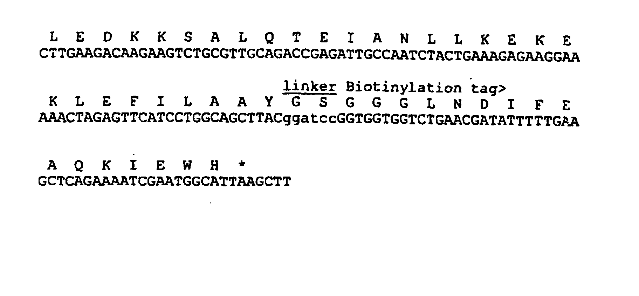

Figure 39. Protein sequence (one-letter code, top) and DNA sequence (bottom) of the soluble, HTLV-1 Tax/HLA-A2 restricted TCR beta chain from clone A6 (Garboczi et al., 1996; Garboczi et al., 1996), as fused to the "leucine zipper" domain of c-fos and the biotinylation tag which acts as a substrate for BirA. The linker sequence between the TCR and c-fos sequences is indicated in small letters. Mutation of the DNA sequence which substitutes an Alanine residue for a Cysteine residue is indicated in bold and underlined.

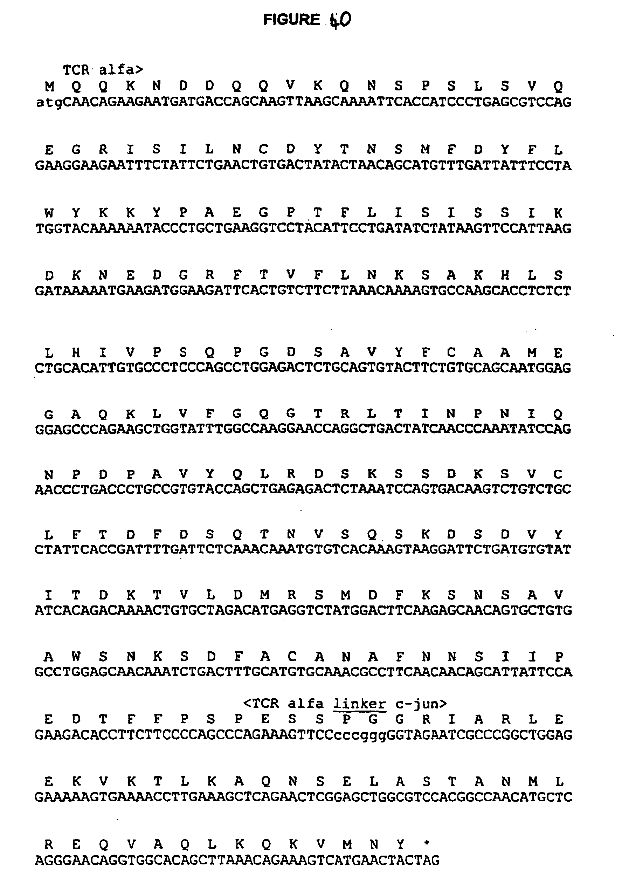

Figure 40. Protein sequence (one-letter code, top) and DNA sequence (bottom) of the soluble, HTLV-1 Tax/HLA-A2 restricted TCR alfa chain from clone M10B7/D3 (Ding et al., 1998), as fused to the "leucine zipper" domain of c-jun. The linker sequence between the TCR and c-jun sequences is indicated in small letters.

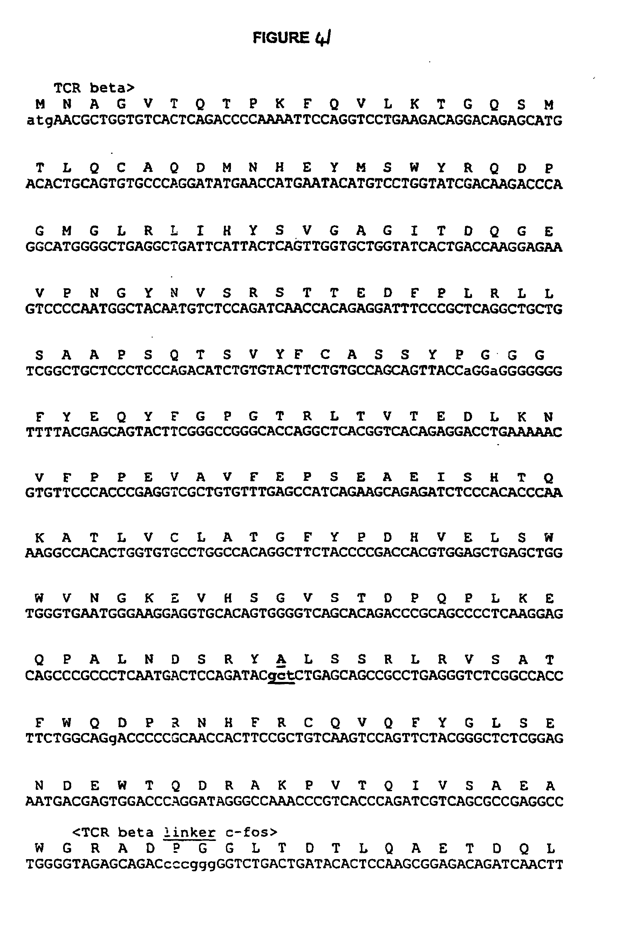

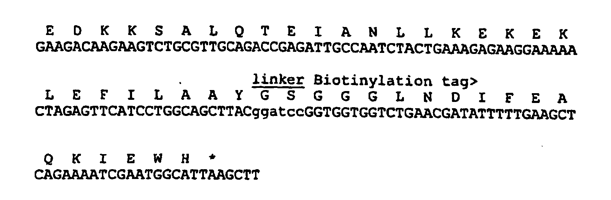

Figure 41. Protein sequence (one-letter code, top) and DNA sequence (bottom) of the soluble, HTLV-1 Tax/HLA-A2 restricted TCR beta chain from clone m10B7/D3 (Ding et al., 1998), as fused to the "leucine zipper" domain of c-fos and the biotinylation tag which acts as a substrate for BirA. The linker sequence between the TCR and c-fos sequences is indicated in small letters. Mutation of the DNA sequence which substitutes an Alanine residue for a Cysteine residue is indicated in bold and underlined. Two silent mutations (P-G codons) introduced for cloning purposes and to remove a XmaI restriction site are also indicated in small letters.

**EXAMPLES**

In the following examples, the general methods and materials set out below were used.

Materials

Restriction enzymes (Ndel, BamHI, HindIII, Bsu361, Xmal) were from New England Biolabs.
Tris pH 8.1 was made up as a 2M stock solution from equal parts of Tris base and Tris-HCl both from USB.
EDTA (Sigma) was made up as a 0.5M stock solution and the pH was adjusted to 8.0 using 5M NaOH (Sigma).
Glutathione in oxidised and reduced forms was from Sigma.
Cystamine and cysteamine were from Sigma.
Sodium Chloride was from USB and was made up to a 4M stock solution.
Miniprep kits for plasmid purification were from Quiagen.
PCR purification kits were from Quiagen.
DTT was from Sigma.
Guanidine was from Fluka.
Urea was from Sigma.
RPMI medium was from Sigma.
PBS was made up from tablets from Oxoid.
Glycerol was from BDH.

General Methods

Bacterial media (TYP media) were prepared as follows: 160 g Yeast Extract (Difco), 160 g Tryptone (Difco), 50 g NaCl (USB) and 25 g $K_2HPO_4$ (BDH) were dissolved in 2 L demineralised water. 200 ml aliquots of this solution were measured into 10 x 2 L conical flasks and made up to 1 L by adding 800 ml demineralised water. Flasks were covered with four layers of aluminium foil, labelled and autoclaved. After cooling, the flasks were stored at room temperature out of direct sunlight prior to use.

Protein concentrations were measured using a Pierce Coomassie-binding assay and BSA as a standard protein. Briefly, 0-25 µg BSA standards in a volume of 1 ml water were prepared from a stock 2 mg/ml BSA (Pierce) in 4 ml plastic cuvettes. Approximately 10 µg of unknown protein was made up to 1 ml with water in the same way. 1 ml Pierce Coomassie reagent was added to each cuvette and the contents were thoroughly mixed. The optical density was measured within 15 minutes at 595 nm using a Beckman DU-530 UV spectrophotometer. A linear regression was performed on the results from the BSA standards (linearity was good up to 25 µg BSA) and the unknown protein concentration was estimated by interpolation with these results.

Gel filtration chromatography was performed on a Pharmacia FPLC system equipped with a computer controller. Protein elution was monitored using a UV-M II system measuring absorbance at 280 nm wavelength. For small-scale separations, a Superdex 200 HR 10/30 column was employed and sample was loaded using a 1 ml loop. Prior to running the column was equilibrated with 30 ml of PBS and the sample was run at 0.5 ml/min with 1 ml fractions being collected. For large-scale separations, a Superdex 75 or 200 PG 26/60 column was used with a 10 ml superloop. In this case 5 or 10 ml samples were collected and the column was run at 4 ml/min. All separations were performed at room temperature.

Ion exchange chromatography was performed on a Biocad Sprint system (Perkin-Elmer). For cation exchange, a 20 HS or a 50 HS column was employed. For anion exchange, a 10 HQ, 20 HQ or a 50 HQ column was employed. Columns were run using the recommended buffers attached to a 6-way mixer. Small samples (5 - 25 ml) were injected using a 5 ml injection loop. Larger samples (> 100 ml) were injected using one of the buffer lines. 1 ml fractions were collected during the elution phase of the column run. Protein elution was measured by in-line absorbance at 280 nm.

SDS polyacrylamide gel eletrophoresis (SDS-PAGE) was performed using a Bio-Rad Mini-Protean II gel set. Gels were poured prior to use using the following procedure. The gel plate assembly was prepared and checked to ensure against leakage. Then the following mixture was prepared: 12 % acrylamide I bisacrylamide (from a 30 % acrylamide / 0.8 % bisacrylamide stock solution (National Diagnostics)), 0.375 M Tris pH 8.8 (from a 1.5 M stock of the same pH), 0.1 % SDS (from a 10 % SDS stock solution), 0.05 % Ammonium persulphate (from a 10% stock of the same, stored at 4 C) and 0.1 % TEMED (Sigma). The mixture was immediately poured into the gel plate assembly and water-saturated butanol was layered on top to ensure a flat upper surface. After the gel had set (10 - 15 minutes minimum), the stacking gel was mixed as follows. 4 % acrylamide (from stock as before), 0.125 M Tris pH 6.8 (from 0.5 M stock of the same pH), 0.1 % SDS, 0.05 % Ammonium persulphate, and 0.2 % TEMED. The butanol was removed from the surface of the resolving gel by absorption onto a tissue and the stacking gel mixture was poured on top of the resolving gel. A gel comb was immediately inserted taking care to avoid introducing air bubbles into the gel and the stacking gel was allowed to set for a minimum of 5 minutes.

The gel was then assembled into the gel apparatus and running buffer (3 g/L Tris-base, 14.4 g/L glycine, 1 g/L SDS (diluted from a 10x concentrated stock solution) was poured into the apparatus at the anode and the cathode.

After removing the gel comb, the wells were washed out with running buffer to prevent residual acrylamide mixture from setting in the bottom of the wells. Samples were prepared by mixing protein 1:1 with the following mixture: 4 % SDS, 0.125 M Tris pH 6.8, 20 % glycerol, 10 % β-mercaptoethanol, 1 % bromophenol blue (Sigma). Samples were then heated to 95 °C for 2 minutes and cooled prior to loading up to 25 μl into the wells in the stacking gel. Approximately 1 - 10 μg of protein was usually loaded to ensure good staining and running of the gel. After loading, the gels were run at a constant voltage of 200 V for approximately 40 minutes or until the bromophenol blue dye was approximately 5 mm from the end of the gel.

After completing of the electrophoresis, the gels were removed from the apparatus and carefully dropped into a 0.1 % solution of Coomassie R-250 (Sigma) in 10 % acetic acid, 40 % methanol, 50 % water. Gels were then gently agitated for at least 30 minutes prior to destaining in several changes of 10 % acetic acid, 40 % methanol, 50 % water until the gel background was clear. Gels were then stored in water and recorded using a UVP gel documentation system consisting of a light box, a digital camera and a thermal printer.

### Example 1 - Recombinant Soluble TCR

A recombinant soluble form of the heterodimeric TCR molecule was engineered as outlined in Figure 1. Each chain consists of membrane-distal and -proximal immunoglobulin domains which are fused via a short flexible linker to a coiled coil motif which helps stabilise the heterodimer.

The TCR constant domains have been truncated immediately before cysteine residues which *in vivo* form an interchain disulphide bond. Consequently, the two chains pair by non-covalent quaternary contacts, and this is confirmed in Figure 2b. As the Fos-Jun zipper peptide heterodimers are also capable of forming an interchain disulphide immediately N-terminal to the linker used (O'Shea *et al* 1989), the alignment of the two chains relative to each other was predicted to be optimal. Fusion proteins need to be joined in a manner which is compatible with each of the separate components, in order to avoid disturbing either structure.

cDNA encoding alpha and beta chains of a TCR specific for the influenzamatrix protein 58-66 epitope in HLA-A2 was obtained from a Vβ17+ human CTL clone (JM22) by anchored PCR as described previously (Moss *et al* 1991).

Alpha and beta TCR-zipper constructs pJM22α-Jun and pJM22β-Fos were separately constructed by amplifying the variable and constant domain of each chain using standard PCR technology and splicing products onto leucine zipper domains from the eukaryotic transcription factors Jun and Fos respectively (See Figure 1). These 40 amino acid long sequences have been shown to specifically heterodimerise when refolded from synthetic peptides, without the need for a covalent interchain linkage (O'Shea *et al* 1989).

Primers were designed to incorporate a high AT content immediately 3' to the initiation codon (to destabilise mRNA secondary structure) and using *E.coli* codon preferences, in order to maximise expression (Gao *et al*). The spare cysteine in the TCR beta constant domain was mutated to serine to ensure prevention of incorrect disulphide bonding during refolding.

DNA constructs were ligated separately into the *E.coli* expression vector pGMT7. Plasmid digests and DNA sequencing confirmed that the constructs were correct.

In detail the procedures used were as follows.

Expression of TCR zipper chains and purification of denatured inclusion bodies: GFG020 and GFG021, the pGMT7 expression plasmids containing JM22α-Jun and JM22β-Fos respectively were transformed separately into *E. coli* strain BL21pLysS, and single ampicillin-resistant colonies were grown at 37°C in TYP (ampicillin 100μg/ml) medium to $OD_{600}$ of 0.4 before inducing protein expression with 0.5mM IPTG. Cells were harvested three hours post-induction by centrifugation for 30 minutes at 4000rpm in a Beckman J-6B. Cell pellets were resuspended in a buffer containing 50mM Tris-HCl, 25% (w/v) sucrose, 1mM NaEDTA, 0.1% (w/v) NaAzide, 10mM DTT, pH 8.0. After an overnight freeze-thaw step, resuspended cells were sonicated in 1 minute bursts for a total of around 10 minutes in a Milsonix XL2020 sonicator using a standard 12mm diameter probe. Inclusion body pellets were recovered by centrifugation for 30 minutes at 13000rpm in a Beckman J2-21 centrifuge. Three detergent washes were then carried out to remove cell debris and membrane components. Each time the inclusion body pellet was homogenised in a Triton buffer (50mM Tris-HCl, 0.5% Triton-X100, 200mM NaCl, 10mM NaEDTA, 0.1% (w/v) NaAzide, 2mM DTT, pH 8.0) before being pelleted by centrifugation for 15 minutes at 13000rpm in a Beckman J2-21. Detergent and salt was then removed by a similar wash in the following buffer: 50mM Tris-HCl, 1mM NaEDTA, 0.1% (w/v) NaAzide, 2mM DTT, pH 8.0. Finally, the JM22α-Jun and JM22β-Fos inclusion body pellets were dissolved separately in a urea solution (50mM MES, 8M urea, 10mM NaEDTA, 2mM DTT, pH 6.5) for 3 to 4 hours at 4°C.

Insoluble material was pelleted by centrifugation for 30 minutes at 13000rpm in a Beckman J2-21, and the supernatant was divided into 1ml aliquots and frozen at -70°C. Inclusion bodies solubilised in urea were quantitated with a Bradford dye-binding assay (Biorad). For each chain a yield of around 100mg of purified inclusion body was obtained from one litre of culture. Each inclusion body (JM22α-Jun, JM22β-Fos) was solubilised in urea solution at a concentration of around 20mg/ml, and was estimated from gel analysis to be around 90% pure in this form (data not shown).

*Co-refolding of TCR-zipper fusion proteins:*

Initial refolding experiments using a standard refolding buffer (100mM Tris pH 8.5, 1M L-Arginine, 2mM EDTA, 5mM reduced Glutathione, 0.5mM oxidised Glutathione, 0.2mM PMSF) resulted in severe protein precipitation which was dependent upon the presence of the zipper domains. The fact that this phenomenon occurred at concentrations below the dissociation constant of zipper dimerisation (i.e. when most zipper helices are expected to be monomeric) suggested additional forces were stabilising misfolded species. The most likely explanation is that the entirely alpha-helical zipper domains fold first and that their transient heterodimerisation induces interchain aggregation of partially folded intermediates of the more complex immunoglobulin domains. The refolding buffer was therefore altered to include 5M urea in order to prevent hydrophobic interactions between partially folded immunoglobulin domains and allow individual chains to fold completely before heterodimerisation. This step is sufficient to prevent precipitation occurring, and allows correctly folded TCR-zipper heterodimers to assemble with acceptable yields using the following protocol.

Urea-solubilised stocks of TCR-zipper chains JM22α-Jun and JM22β-Fos were renatured by dilution co-refolding. Approximately 30mg (i.e. 1μMole) of each solubilised inclusion body chain was thawed from frozen stocks and a further pulse of DTT (4μmoles/ml) was added to ensure complete reduction of cysteine residues. Samples were then mixed and the mixture diluted into 15ml of a guanidine solution (6 M Guanidine-hydrochloride, 10mM Sodium Acetate, 10mM EDTA), to ensure complete chain denaturation. The guanidine solution containing fully reduced and denatured TCR-zipper chains was then injected into l litre of the following refolding buffer 100mM Tris pH 8.5, 400mM L-Arginine, 2mM EDTA, 5mM reduced Glutathione, 0.5mM oxidised Glutathione, 5M urea, 0.2mM PMSF. The solution was left for 24 hrs. The refold was then dialysed twice, firstly against 10 litres of 100mM urea, secondly against 10 litres of 100mM urea, 10mM Tris pH 8.0. Both refolding and dialysis steps were carried out at 6-8°C.

*Purification of refolded TCR-zipper:*

TCR-zipper JM22zip was separated from degradation products and impurities by loading the dialysed refold onto a POROS 10HQ analytical anion exchange column in seven 200ml aliquots and eluting bound protein with a gradient of 0-400mM NaCl over 50 column volumes using a BioCad workstation (Perseptive Biosystems). Non-covalently associated heterodimer eluted in a single peak at approximately 100mM NaCl. Peak fractions (typically containing heterodimer at a concentration of 100-300μg/ml) were stored at 4°C before being pooled and concentrated. The yield of heterodimer is approximately 15%.

*Characterisation of the refolded TCR-zipper JM22zip:*

The JM22zip heterodimer purified by anion exchange elutes as an approximately 70kDa protein from a Superdex 200 gel filtration sizing column (Pharmacia). It is especially important to include gel filtration steps prior to surface plasmon resonance binding analysis since accurate affinity and kinetic measurements rely on monomeric interactions taking place. In this way, higher order aggregates can be excluded from the soluble protein fraction used for analysis. In particular, aggregates cause artifactually slow association and dissociation rate constants to be detected.

The oxidation state of each chain has been examined by a reducing/nonreducing gel analysis in Fig 2. In the presence of SDS, the non-covalently associated heterodimer is dissociated into alpha and beta chains. If DTT is used in loading buffer, the two chains run either side of the 31kDa marker. In the absence of such denaturants both chains still behave as a single species, but the mobility of each increases, which suggests each chain has formed a single, disulphide-bonded species (Garboczi *et al* 1996).

The antibody reactivity of refolded receptor has been tested using surface plasmon resonance on a Biacore 2000 machine (Biacore). The TCR-zipper JM22z was immobilised to a dextran matrix (CM chip) binding surface at pH 5.5 using standard amine coupling methods. A variable region antibody specific for the beta chain (Vβ17) specifically binds to the immobilised receptor, implying correct conformation.

*Stability:*

The soluble TCRs expressed as alpha-jun and beta-fos leucine zipper fusions are stable over periods of months and are therefore suitable for the detection of specific antigens presented by class I MHC.

**Example 2 - Kinetics and Affinity Study of human TCR-viral peptide-MHC**

*Specific binding of refolded TCR-zipper to peptide-MHC complexes:*

A surface plasmon resonance biosensor (Biacore) was used to analyse the binding of a TCR-zipper (JM22zip, specific for HLA-A2 influenza matrix protein M58-66 complex) to its peptide-MHC ligand (see Fig. 3). We facilitated this by producing single pMHC complexes (described below) which can be immobilised to a streptavidin-coated binding surface in a semi-oriented fashion, allowing efficient testing of the binding of a soluble T-cell receptor to up to four different pMHC (immobilised on separate flow cells) simultaneously. Manual injection of HLA complex allows the precise level of immobilised class I molecules to be manipulated easily.

Such immobilised complexes are capable of binding both T-cell receptors (see Fig. 3) and the coreceptor CD8$\alpha\alpha$, both of which may be injected in the soluble phase. Specific binding of TCR-zipper is obtained even at low concentrations (at least 40$\mu$g/ml), implying the TCR zipper is relatively stable. The pMHC binding properties of JM22z are observed to be qualitatively and quantitatively similar if TCR is used either in the soluble or immobilised phase. This is an important control for partial activity of soluble species and also suggests that biotinylated pMHC complexes are biologically as active as non-biotinylated complexes.

*Preparation of chemically biotinylated HLA complexes:*

Methods for the production of soluble, recombinant single peptide class I HLA complexes have already been described (Garboczi *et al* 1992). These have been modified in order to produce HLA complexes which have $\beta$-2-microglobulin domains chemically biotinylated and may therefore be immobilised to a streptavidin coated binding chip and used for surface plasmon binding studies.

$\beta$-2-microglobulin was expressed and 40mg refolded in a standard refolding buffer (100mM Tris pH 8.0, 400mM L-Arginine, 2mM EDTA, 5mM reduced Glutathione, 0.5mM oxidised Glutathione, 0.1mM PMSF) essentially as described (Garboczi *et al* 1992). After an optional gel filtration step, protein was exchanged to 0.1M Sodium Borate pH 8.8, and finally concentrated to 5-10mg/ml. $\beta$-2-microglobulin was also quantitated using the Bradford assay (Biorad). A 5 molar excess of biotin hydroxysuccinimide (Sigma) was added from a stock made up at 10mg/ml in DMSO. The reaction was left for 1 hour at room temperature, and stopped with 20$\mu$l of 1M Ammonium Chloride/250$\mu$g of biotin ester used. Refolded HLA complex was separated from free biotin and free biotinylated beta-2-microglobulin using a Superdex 200 gel filtration sizing column (Pharmacia). Streptavidin was immobilised by standard amine coupling methods.

*Conclusions:*

Thus, the protein refolding methods described in Example 1 produce a stable, correctly folded, functional recombinant receptor fusion protein which is suitable for biophysical analysis using an optical biosensor. This has provided a reagent used to carry out a detailed affinity and kinetic analysis of a human TCR-pMHC interaction. The effects of T-cell coreceptor-MHC and TCR-pMHC interactions on each other have also been studied. The recombinant techniques used are applicable in principle to both murine and human TCRs, both class I and class II - restricted, and will enable similar analyses of a range of TCRs. This would allow various questions to be addressed, such as the span of TCR affinities within an antiviral response, the properties of dominantly selected receptors and the kinetic requirements for receptor triggering. The methods also provide a way of verifying the ligand specificity of a TCR prior to crystallization trials, and may also have implications for the recombinant production of other cell surface receptors.

**Example 3 - Biotinylation and tetramerisation of soluble T-cell receptors**

2.5 ml purified soluble TCR prepared as described in Example 1 (~ 0.2 mg/ml) was buffer exchanged into biotinylation reaction buffer (10 mM Tris pH 8.0, 5 mM NaCl, 7.5 mM MgCl$_2$) using a PD-10 column (Pharmacia). The eluate (3.5 ml) was concentrated to 1 ml using a centricon concentrator (Amicon) with a 10 kDa molecular weight cut-off. This was made up to 5mM with ATP added from stock (0.1 g/ml adjusted to pH 7.0). A cocktail of protease inhibitors was added: leupeptin, pepstatin and PMSF (0.1 mM), followed by 1 mM biotin (added from 0.2M stock) and 5 $\mu$g/ml enzyme (from 0.5 mg/ml stock). The mixture was then incubated overnight at room temperature. Excess biotin was removed from the solution by dialysis against 10 mM Tris pH 8.0, 5mM NaCl (200 volumes, with 2 changes at 4°C). The protein was then tested for the presence of bound biotin by blotting onto nitrocellulose followed by blocking with 5% skimmed milk powder, and detection using streptavidin-HRP conjugate (Biorad). Tetramerisation of the biotinylated soluble TCR was with either extravidin-RPE or extravidin-FITC conjugate (Sigma). The concentration of biotin-soluble TCR was measured using a Coomassie binding protein assay (Pierce), and a ratio of extravidin conjugate to soluble TCR of 0.224 mg / mg TCR was calculated to achieve saturation of the extravidin by biotinylated TCR at a ratio of 1: 4. The extravidin conjugate was added in aliquots of 1/10th of the total added, on ice, for at least 15 minutes per aliquot (to ensure saturation of the extravidin). Soluble TCR tetramers were stored at 4°C in the dark. The tetramers are extremely stable over a period of months.

### Example 4 - Molecular cloning of T cell receptor genes from T cell lines or T cell clones of known specificity.

The methods and procedures for molecular cloning of TCR genes from cells is identical for all $\alpha$ chains and for all $\beta$ chains, respectively, and are therefore only described in this example.

A suitable number of T cells, typically 1-5 million, were lysed in Lysis Buffer from the 'mRNA Capture Kit' (Boehringer Mannheim). mRNA was isolated with kit reagents by hybridising biotinylated oligo-dT to the poly-A tails of the mRNA. The hybridised complexes were then captured by binding of biotin to a PCR tube coated with streptavidin. Following immobilisation of the mRNA in the PCR tube, cDNA was generated using AMV reverse transcriptase (Stratagene) as described (Boehringer Mannheim manual for 'mRNA Capture Kit').

With the cDNA still immobilised, a poly-G tails were generated at the 3' ends using the Terminal Transferase enzyme (Boehringer Mannheim). PCR reaction mix was then added, including the high fidelity thermostable polymerase *pfu* (cloned, Stratagene), which was used in order to minimise the risk of errors in the PCR products. PCR reactions were performed using a poly-C 'anchor primer' (Figure 4A) and $\alpha$ or $\beta$ chain specific primers (Figures 4B and C, respectively) annealing in the respective TCR constant regions. PCR reactions of 30 cycles of denaturation at 95°C for 1 minute, annealing at 50°C for 1 minute, and extensions at 72°C for 5 minutes were performed to amplify TCR gene fragments.

PCR products were ligated into a Bluescript sequencing vector (pBluescript II KS-, Stratagene) using the Xhol and Xmal restriction enzyme sites contained in the PCR primers (all enzymes from New England Biolabs). Following transfection of the ligation mixes in the E.coli strain XL-1Blue, several clones for each chain were selected for DNA sequencing which was performed on an ABI 377 Prism automatic sequencer using BigDye™ terminators (Applied Biosystems Inc.).

### Example 5 - Molecular cloning of DNA fragments encoding the 40 amino acid coiled-coil ('leucine zipper') regions of c-jun and c-fos.

DNA fragments encoding the 40 amino acid coiled-coil ('leucine zipper') regions of c-jun and c-fos were generated by PCR reactions using human cDNA as template and the primers shown in Figure 5. PCR reactions were carried out in reaction buffer including cloned *pfu* polymerase (Stratagene) for 30 cycles consisting of denaturation at 95°C for 1 minute, primer annealing at 58°C for 1 minute, and extension at 72°C for 2 minutes.

The *c-jun* and *c-fos* fragments were ligated into pBluescript II KS-(Stratagene) using the unique Xhol and Xmal restriction sites to obtain constructs pBJ107 and pBJ108, respectively (Figure 6). The DNA sequences of the *c-jun* and *c-fos* fragments were verified by DNA sequencing performed on an ABI 377 Prism automatic sequencer using BigDye™ terminators (Applied Bioystems Inc.).

The sequenced *c-jun* and *c-fos* fragments were then subcloned, using the unique Xmal and BamHI restriction sites, into the polylinker region of the T7 polymerase expression vector, pGMT7 (Studier, Rosenberg et al. 1990).

### Example 6 - Design of TCR-leucine zipper fusion proteins for the production of stable, soluble TCRs

Attempts to co-refold extracellular fragments of TCR $\alpha$ and $\beta$ chains, truncated so that they contained the cysteine residue which *in vivo* forms a disulphide bond, produced limited success (data not shown, see Example 9 for expression methods and general methods and materials for refolding conditions). However, when the TCR $\alpha$ and $\beta$ chains were truncated immediately before, that is on the N-terminal side of, the cysteine residue forming the interchain disulphide bond, analytical chromatography on a Superdex G-75 column (Pharmacia) indicated that a small fraction of protein, approximately 1-2% of the amount used in the refolding reaction, had refolded into a complex of the expected molecular size for the truncated $\alpha/\beta$ heterodimer (see also (Garboczi, Utz et al. 1996) for reference to method).

Because incorrect disulphide bond formation can cause irreversible misfolding of protein during *in vitro* refolding, the probabilities for this to happen were sought to be minimised by mutating a cysteine residue in the TCR $\beta$ constant region which is unpaired in the cellular TCR. The cysteine residue is substituted for a serine or an alanine reside. The synthetic DNA primers used for these mutation steps are shown in Figure 7. Co-refolding of TCR $\alpha$ and mutated $\beta$ chains, both truncated immediately before the cysteine residue which forms the interchain disulphide bond, showed a dramatic improvement in yields of heterodimer, the protein fraction of correct molecular weight typically constituting 15-30% of total protein. However, when these soluble TCRs were stored overnight, analysis of the protein showed that the fraction with a molecular weight corresponding to the heterodimeric TCR had split into two peaks of molecular weight corresponding to the monomeric TCR α and β chains. Similar observations were made upon dilution of the soluble TCRs, indicating that α/β chain stability was low and insufficient for analyses which would require a timespan longer than a limited number of hours or dilution of the protein. In conclusion, these methods for producing soluble TCR only generated receptor with extremely limited stability.

To improve TCR α/β chain stability, and to potentially aid heterodimer formation during refolding, the TCR chains were fused to the 'leucine zipper' domains of *c-jun* and *c-fos* which are known preferentially to form heterodimers (O'Shea, Rutkowski et al. 1989; Schuermann, Hunter et al. 1991; O'Shea, Rutkowski et al. 1992; Glover and Harrison 1995). Two designs for the fusion TCRs were tested.

In one, the leucine zippers were fused just after, that is C-terminal to, the cysteine residues forming the interchain disulphide bond in the TCR α and β chains. As the *c-jun* and *c-fos* leucine zipper peptides are also capable of forming an interchain disulphide immediately N-terminal to the linker used (O'Shea, Rutkowski et al. 1989), the alignment of the two chains relative to each other, and to the interchain disulphide bond, was predicted to be optimal.

In the other design, the leucine zippers were fused just before, that is N-terminal to, the cysteine residues forming the interchain disulphide bond in the TCR α and β chains (Figure 8). Thus, in the second design the cysteine residues are omitted from the recombinant receptor.

In refolding experiments with TCR-zipper (TCR-z) chains of these designs, it was found that the yield of heterodimeric, soluble receptor was better when the cysteine residues forming the interchain disulphide bond were omitted from the TCR α and β chains, as in the design shown in Figure 8.

**Example 7 - Construction of DNA expression vectors for TCR-leucine zipper proteins.**

This example describes the construction of expression vectors for the α and β chains of five TCRs. The strategy and design described should be adaptable to any human or animal TCR genes. Although the five TCRs described here are all restricted by MHC class I epitopes, the methods could be identically employed for the cloning and construction of expression vectors for MHC class II restricted TCRs. All vectors express protein aimed for refolding soluble TCRs according to the design shown in Figure 9, with the exception that two TCRs were expressed with a biotinylatable tag sequence at the C-terminus (see below and Figures 17, 18, and 19). The cloning strategies are identical for all TCR α and β chains, respectively.

The extent of the leader, or signal, peptide sequences of TCR α and β chains were predicted from analyses of the sequence data obtained from plasmids containing TCR anchor PCR products (see Example 4). On this basis, 5' primers for generating PCR fragments for the expression of TCR chains without leader sequences were designed (Figure 9). All 5' primers encode a methionine residue just prior to the mature TCR protein sequences in order to allow translation in *E.coli*. Silent mutations, substituting C or G bases for A or T (Figure 9), were introduced in a number of the 5' proximal codons of the genes in order to decrease the tendency for secondary mRNA structure formations which could adversely inhibit expression levels in *E.coli* (PCT/GB 98/03235; (Gao, Tormo et al. 1997; Gao, Gerth et al. 1998).

The genes encoding the Vα0.2 and the Vβ17 chains of the human JM22 Influenza Matrix peptide-HLA-A0201 (peptide sequence GILGFVFTL) restricted TCR, the human Vα23 and the Vβ5.1 chains of the 003 HIV-1 Gag peptide-HLA-A0201 (peptide sequence SLYNTVATL) restricted TCR, and the murine Vα4 and Vβ11 chains of the F5 NP peptide-H2-Db (peptide sequence ASNENMDAM) were amplified by PCR using plasmids containing TCR anchor PCR products generated as described in Example 6. The genes for the human A6 (Vα2.3/Vβ12.3) and B7 (Vα17.2/Vβ12.3) TCRs which are specific the HTLV-1 Tax peptide presented by HLA-A0201 (peptide sequence LLFGYPVYV), were obtained in plasmid form (Garboczi, Utz et al. 1996; Ding, Smith et al. 1998) which were used for the generation of PCR products for the construction of expression vectors for these TCR chains. The genes for these TCRs were cloned into expression vectors that contained the sequence for a *c-fos* leucine zipper-biotinylatable tag fusion fragment (see Example 8).

PCR reactions were performed with cloned *pfu* polymerase at standard buffer conditions (Stratagene) and with 25 cycles of denaturation at 95°C for 1 minute, primer annealing at 60°C for 1 minute, and extensions at 72°C for 6 minutes. The PCR products were restriction digested with the enzymes NdeI and XmaI and ligated into the pGMT7 vectors containing the *c-jun* (TCR α chains) and *c-fos* (TCR β chains) inserts (see Example 5).

Figures 10-19 show the sequences of the TCR-z inserts and the predicted protein sequences expressed by the pGMT7 vectors. Figure 20 shows the sequence of the A6 TCR β chain containing a mutation in the constant region but which did not detectably affect the folding and function of the soluble TCR (see Examples 9 and 10).

**Example 8 - Construction of DNA vectors for the expression of TCR β chains fused to a *c-fos* leucine zipper-blotInylatable fragment.**

In order to enable soluble TCRs to be immobilised or to allow detection or attachments to the receptor, it would be useful if the protein could be produced with a further functional fusion component. This could allow the soluble TCR to be derivatised, such as to be produced as multimers, or allow detection with high sensitivity, or attach other functions to the receptor/receptor complexes.

This example demonstrates the construction of expression vectors for TCR β chains onto which is engineered a fusion polypeptide which can be specifically biotinylated in *E.coli in vivo* or with the enzyme BirA *in vitro* (Barker and Campbell 1981; Barker and Campbell 1981; Howard, Shaw et al. 1985; Schatz 1993; O'Callaghan, Byford et al. 1999). As shown in Examples 10 and 11, these soluble TCR fusions can be expressed and refolded together with α chain in an identical manner and with similar yields to the TCR β chain which is not fused to the 'biotinylation tag' (BT-tag). These results demonstrate that the soluble TCR described herein is likely to be suitable for expression with a multitude of different polypeptides as fusion partners.

T Cell Receptor p-chains were sub-cloned into a pGMT7 expression vector with a biotin-tag sequence C-terminal to the *fos* leucine zipper sequence as follows:

start - TCR β-chain - fos zipper - biotin-tag - stop

The exact sequence of the ends of the constructs was as follows (see also Figure 21):

Linker →|fos zipper →| BamHI|← linker →|←biotin tag

Two approaches were used to produce soluble TCRs with the biotin tag. In the case of the human JM22 Influenza Matrix peptide-HLA-A0201 restricted TCR, the cloned β-chain-*c-fos* leucine zipper fusion was modified at the 3'-end using the synthetic DNA primer shown in Figure 22 to introduce a BamHI site instead of a HindIII site using a standard PCR reaction with *pfu* polymerase (Stratagene).

The original 5' primer (see Figure 9) containing an NdeI site was used as the forward primer. The PCR product produced was cloned into a modified pGMT7 vector containing the biotin-tag sequence (Figure 21) to form the construct outlined above. This plasmid is known as JMB002.

The cloned TCR specific for the HLA-A0201 restricted HTLV-1 epitope LLFGYPVYV, known as the A6 tax TCR (Vα2.3/ Vβ12.3) was truncated using PCR with the forward and reverse primers shown in Figure 9. This TCR β-chain was cloned into the NdeI and XmaI sites of a pGMT7 vector (JMB002) containing the *c-fos*-BT fragment.

After construction of the fusion expression vectors, DNA sequencing was carried out to ensure no mistakes had been introduced during the subcloning procedure (all sequencing was carried out in the Biochemistry Dept. DNA Sequencing Facility, Oxford University using an ABI 377 Prism sequencer and ABI BigDye fluorescent terminators). It emerged that there were two errors in the tax TCR β-chain compared with the published sequence and upon further investigation, we discovered that these were both present in the original plasmid we had received. Since both of these errors were 3' of a unique Bsu361 site in the TCR β-chain, this was used to clone into the (correct) JMB002 plasmid. Both versions of the tax TCR β-chain were expressed and refolded with α-chain and compared using Biacore. Both versions of the protein specifically bound to the tax peptide - MHC class I molecules with similar apparent affinities (see Example 17). In subsequent experiments, only the correct version of the β-chain was used.

## Example 9 - Expression of TCR chains in *E.coli* and purification of inclusion bodies

TCR α and β chains were expressed separately in the *E.coli* strain BL21DE3pLysS under the control of the vector pGMT7 in TYP media, using 0.5mM IPTG to induce protein production when the optical density (OD) at 600nm reached between 0.2 and 0.6. Induction was allowed to continue overnight and the bacteria were harvested by centrifugation at 4000 rpm in a Beckman J-6B centrifuge.

Bacterial cell pellets were then resuspended in 'lysis buffer' (10 mM Tris pH 8.1, 10 mM EDTA, 150 mM NaCl, 2 mM DTT, 10% glycerol). The mixture was cooled on ice and the following were added: 20 µg/ml lysozyme, 10 mM MgCl$_2$, and 20 µg/ml DNase I, followed by incubation on ice for a minimum of an hour.

The mixture was then sonicated using a 12mM probe sonicator (Milsonix XL2020) at full power for 5 bursts of 30s with intervals of 30s to allow the mixture to cool down. Temperature was maintained during this procedure by use of an ice-water mixture. The mixture was then diluted with 5 volumes of 'Triton wash buffer' (50 mM Tris pH 8.1, 0.5% Triton X-100, 100 mM NaCl, 0.1% sodium azide, 10 mM EDTA, 2 mM DTT). After incubation on ice for a minimum of 1 hour, the mixture was then centrifuged at 3,500 rpm in a Beckman GS-6R centrifuge and the supernatant was discarded. The pellet was resuspended in 'Resuspension buffer' (50 mM Tris pH 8.1, 100 mM NaCl, 10 mM EDTA, 2 mM DTT) using a small plastic disposable pipette. The mixture was then centrifuged at 8,000 rpm in a Beckman J2-21 centrifuge and the supernatant discarded. The pellet was then resuspended in 'Guanidine buffer' (50 mM Tris pH 8.1, 6.0 M Guanidine-HCl, 100 mM NaCl, 10 mM EDTA, 10 mM DTT) using a hand-operated homogeniser. After low-speed centrifugation to remove insoluble material, the supernatant was aliquotted and stored at -70 °C. An approximate yield of 100 mg per litre of bacterial culture was routinely obtained.

SDS-PAGE analysis of the purified inclusion body preparation was achieved by diluting 2 µl of inclusion body preparation in Guanidine buffer with SDS-PAGE sample buffer followed by heating to 100 °C for 2 minutes. Samples were loaded onto the gel while still warm to prevent the Guanidine/SDS mixture from precipitating during loading. Inclusion body protein purified in this way was judged to be approximately 90% pure by Coomassie staining of SDS-PAGE performed in this way (see Figure 23).

**Example 10 - Refolding and purification of the TCRz heterodimer.**

Urea-solublised proteins in equal proportions were further denatured in 'guanidine buffer' (6 M guanidine-HCl, 10 mM sodium acetate pH 5.5, 10 mM EDTA, 2 mM DTT) at 37 °C. The mixture of proteins was injected into ice-cold refolding buffer (100 mM Tris pH 8.1, 0.4 M L-Arginine-HCl, 5.0 M Urea, 5 mM reduced glutathione, 0.5 mM oxidised glutathione) at a total protein concentration of 60 mg/L ensuring rapid mixing. After incubation on ice for at least 5 hours to allow refolding, the mixture was dialysed against 10 volumes of demineralised water for 24 hours and then against 10 volumes of 10 mM Tris pH 8.1 for 24 hours.

The dialysed refolded protein was then filtered to remove aggregated protein (produced as a by-product during the refolding) through a 0.45μ nitro-cellulose membrane (Whatman). Purification of the biotin-tagged soluble TCR was then performed by loading onto a POROS 20HQ column run on a Biocad Sprint system. Approximately 500 ml of refolded protein solution could be loaded per run and elution of the protein was achieved by a gradient of sodium chloride in Bis-Tris-Propane buffer pH 8.0. The protein eluted at approximately 100 mM sodium chloride and the relevant fractions were immediately chilled on ice and protease inhibitor cocktail was added. Fractions were analysed by Coomassie-stained SDS-PAGE.

**Example 11 - Refolding and purification of the TCRz heterodimer with a biotinylatable βchain.**

Biotin-tagged TCR β-chains were mixed with an equal quantity of α-chain expressed and purified as for the soluble T cell receptor. Heterodimeric TCRz-β-BT was refolded according to identical procedures as described in Example 10 for TCRz (see Figure 26).

**Example 12 - Biotinylation of biotin-tagged soluble TCRz-BT**

Protein-containing fractions were concentrated to 2.5 ml using 10K-cut-off centrifugal concentrators (Ultrafree, Millipore). Buffer was exchanged using PD-10 desalting columns equilibrated with 10 mM Tris pH 8.1, 5mM NaCl, further protease inhibitor cocktail was added, and the protein was concentrated to ~1ml using centrifugal concentrators again. To this 1ml of biotin-tagged soluble TCR the following were added: 7.5 mM $MgCl_2$, 5 mM ATP (pH 8.0), 1 mM biotin, 2.5 μg/ml BirA biotinylation enzyme. The biotinylation reaction was then allowed to proceed at room temperature (20-25 °C) overnight.

Enzymatically biotinylated soluble TCR was then separated from residual unreacted biotin by gel filtration on a Superdex 200 HR column (Pharmacia) run on a Pharmacia FPLC system (see Figure 27). The column was equilibrated with PBS and 1ml fractions were collected which were immediately chilled on ice and protected with protease inhibitor cocktail again. Protein concentration was estimated using a Coomassie-binding assay (Pierce) and the biotinylated protein was then stored at 4 °C for up to a month or at -20 °C for longer periods.

The efficacy of the biotinylation reaction was checked using Western blotting of the biotinylated protein. An SDS-PAGE gel was run using the methods described before, but instead of staining, the gel was blotted onto a PVDF membrane (Bio-Rad) using a SemiPhor semi-dry electoblotting apparatus (Hoefer). The blotting stack comprised of 6 layers of filter paper (Whatman 4M) cut to the size of the gel and soaked in transfer buffer (25 mM Tris base, 150 mM glycine) followed by the PVDF membrane which was pre-wetted with methanol and then soaked in transfer buffer, followed by the gel which was gently agitated in transfer buffer for 5 minutes, followed by 6 more layers of soaked filter paper. The stack was gently compressed using a test-tube to roll out any air-bubbles and approximately 10 ml of additional transfer buffer was added to aid conduction. The cathode was placed on top of the stack and current was passed through the apparatus at a constant current of 50 mA for 1 hour. The membrane was then incubated in a 2 % solution of gelatin (Bio-Rad) in PBS-T buffer (PBS + 0.05% Tween-20) for > 1 hour at room temperature with gentle agitation. Overnight incubations also included 0.01% sodium azide to inhibit bacterial growth. The membrane was washed with several (4-5) changes of PBS-T followed by staining with avidin-HRP conjugate (Sigma) diluted 1:1000 in a 1 % solution of gelatin in PBS-T for >30 minutes at room temperature with gentle agitation. The membrane was then washed with several (4-5) changes of PBS-T prior to detection with Opti-4CN (Bio-Rad). This is a reagent with reacts in the presence of HRP to form an insoluble blue dye which stains the membrane in the place where relevant protein is present as indicated by the presence of bound HRP. When avidin-HRP conjugate is used to stain, this therefore indicates the presence of a biotin-containing protein.

Figure 28 shows a blot performed in such a way on several biotinylated TCRs. The standards run on this blot were biotinylated broad range molecular weight markers (Bio-Rad). The blot clearly shows that a high level of biotinylation of the TCRs containing the biotinylation tag which have been reacted with the BirA enzyme

**Example 13 - Production of biotinylated soluble MHC-peptide complexes**

Biotinylated soluble MHC-peptide complexes can be produced as described in Example 2.

**Example 14 - Assay for specific binding between soluble TCR and MHC-flu-peptide**

The soluble TCR molecule, JM22z, is specific for HLA-A2 MHC molecules presenting an immuno dominant antigen consisting of amino acid residues 58-66 (GILGFVFTL) of the influenza matrix protein. The cloning, expression, and purification of JM22z is described in Examples 4, 7, 9 and 10 and in Figures 24 and 25. The interactions between JM22z and its ligand/ MHC complex (HLA-A2-flu) or an irrelevant HLA-A2 peptide combination, the production of which is described in Example 13, were analysed on a Biacore 2000™ surface plasmon resonance (SPR) biosensor. SPR measures changes in refractive index expressed in response units (RU) near a sensor surface within a small flow cell, a principle that can be used to detect receptor ligand interactions and to analyse their affinity and kinetic parameters. The probe flow cells were prepared by immobilising the individual HLA-A2-peptide complexes in separate flow cells via binding between the biotin cross linked onto $\beta 2m$ and streptavidin which had been chemically cross linked to the activated surface of the flow cells. The assay was then performed by passing JM22z over the surfaces of the different flow cells at a constant flow rate, measuring the SPR response in doing so. Initially, the specificity of the interaction was verified by passing 28 $\mu M$ JM22z at a constant flow rate of 5 $\mu l$ min$^{-1}$ over three different surfaces; one coated with 2800 RU of HLA-A2-flu, the second coated with 4200 RU of HLA-A2 folded with an irrelevant peptide from HIV reverse transcriptase (HLA-A2-pol: ILKEPVHGV), and the third coated with 4300 RU of CD5 (Fig. 29a inset). Injections of soluble JM22z at constant flow rate and different concentrations over HLA-A2-pol were used to define the background resonance (Fig. 29a). The values of these control measurements were subtracted from the values obtained with HLA-A2-flu (Fig. 29b) and used to calculate binding affinities expressed as the dissociation constant, Kd (Fig. 29c). The Kd of JM22z and the relevant MHC molecule was determined to be 15 $\pm$ 4 $\mu M$ (n=7) at 37 °C and 6.6 $\pm$2 $\mu M$ (n=14) at 25 °C. Determination using immobilised TCR in the probe flow cell and soluble MHC-peptide complex gave a similar Kd of 5.6 $\pm$ 4 $\mu M$ (n=3) at 25 °C. The on-rate of the interaction was determined to be between 6.7 x 10$^4$ and 6.9 x 10$^4$ M$^{-1}$s$^{-1}$ at 37 °C while the off-rate was 1.1 s$^{-1}$ (Willcox, Gao et al. 1999).

**Example 15 - Assay for specific binding between soluble murine TCR and murine MHC H2-D$^b$-NP**

In this experiment, we used a murine TCR, F5, specific for a peptide derived from the influenza virus nucleoprotein (aa.366-374: ASNENMDAM) presented by the murine H2-D$^b$ MHC molecule (H2-D$^b$-NP). The MHC heavy chain gene used was slightly modified in the sense that it encoded only amino acids 1-280 of the native protein plus a 13-amino acid sequence recognised by the BirA enzyme. The resulting protein can be biotinylated enzymatically (Schatz 1993; O'Callaghan, Byford et al. 1999). SPR analysis on the Biacore 2000™ SPR biosensor using this soluble TCR specific for immobilised H2-D$^b$-NP showed that it bound specifically to the ligand MHC-peptide combination (data not shown).

**Example 16 - Comparison of binding of biotinylated soluble tax-TCR with biotinylated soluble mutant tax-TCR**

Biotinylated soluble tax-TCRs were prepared as in Examples 9-11 and Biacore 2000 analysis was performed as in Example 14 using biotinylated pMHC complexes refolded with either influenza matrix peptide (GILGFVFTL) or HTLV tax 11-19 peptide (LLFGYPVYV). Biotinylated soluble TCRs were flowed over all cells at 5 $\mu l$/minute for a total of 1 minute. Figure 30 shows the binding of firstly the biotinylated soluble tax-TCR and then the biotinylated soluble mutant tax-TCR to HTLV tax 11-19 peptide-MHC complex (A). Neither the wild-type nor the mutant tax-TCR showed binding to either the influenza matrix peptide-MHC complex (B/C) or OX68 monoclonal antibody control (D). Therefore, we conclude that both the wild-type and the mutant biotinylated soluble TCRs clearly bind effectively and specifically to the tax-pMHC complex and show very little difference in the degree of binding.

**Example 17 - Analysis of simultaneous TCR- and CD8 co-receptor binding to immobilised MHC peptide complex**

CD8 and CD4 are surface glycoproteins believed to function as coreceptors for TCRs by binding simultaneously to the same MHC molecules as the TCR. CD8 is characteristic for cytotoxic T cells and binds to MHC class I molecules while CD4 is expressed on T cells of the helper lineage and binds MHC class II molecules. CD8 is a dimer consisting of either two identical $\alpha$-chains or of an $\alpha$- and a $\beta$-chain. The homodimeric $\alpha\alpha$-CD8 molecule was produced as described (PCT/GB98/03235; (Gao, Tormo et al. 1997; Gao, Gerth et al. 1998). In this example, we describe the simultaneous binding of soluble TCR and CD8 molecules to immobilised HLA-A2-flu complex. As seen in Figure 31A, the binding response was simply additive. Subtracting the values of the TCR response (open circles) from the values of the combined response(closed circles) gave values (open squares) very close to the value of the response of 120 μM CD8 alone (open triangles). Figure 31 B shows that the kinetics of the TCR-MHC-peptide interaction was unaffected by simultaneous CD8 binding. The observed additive biding indicate that TCR and CD8 bind the MHC peptide complex at separate interfaces. The example also illustrates that in some cases specific binding of one molecule will not influence specific binding of another molecule, a situation most likely to be different for other combinations of molecules.

**Example 18 - Expression, refolding and site-specific biotinylation of soluble α/β TCR**

a) Engineering of TCR α and β chains.

A recombinant soluble form of the heterodimeric TCR molecule was engineered as outlined in Figure 36. Each chain consists of membrane-distal and -proximal immunoglobulin domains which are fused via a short flexible linker to a coiled coil motif which helps stabilise the heterodimer.

Figures 32 to 34 and 38 to 41 show the DNA coding sequences and corresponding amino acid sequences for various TCR alpha and beta chains from TCR having different specificities. This example concentrates on the TCR represented by the sequences of figures 32 to 34 but the methods disclosed can be similarly performed using the TCRs given in figures 38 to 41.

The TCR constant domains have been truncated immediately before cysteine residues which *in vivo* form an interchain disulphide bond. Consequently the two chains pair by non-covalent quaternary contacts. As the Fos-Jun zipper peptide heterodimers are also capable of forming an interchain disulphide immediately N-terminal to the linker used (O'Shea *et al* 1989), the alignment of the two chains relative to each other was predicted to be optimal. Fusion proteins need to be joined in a manner which is compatible with each of the separate components, in order to avoid disturbing either structure.

cDNA encoding alpha and beta chains of a TCR specific for the influenza-matrix protein 58-66 epitope in HLA-A2 was obtained from a Vβ17+ human CTL clone (JM22) by anchored PCR as described previously (Moss *et al* 1991).

Alpha and beta TCR-zipper constructs pJM22α-Jun and pJM22β-Fos were separately constructed by amplifying the variable and constant domain of each chain using standard PCR technology and splicing products onto leucine zipper domains from the eucaryotic transcription factors Jun and Fos respectively. These 40 amino acid long sequences have been shown to specifically heterodimerise when refolded from synthetic peptides, without the need for a covalent interchain linkage (O'Shea *et al* 1989).

Primers were designed to incorporate a high AT content immediately 3' to the initiation codon (to destabilise mRNA secondary structure) and using *E.coli* codon preferences, in order to maximise expression (Gao *et al* 1998). The spare cysteine in the TCR beta constant domain was mutated to serine to ensure prevention of incorrect disulphide bonding during refolding.

The fused DNA and protein sequences are indicated in Figures 32 and 33. In order to enable the site-specific biotinylation of the β chain of this TCR a DNA sequence encoding a so-called "biotin-tag" was engineered into the 3' end of the gene expressing soluble Vβ17. The following PCR primers were employed for the engineering of this DNA construct:

5'-GCTCTAGACATATGGGCCCAGTGGATTCTGGAGTCAC-3'

and

5'-
GGGGGAAGCTTAATGCCATTCGATTTTCTGAGCTTCAAAAATATCGTTCAGACCACCAC
CGGATCCGTAAGCTGCCAGGATGAACTCTAG-3'.

The resulting PCR product was digested with restriction enzymes Ndel and HindIII (New England Biolabs) and ligated with T4 DNA ligase (New England Biolabs) into the vector pGMT7 (Studier *et al.,* 1990). Figure 3 shows the DNA sequence of the insert in this construct and the deduced protein sequence.

b) Expression of TCR chains.

Expression and refolding of a TCR with specificity for the Influenza virus Matrix peptide presented by HLA-A*0201 was carried out as follows:

TCR α and β chains were expressed separately in the *E.coli* strain BL21DE3pLysS under the control of the vector pGMT7 in TYP media (1.6% bacto-tryptone, 1.6% yeast extract, 0.5% NaCl, 0.25% K2HPO4). Expression was induced in mid-log phase with 0.5 mM IPTG and, after 3-5 hours, bacteria were harvested by centrifugation. The bacterial cells were lysed by resuspension in 'lysis buffer' (10 mM EDTA, 2 mM DTT, 10 mM Tris pH 8, 150 mM NaCl, 0.5 mM PMSF, 0.1 mg/ml lysozyme, 10% glycerol) followed by addition of 10 mM MgCl2 and 20 ug/ml DNaseI, incubation for 20 minutes on ice, and sonication using a probe sonicator in 10x bursts of 30 seconds. The protein, in inclusion bodies, was then purified by several washes (usually 3) of 'Triton buffer' (0.5% Triton X-100, 50 mM Tris pH8, 100 mM NaCl, 0.1% sodium azide, 10 mM EDTA, 2 mM DTT) using centrifugation at 15,000 rpm for 20 minutes to pellet the inclusion bodies and a 'dounce' homogeniser to resuspend them. Detergent was removed from the preparation with a single wash of 50 mM Tris pH 8, 100 mM NaCl, 10 mM EDTA, 2 mM DTT and the protein was solubilised with 'urea buffer' (20 mM Tris pH 8, 8 M urea, 10% glycerol, 500 mM NaCl, 10 mM EDTA, 2 mM DTT). After end-over-end mixing overnight at 4°C, the solution was clarified by centrifugation, and the solubilised protein was stored at -70°C. The protein concentration was measured by a Coomassie-binding assay (Pierce).

c) Refolding of the TCR.

Urea-solublised protein in equal proportions was further denatured in 'guanidine buffer' (6 M guanidine-HCl, 10 mM sodium acetate pH 5.5, 10 mM EDTA, 2 mM DTT) at 37 C. This solution was added to refolding buffer (5 M urea, 100 mM Tris pH 8, 400 mM L-arginine, 5 mM reduced glutathione, 0.5 mM oxidised glutathione, 0.1 mM PMSF) on ice ensuring rapid mixing. After >12 hours at 4°C, the solution was dialysed against 10 volumes of water, then 10 volumes of 10 mM Tris pH 8, 100 mM urea. The protease inhibitor PMSF was added at all stages to minimise proteolytic loss of the biotinylation tag on the TCR.

d) Purification of the TCR.

The dilute solution of the TCR was filtered through a 0.45 micron filter to remove aggregated protein and was then loaded onto a POROS 10HQ column. The refolded TCR was eluted with a gradient of sodium chloride in 10 mM Tris pH 8 and 1ml fractions were collected and analysed by SDS-PAGE (see Figure 36). Fractions containing TCR were pooled and concentrated to 1ml using a 30 kDa cut-off centrifugal concentrator.

e) Biotinylation of the TCR.

The 1ml of TCR solution was made up to 7.5 mM ATP using buffered ATP, 5 mM MgCl2, 1 mM biotin, and a cocktail of protease inhibitors was added which included PMSF, leupeptin, and pepstatin. Finally, the enzyme BirA was added to a final concentration of 5 ug/ml and the reaction was allowed to proceed overnight at room temperature. The TCR was then separated from free biotin by gel filtration (see Figure 37). Fractions containing biotinylated TCR were pooled and protease inhibitor cocktail was added. Protein concentration was also determined. Figure 35 shows a schematic diagram of the soluble, biotinylated TCR.

**References**

**Aifantis, I.,** O. Azogui, et al. (1998). Immunity **9**(5): 649-55.
**Altamirano**, M. M., C. Garcia, et al. (1999). Nature Biotechnology **17**: 187-191.
**Altamirano,** M. M., R. Golbik, et al. (1997). Proc Natl Acad Sci U S A **94**(8): 3576-8.
**Amati,** B., S. Dalton, et al. (1992). Nature **359**(6394): 423-6.
**Barker,** D. F. and A. M. Campbell (1981). J Mol Biol **146**(4): 469-92.
**Barker,** D. F., and Campbell, A. M. (1981). J Mol Biol **146**, 469-92.
**Bentley,** G. A., G. Boulot, et al. (1995). Science **267**(5206): 1984-7 Issn: 0036-8075.
**Bjorkman**, P. J., J. L. Strominger, et al. (1985). J. Mol. Biol **186**(1): 205-10 Issn: 0022-2836
**Boice**, J. A., G. R. Dieckmann, et al. (1996). Biochemistry **35**(46): 14480-5.
**Boulot**, G., G. A. Bentley, et al. (1994). J Mol Biol **235**(2): 795-7 Issn: 0022-2836.
**Brocker**, T., A. Peter, et al. (1993). Eur J Immunol **23**(7): 1435-9 Issn: 0014-2980.

**Buday**, L. and J. Downward (1993). Cell **73**(3): 611-20.

**Calaman**, S. D., G. R. Carson, et al. (1993). J Immunol Methods **164**(2): 233-44 Issn: 0022-1759.

**Chao**, H., M. E. Houston, Jr., et al. (1996). Biochemistry **35**(37): 12175-85.

**Chang**, H. C., Z. Bao, et al. (1994). Proc Natl Acad Sci U S A **91**(24): 11408-12.

**Chevray**, P. M. and D. Nathans (1992). Proc Natl Acad Sci U S A **89**(13): 5789-93.

**Chicz**, R. M., R. G. Urban, et al. (1993). J Exp Med **178**(1): 27-47.

**Corr**, M., A. E. Slanetz, et al. (1994). Science **265**(5174): 946-9.

**Davis,** M. M., J. J. Boniface, et al. (1998). Annu. Rev. Immunol. **16**: 523-544.

**de Kruif**, J. and T. Logtenberg (1996). J Biol Chem **271**(13): 7630-4.

**Ding**, Y. H., K. J. Smith, et al. (1998). Immunity **8**(4): 403-11.

**Eilat,** D., G. E. Kikuchi, et al. (1992). Proc Natl Acad Sci U S A **89**(15): 6871-5 Issn: 0027-8424.

**Engelhard**, V. H. (1994). Annu Rev Immunol **12**: 181-207.

**Engelhard,** V. H., E. Appella, et al. (1993). Chem Immunol **57**: 39-62.

**Fields,** B. A., E. L. Malchiodi, et al. (1996). Nature **384**(6605): 188-92 Issn: 0028-0836.

**Gao**, G. F., U. C. Gerth, et al. (1998). Prot. Sci. **7**: 1245-49.

**Gao**, G. F., J. Tormo, et al. (1997). Nature **387**(6633): 630-4.

**Garboczi**, D. N. and W. E. Biddison (1999). Immunity **10**(1): 1-7.

**Garboczi,** D. N., P. Ghosh, et al. (1996). Nature **384**(6605): 134-41.

**Garboczi,** D. N., D. T. Hung, et al. (1992). Proc Natl Acad Sci U S A **89**(8): 3429-33 Issn: 0027-8424.

**Garboczi,** D. N., D. R. Madden, et al. (1994). J Mol Biol **239**(4): 581-7 Issn: 0022-2836.

**Garboczi,** D. N., U. Utz, et al. (1996). J Immunol **157**(12): 5403-10.

**Garcia**, K. C., M. Degano, et al. (1996). Science **274**(5285): 209-19 Issn: 0036-8075.

**Garcia**, K. C., C. A. Scott, et al. (1996). Nature **384**(6609): 577-81 Issn: 0028-0836.

**Glover**, J. N. and S. C. Harrison (1995). Nature **373**(6511): 257-61.

**Golden**, A., S. S. Khandekar, et al. (1997). J Immunol Methods **206**(1-2): 163-9.

**Greenfield**, N. J., G. T. Montelione, et al. (1998). Biochemistry **37**(21): 7834-43.

**Gregoire**, C., B. Malissen, et al. (1996). Eur J Immunol **26**(10): 2410-6 Issn: 0014-2980.

**Gregoire**, C., N. Rebai, et al. (1991). Proc Natl Acad Sci U S A **88**(18): 8077-81 Issn: 0027-8424.

**Hilyard** et al (1994) Proc. Natl.. Acad. Sci. **91**: 9057-9061.

**Howard**, P. K., J. Shaw, et al. (1985). Gene **35**(3): 321-31.

**Hu**, J. C., N. E. Newell, et al. (1993). Protein Sci **2**(7): 1072-84.

**Huczko**, E. L., W. M. Bodnar, et al. (1993). J Immunol **151**(5): 2572-87.

**Hunt**, D. F., H. Michel, et al. (1992). Science **256**(5065): 1817-20 Issn: 0036-8075.

**Ishii,** Y., T. Nakano, et al. (1995). J Immunol Methods **186**(1): 27-36 Issn: 0022-1759.

**Kouzarides**, T. and E. Ziff (1989). Nature **340**(6234): 568-71.

**Landschulz**, W. H., P. F. Johnson, et al. (1988). Science **240**(4860): 1759-64.

**Lowenstein,** E. J., R. J. Daly, et al. (1992). Cell **70**(3): 431-42.

**Lumb**, K. J. and P. S. Kim (1995). Biochemistry **34**(27): 8642-8.

**Madden,** D. R., D. N. Garboczi, et al. (1993).[published erratum appears in Cell 1994 Jan 28;76(2):following 410]. " Cell **75**(4): 693-708 Issn: 0092-8674.

**Matsui,** K., J. J. Boniface, et al. (1994). Proc Natl Acad Sci U S A **91**(26): 12862-6 Issn: 0027-8424.

**McKnight**, S. L. (1991). Sci Am **264**(4): 54-64.

**Moss** et al (1991) Proc. Natl. Acad. Scj. USA **88:** 8987-8990 Issn: 0027-8424.

**Nautiyal,** S., D. N. Woolfson, et al. (1995). Biochemistry **34**(37): 11645-51.

**Necker,** A., N. Rebai, et al. (1991). Eur J Immunol **21**(12): 3035-40 Issn: 0014-2980.

**O'Callaghan**, C. A., M. F. Byford, et al. (1999). Anal Biochem **266**(1): 9-15.

**O'Shea,** E. K., R. Rutkowski, et al. (1992). Cell **68**(4): 699-708.

**O'Shea,** E. K., R. Rutkowski, et al. (1989). Science **245**(4918): 646-8.

**O'Shea**, E.K., Lumb, K.J. and Kim, P.S. (1993) Curr. Biol. **3**: 658-667.

**Plaksin**, D., K. Polakova, et al. (1997). J Immunol **158**(5): 2218-27.

**Rabinowitz,** J. D., C. Beeson, et al. (1996). Proc Natl Acad Sci U S A **93**(4): 1401-5 Issn: 0027-8424.

**Ramiro**, A. R., C. Trigueros, et al. (1996). J Exp Med **184**(2): 519-30 Issn: 0022-1007.

**Reid**, S. W., S. McAdam, et al. (1996). J Exp Med **184**(6): 2279-86.

**Reid**, S. W., K. J. Smith, et al. (1996). FEBS Lett **383**(1-2): 119-23.

**Riley,** L. G., G. B. Ralston, et al. (1996).[published erratum appears in Protein Eng 1996 Sep;9(9):831]." Protein Eng **9**(2): 223-30.

**Romagne**, F., M. A. Peyrat, et al. (1996). J Immunol Methods **189**(1): 25-36 Issn: 0022-1759.

**Saint Ruf,** C., K. Ungewiss, et al. (1994). Science **266**(5188): 1208-12 Issn: 0036-8075.

**Schatz,** P. J. (1993). Biotechnology N Y **11**(10): 1138-43.

**Schlessinger,** J. (1994). Curr Opin Genet Dev **4**(1): 25-30.

**Schlueter,** C. J., B. A. Schodin, et al. (1996). J Mol Biol **256**(5): 859-69.

**Schuermann,** M., J. B. Hunter, et al. (1991). Nucleic Acids Res **19**(4): 739-46.

**Smith,** K. J., S. W. Reid, et al. (1996). Immunity **4**(3): 215-28 Issn: 1074-7613.

**Smith**, K. J., S. W. Reid, et al. (1996). Immunity **4**(3): 203-13 Issn: 1074-7613.

**Studier,** F. W., A. H. Rosenberg, et al. (1990). Methods Enzymol **185**: 60-89 Issn: 0076-6879.

**von Boehmer**, H., I. Aifantis, et al. (1998). Immunol Rev **165**: 111-9.

**Weber**, S., A. Traunecker, et al. (1992). Nature **356**(6372): 793-6 Issn: 0028-0836.

**Willcox,** B. E., G. F. Gao, et al. (1999). Immunity **10**: 357-65.

**Wilson**, A. and H. R. MacDonald (1995). Int Immunol **7**(10): 1659-64 Issn: 0953-8178.

**Wurch,** A., J. Biro, et al. (1998). J Exp Med **188**(9): 1669-78.

**Wyer**, J. R., B. E. Willcox, et al. (1999). Immunity **10**: 219-225.

**Zhang**, Z., A. Murphy, et al. (1999). Curr Biol **9**(8): 417-20.

**Claims**

1. A refolded recombinant T cell receptor (TCR) which comprises:

   i) a recombinant TCR $\alpha$ or $\gamma$ chain extracellular domain having a first heterologous C-terminal dimerisation peptide; and
   ii) a recombinant TCR $\beta$ or $\delta$ chain extracellular domain having a second C-terminal dimerisation peptide which is specifically heterodimerised with the first dimerisation peptide to form a heterodimerisation domain, wherein a disulphide bond present in native TCRs between the $\alpha$ and $\beta$ or $\gamma$ and $\delta$ chains adjacent to the cytoplasmic domain Is absent.

2. A biologically-active recombinant T cell receptor (TCR) which comprises:

   i) a recombinant TCR $\alpha$ or $\gamma$ chain extracellular domain having a first heterologous C-terminal dimerisation peptide; and
   ii) a recombinant TCR $\beta$ or $\delta$ chain extracellular domain having a second C-terminal dimerisation peptide which Is specifically heterodimerised with the first dimerisation peptide to form a heterodimerisation domain, wherein a disulphide bond present in native TCRs between the $\alpha$ and $\beta$ or $\gamma$ and $\delta$ chains adjacent to the cytoplasmic domain is absent.

3. The recombinant TCR according to claim 1 or claim 2, wherein the heterodimerisation domain is a coiled coil domain.

4. The recombinant TCR according to claim 3, wherein the dimerisation peptides are c-jun and c-fos dimerisation peptIdes.

5. The recombinant TCR according to any one of claims 1 to 4, comprising a flexible linker located between the TCR chains and the heterodimerisation peptIdes.

6. The recombinant TCR according to any one of claims 1 to 5, expressed in an *E. coli* expression system.

7. The recombinant TCR according to any one of claims 1 to 6, which is biotinylated at the C-terminus.

8. The recombinant TCR according to any one of claims 1 to 7, labelled with a detectable label.

9. The recombinant TCR according to any one of claims 1 to 8, linked to a therapeutic agent such as a cytotoxic agent or an immunostimulating agent.

10. Nucleic add sequences encoding the recombinant TCR chains of the recombinant TCR according to any one of claims 1 to 8.

11. A nucleic acid sequence according to claim 10. in an *E. coli* expression vector.

**12.** A method of making a recombinant non membrane bound T cell receptor, which method comprises expressing:

i) a recombinant TCR α or γ chain extracellular domain having a first heterologous C-terminal dimerisation peptide; and

ii) a recombinant TCR β or δ chain extracellular domain having a second C-terminal dimerisation peptide which specifically heterodimerises with the first dimerisation peptide to form a heterodimerisation domain; and refolding the chains together *in vitro* to produce a TCR heterodimer.

**13.** The method according to claim 12, wherein refolding is carried out in a refolding buffer comprising a solubilising agent.

**14.** The method according to claim 13, wherein the solubilising agent is urea at a concentration of at least 0.1 M.

**15.** The method according to claim 14, wherein the solubilising agent is urea at a concentration of about 5M.

**16.** The method according to any one of claims 12 to 14, wherein the chains are denatured in a denaturing buffer prior to refolding.

**17.** The method according to claim 16, wherein the denaturing buffer contains DTT or guanidine as a reducing agent.

**18.** The method according to any one of claims 12 to 17, wherein the TCR is the recombinant TCR according to any one of claims 1 to 6.

**Patentansprüche**

**1.** Rückgefalteter rekombinanter T-Zell-Rezeptor (TZR), welcher aufweist:

i) die extrazelluläre Domäne der α- oder γ-Kette eines rekombinanten TZR mit einem ersten heterologen C-terminalen Dimerisierungspeptid; und

ii) die extrazelluläre Domäne der β- oder δ-Kette eines rekombinanten TZR, mit einem zweiten C-terminalen Dimerisierungspeptid, das spezifisch heterodimerisiert ist mit dem ersten Dimerisierungspeptid, um eine Heterodimerisierungsdomäne zu bilden, wobei eine Disulfid-Bindung, welche in nativen TZR zwischen der α- und β-Kette oder der γ- und δ-Kette an die zytoplasmatische Domäne angrenzt, fehlt.

**2.** Biologisch aktiver rekombinanter T-Zell-Rezeptor (TZR), welcher aufweist:

i) die extrazelluläre Domäne der α- oder γ-Kette eines rekombinanten TZR mit einem ersten heterologen C-terminalen Dimerisierungspeptid; und

ii) die extrazelluläre Domäne der β- oder δ-Kette eines rekombinanten TZR, mit einem zweiten C-terminalen Dimerisierungspeptid, das spezifisch heterodimerisiert ist mit dem ersten Dimerisierungspeptid, um eine Heterodimerisierungsdomäne zu bilden, wobei eine Disulfid-Bindung, welche in nativen TZR zwischen der α- und β-Kette oder der γ- und δ-Kette an die zytoplasmatische Domäne angrenzt, fehlt.

**3.** Rekombinanter TZR nach Anspruch 1 oder 2, wobei die Heterodimerisierungsdomäne eine superspiralisierte Domäne ist.

**4.** Rekombinanter TZR nach Anspruch 3, wobei die Dimerisierungspeptide c-jun- und c-fos-Dimerisierungspeptide sind.

**5.** Rekombinanter TZR nach einem der Ansprüche 1 bis 4, welcher einen flexiblen Linker zwischen den TZR-Ketten und den Heterodimerisierungspeptiden aufweist.

**6.** Rekombinanter TZR nach einem der Ansprüche 1 bis 5, welcher in einem E. coli-Expressionssystem exprimiert ist.

**7.** Rekombinanter TZR nach einem der Ansprüche 1 bis 6, welcher C-terminal biotinyliert ist.

**8.** Rekombinanter TZR nach einem der Ansprüche 1 bis 7, welcher mit einem nachweisbaren Marker markiert ist.

**9.** Rekombinanter TZR nach einem der Ansprüche 1 bis 8, welcher mit einem therapeutischen Mittel, wie einem zytotoxischen Mittel oder einem immunstimulierenden Mittel, verbunden ist.

**10.** Nukleinsäuresequenzen, welche für die rekombinanten TZR-Ketten des rekombinanten TZR nach einem der Ansprüche 1 bis 8 kodieren.

**11.** Nukleinsäuresequenz nach Anspruch 10, in einem E. coli-Expressionsvektor.

**12.** Verfahren zur Herstellung eines rekombinanten nicht membrangebundenen T-Zell-Rezeptors, welches Verfahren umfasst das Exprimieren:

i) der extrazellulären Domäne der α- oder γ-Kette eines rekombinanten TZR mit einem ersten heterologen C-terminalen Dimerisierungspeptid; und
ii) der extrazellulären Domäne der β- oder δ-Kette eines rekombinanten TZR, mit einem zweiten C-terminalen Dimerisierungspeptid, das spezifisch mit dem ersten Dimerisierungspeptid heterodimerisiert, um eine Heterodimerisierungsdomäne zu bilden; und Rückfalten der Ketten zu einer zusammengefalteten Kette in vitro, um ein TZR-Heterodimer zu produzieren.

**13.** Verfahren nach Anspruch 12, wobei das Rückfalten in einem Rückfaltungspuffer, der ein Lösungsmittel enthält, durchgeführt wird.

**14.** Verfahren nach Anspruch 13, wobei das Lösungsmittel Harnstoff mit einer Konzentration von mindestens 0,1 M ist.

**15.** Verfahren nach Anspruch 14, wobei das Lösungsmittel Harnstoff mit einer Konzentration von etwa 5 M ist.

**16.** Verfahren nach einem der Ansprüche 12 bis 14, wobei die Ketten vor dem Rückfalten in einem Denaturierungspuffer denaturiert werden.

**17.** Verfahren nach Anspruch 16, wobei der Denaturierungspuffer DTT oder Guanidin als Reduktionsmittel enthält.

**18.** Verfahren nach einem der Ansprüche 12 bis 17, wobei der TZR der rekombinante TZR nach einem der Ansprüche 1 bis 6 ist.

**Revendications**

**1.** Récepteur de lymphocytes T (TCR) recombinant replié qui comprend :

i) un domaine extracellulaire à chaîne α ou γ de TCR recombinant présentant un premier peptide hétérologue de dimérisation en extrémité C, et
ii) un domaine extracellulaire à chaine β ou δ de TCR recombinant présentant un deuxième peptide de dimérisation en extrémité C qui réalise spécifiquement une hétérodimérisation avec le premier peptide de dimérisation pour former un domaine d'hétérodimérisation, dans lequel un pont disulfure présent dans les TCR natifs entre les chaînes α et β ou γ et δ adjacent au domaine cytoplasmique est absent.

**2.** Récepteur de lymphocytes T (TCR) recombinant actif sur le plan biologique qui comprend :

i) un domaine extracellulaire à chaîne α ou γ de TCR recombinant présentant un premier peptide hétérologue de dimérisation en extrémité C, et
ii) un domaine extracellulaire à chaîne β ou δ de TCR recombinant présentant un deuxième peptide de dimérisation en extrémité C qui réalise spécifiquement une hétérodimérisation avec le premier peptide de dimérisation pour former un domaine d'hétérodimérisation, dans lequel un pont disulfure présent dans les TCR natifs entre les chaînes α et β ou γ et δ adjacent au domaine cytoplasmique est absent.

**3.** TCR recombinant suivant la revendication 1 ou la revendication 2, dans lequel le domaine d'hétérodimérisation est un domaine à double hélice.

**4.** TCR recombinant suivant la revendication 3, dans lequel les peptides de dimérisation sont les peptides de dimérisation c-jun et c-fos.

**5.** TCR recombinant suivant l'une quelconque des revendications 1 à 4, comprenant un bras flexible de liaison situé entre les chaînes de TCR et les peptides d'hétérodimérisation.

**6.** TCR recombinant suivant l'une quelconque des revendications 1 à 5, exprimé dans un système d'expression de *E. coli*.

**7.** TCR recombinant suivant l'une quelconque des revendications 1 à 6, qui est biotinylé à l'extrémité C.

**8.** TCR recombinant suivant l'une quelconque des revendications 1 à 7, marqué par un marquage pouvant être détecté.

**9.** TCR recombinant suivant l'une quelconque des revendications 1 à 8, lié à un agent thérapeutique tel qu'un agent cytotoxique ou un agent provoquant une immunostimulation.

**10.** Séquences d'acides nucléiques codant pour les chaînes de TCR recombinant du TCR recombinant suivant l'une quelconque des revendications 1 à 8.

**11.** Séquence d'acide nucléique suivant la revendication 10, dans un vecteur d'expression de *E. coli*.

**12.** Procédé de fabrication d'un récepteur recombinant de lymphocyte T non lié à une membrane, lequel procédé comprend l'expression :

i) d'un domaine extracellulaire à chaîne $\alpha$ ou $\gamma$ de TCR recombinant présentant un premier peptide hétérologue de dimérisation en extrémité C, et
ii) d'un domaine extracellulaire à chaîne $\beta$ ou $\delta$ de TCR recombinant présentant un deuxième peptide de dimérisation en extrémité C qui réalise spécifiquement une hétérodimérisation avec le premier peptide de dimérisation pour former un domaine d'hétérodimérisation, et le repliement des chaînes ensemble *in vitro* pour produire un hétérodimère de TCR.

**13.** Procédé suivant la revendication 12, dans lequel le repliement est réalisé dans un tampon de repliement comprenant un agent de solubilisation.

**14.** Procédé suivant la revendication 13, dans lequel l'agent de solubilisation est de l'urée à une concentration d'au moins 0,1 M.

**15.** Procédé suivant la revendication 14, dans lequel l'agent de solubilisation est de l'urée à une concentration d'environ 5 M.

**16.** Procédé suivant l'une quelconque des revendications 12 à 14, dans lequel les chaînes sont dénaturées dans un tampon de dénaturation avant le repliement.

**17.** Procédé suivant la revendication 16, dans lequel le tampon de dénaturation contient du DTT ou de la guanidine comme agent réducteur.

**18.** Procédé suivant l'une quelconque des revendications 12 à 17, dans lequel le TCR est le TCR recombinant suivant l'une quelconque des revendications 1 à 6.

EP 1 066 380 B1

Figure 1

30

Figure 2

Figure 3

Figure 4

# A

Poly-C 'anchor primer' :

```
              Xho I
5'- TAA ATA CTC GAG GCG CGC CCC CCC CCC CCC CCC -3'
```

# B

TCR α chain constant region specific primer:

```
              Xma I
5'- ATA TAA CCC GGG GAA CCA GAT CCC CAC AGG AAC TTT CTG GGC TGG GGA -3'
```

# C

TCR β chain constant region specific primer:

```
              Xma I
5'- ATA TAA CCC GGG GAA CCA GAT CCC CAC AGT CTG CTC TAC CCC AGG CC -3'
```

Figure 5

## A

*c-jun* 5′ primer:

        Xma I

5′– CATACACCCGGGGGTAGAATCGCCCGGCTGGAG –3′

## B

*c-jun* 3′ primer:

        Xho I

5′ – GTGTGTGCTCGAGGATCCTAGTAGTTCATGACTTTCTGTTTAAGCTGTGC –3′

          Bam HI

## C

*c-fos* 5′ primer:

        Xma I

5′ –CATACACCCGGGGGGTCTGACTGATACACTCCAAGCGGAG –3′

## D

*c-fos* 3' primer:

        Xho I

5′– TGTGTGCTCGAGGATCCTAGTAAGCTGCCAGGATGAACTCTAGTTTTTC –3′

        Bam HI

Figure 6.

**A**

```
       R    I    A    R    L    E    E    K    V    K    T    L    K
5' - AGA  ATC  GCC  CGG  CTG  GAG  GAA  AAA  GTG  AAA  ACC  TTG  AAA

       A    Q    N    S    E    L    A    S    T    A    N    M    L
     GCT  CAG  AAC  TCG  GAG  CTG  GCG  TCC  ACG  GCC  AAC  ATG  CTC

       R    E    Q    V    A    Q    L    K    Q    K    V    M    N
     AGG  GAA  CAG  GTG  GCA  CAG  CTT  AAA  CAG  AAA  GTC  ATG  AAC

       Y
     TAC  -  3'
```

C-jun leucine zipper DNA and amino acid (one-letter code) sequences as fused to TCR alpha chains.

------------------------------------------------------------------

**B**

```
       L    T    D    T    L    Q    A    E    T    D    Q    L    E
5' - CTG  ACT  GAT  ACA  CTC  CAA  GCG  GAG  ACA  GAC  CAA  CTA  GAA

       D    E    K    S    A    L    Q    T    E    I    A    N    L
     GAT  GAG  AAG  TCT  GCT  TTG  CAG  ACC  GAG  ATT  GCC  AAC  CTG

       L    K    E    K    E    K    L    E    F    I    L    A    A
     CTG  AAG  GAG  AAG  GAA  AAA  CTA  GAG  TTC  ATC  CTG  GCA  GCT

       Y
     TAC  -  3'
```

C-fos leucine zipper DNA and amino acid (one-letter code) sequences as fused to TCR beta chains.

Figure 7

# A

Mutation of cysteine to serine, forwards (sense) primer, indicating amino acid sequence and the mutation:

```
                         C
                         ↓
         D    S    R    Y    S    L    S    S
5'- GAC  TCC  AGA  TAC  AGC  CTG  AGC  AGC  CG -3'
```

# B

Mutation of cysteine to serine, backwards (nonsense) primer:

```
5'- CG  GCT  GCT  CAG  GCT  GTA  TCT  GGA  GTC -3'
```

# C

Mutation of cysteine to alanine, forwards (sense) primer, indicating amino acid sequence and the mutation:

```
                         C
                         ↓
         D    S    R    Y    A    L    S    S
5'- GAC  TCC  AGA  TAC  GCT  CTG  AGC  AGC  CG -3'
```

# D

Mutation of cysteine to alanine, backwards (nonsense) primer:

```
5'- CG  GCT  GCT  CAG  AGC  GTA  TCT  GGA  GTC -3'
```

Figure 8

Figure 9

# A

5' PCR primer for the human Vα10.2 chain of the JM22 Influenza Matrix peptide-HLA-A0201 restricted TCR:

```
                 M   Q   L   L   E   Q   S   P   Q   F   L
5'- gctctagacat ATG CAa CTa CTa GAa CAa AGt CCT CAG TTT CTA
                 Nde I
```

```
 S   I   Q   E
AGC ATC CAA GAG G -3'
```

# B

5' PCR primer for the human Vβ17 chain of the JM22 Influenza Matrix peptide-HLA-A0201 restricted TCR:

```
                 M   V   D   G   G   I   T   Q   S
5'- gctctagacat ATG GTG GAT GGT GGA ATC ACT CAG TCC C -3'
                 Nde I
```

# C

5' PCR primer for the mouse Vα4 chain of the Influenza nucleoprotein peptide-H2-D^b restricted TCR:

```
                 M   D   S   V   T   Q   M   Q   G   Q   V
5'- gctctagacat ATG GAt TCt GTt ACt CAa ATG CAa GGt CAa GTG
                 Nde I
```

```
 T   L   S   S
ACC CTC TCA TCA G -3'
```

Figure 9 (continued)

# D
5′ PCR primer for the mouse Vβ11 chain of the Influenza nucleoprotein peptide-H2-D$^b$ restricted TCR:

```
                M    E    P    T    N    A    G    V    I    Q
5'- gctctagacat ATG  GAa  CCa  ACa  AAt  GCt  GGt  GTt  ATC  CAA

 T    P    R    H
ACA  CCT  AGG  CAC  -3'
```

# E
5′ PCR primer for the human Vα23 chain of the 003 HIV-1 Gag peptide-HLA-A0201 restricted TCR:

```
                M    K    Q    E    V    T    Q    I
5'- ggaattccat atg  AAA  CAa  GAG  GTt  ACa  CAa  ATT  CC  -3'
             Nde I
```

# F
5′ PCR primer for the human Vβ5.1 chain of the 003 HIV-1 Gag peptide-HLA-A0201 restricted TCR:

```
                M    K    A    G    V    T    Q    T
5'- ggaattccat atg  AAa  GCT  GGA  GTt  ACT  CAA  ACT  CC  -3'
```

Figure 9 (continued)

# G

5' PCR primer for the human Vα2.3 chain of the HTLV-1 Tax peptide-HLA-A0201 restricted A6 TCR:

```
                M   Q   K   E   V   E   Q   K
5' -cccccc cat ATG CAG AAG GAA GTG GAG CAG AAC -3'
           Nde I
```

# H

5' PCR primer for the human Vβ12.3 chain of the HTLV-1 Tax peptide-HLA-A0201 restricted A6 TCR:

```
              M   K   A   G   V   T   Q   T
5'- cccccc cat ATG AAC GCT GGT GTC ACT CAG ACC -3'
            Nde I
```

# I

5' PCR primer for the human Vα17.2 chain of the HTLV-1 Tax peptide-HLA-A0201 restricted B7 TCR:

```
                M   Q   Q   K   N   D   D   Q   Q   V
5' -cccccc cat ATG CAA CAa AAa AAT GAT GAC CAG CAA GTT
           Nde I
```

```
 K   Q   N
AAG CAA AAT -3'
```

Figure 9 (continued)

**J**

5' PCR primer for the human Vβ12.3 chain of the HTLV-1 Tax peptide-HLA-A0201 restricted B7 TCR:

```
                  M    N    A    G    V    T    Q    T    P    K    F
5' -cccccc cat ATG AAC GCT GGT GTC ACT CAG ACC CCA AAA TTC
           Nde I
```

**Q**
CAG -3'

**K**

3' PCR primer for human Cα chains, generally applicable:

```
5'- cataca ccc ggg GGA ACT TTC TGG GCT GGG GAA GAA GG -3'
            Xma I
```

**L**

3' PCR primer for human Cβ chains, generally applicable:

```
5'- cataca ccc ggg GTC TGC TCT ACC CCA GGC CTC -3'
            Xma I
```

Figure 10

   TCR alfa>

M  Q  L  L  E  Q  S  P  Q  F  L  S  I  Q  E  G  E  N  L  T
ATGCAaCTaCTaGAaCAaAGtCCTCAGTTTCTAAGCATCCAAGAGGGAGAAAATCTCACT

V  Y  C  N  S  S  S  V  F  S  S  L  Q  W  Y  R  Q  E  P  G
GTGTACTGCAACTCCTCAAGTGTTTTTTTCCAGCTTACAATGGTACAGACAGGAGCCTGGG

E  G  P  V  L  L  V  T  V  V  T  G  G  E  V  K  K  L  K  R
GAAGGTCCTGTCCTCCTGGTGACAGTAGTTACGGGTGGAGAAGTGAAGAAGCTGAAGAGA

L  T  F  Q  F  G  D  A  R  K  D  S  S  L  H  I  T  A  A  Q
CTAACCTTTCAGTTTGGTGATGCAAGAAAGGACAGTTCTCTCCACATCACTGCGGCCCAG

P  G  D  T  G  L  Y  L  C  A  G  A  G  S  Q  G  N  L  I  F
CCTGGTGATACAGGCCTCTACCTCTGTGCAGGAGCGGGAAGCCAAGGAAATCTCATCTTT

G  K  G  T  K  L  S  V  K  P  N  I  Q  N  P  D  P  A  V  Y
GGAAAAGGCACTAAACTCTCTGTTAAACCAAATATCCAGAACCCTGACCCTGCCGTGTAC

Q  L  R  D  S  K  S  S  D  K  S  V  C  L  F  T  D  F  D  S
CAGCTGAGAGACTCTAAATCCAGTGACAAGTCTGTCTGCCTATTCACCGATTTTGATTCT

Q  T  N  V  S  Q  S  K  D  S  D  V  Y  I  T  D  K  T  V  L
CAAACAAATGTGTCACAAAGTAAGGATTCTGATGTGTATATCACAGACAAAACTGTGCTA

D  M  R  S  M  D  F  K  S  N  S  A  V  A  W  S  N  K  S  D
GACATGAGGTCTATGGACTTCAAGAGCAACAGTGCTGTGGCCTGGAGCAACAAATCTGAC

F  A  C  A  N  A  F  N  N  S  I  I  P  E  D  T  F  F  P  S
TTTGCATGTGCAAACGCCTTCAACAACAGCATTATTCCAGAAGACACCTTCTTCCCCAGC

   <TCR alfa linker c-jun>
P  E  S  S  P  G  G  R  I  A  R  L  E  E  K  V  K  T  L  K
CCAGAAAGTTCCcccgggGGTAGAATCGCCCGGCTGGAGGAAAAAGTGAAAACCTTGAAA

A  Q  N  S  E  L  A  S  T  A  N  M  L  R  E  Q  V  A  Q  L
GCTCAGAACTCGGAGCTGGCGTCCACGGCCAACATGCTCAGGGAACAGGTGGCACAGCTT

K  Q  K  V  M  N  Y  *
AAACAGAAAGTCATGAACTACTAG

Figure 11

TCR beta>
```
  M   V   D   G   G   I   T   Q   S   P   K   Y   L   F   R   K   E   G   Q   N
ATGGTGGATGGTGGAATCACTCAGTCCCCAAAGTACCTGTTCAGAAAGGAAGGACAGAAT

  V   T   L   S   C   E   Q   N   L   N   H   D   A   M   Y   W   Y   R   Q   D
GTGACCCTGAGTTGTGAACAGAATTTGAACCACGATGCCATGTACTGGTACCGACAGGAC

  P   G   Q   G   L   R   L   I   Y   Y   S   Q   I   V   N   D   F   Q   K   G
CCAGGGCAAGGGCTGAGATTGATCTACTACTCACAGATAGTAAATGACTTTCAGAAAGGA

  D   I   A   E   G   Y   S   V   S   R   E   K   K   E   S   F   P   L   T   V
GATATAGCTGAAGGGTACAGCGTCTCTCGGGAGAAGAAGGAATCCTTTCCTCTCACTGTG

  T   S   A   Q   K   N   P   T   A   F   Y   L   C   A   S   S   R   S   Sq
ACATCGGCCCAAAAGAACCCGACAGCTTTCTATCTCTGTGCCAGTAGTTCGAGGAGCTCC

  Y   E   Q   Y   F   G   P   G   T   R   L   T   V   T   E   D   L   K   N   V
TACGAGCAGTACTTCGGGCCGGGCACCAGGCTCACGGTCACAGAGGACCTGAAAAACGTT

  F   P   P   E   V   A   V   F   E   P   S   E   A   E   I   S   H   T   Q   K
TTCCCACCCGAGGTCGCTGTGTTTGAACCATCAGAAGCAGAGATCTCCCACACCCAAAAG

  A   T   L   V   C   L   A   T   G   F   Y   P   D   H   V   E   L   S   W   W
GCCACACTGGTGTGCCTGGCCACAGGCTTCTACCCCGACCACGTGGAGCTGAGCTGGTGG

  V   N   G   K   E   V   H   S   G   V   S   T   D   P   Q   P   L   K   E   Q
GTGAATGGGAAGGAGGTGCACAGTGGGGTCAGCACAGACCCGCAGCCCCTCAAGGAGCAG

  P   A   L   N   D   S   R   Y   C   L   S   S   R   L   R   V   S   A   T   F
CCCGCCCTCAATGACTCCAGATACTGCCTGAGCAGCCGCCTGAGGGTCTCGGCCACCTTC

  W   Q   N   P   R   N   H   F   R   C   Q   V   Q   F   Y   G   L   S   E   N
TGGCAGAACCCCCGCAACCACTTCCGCTGTCAAGTCCAGTTCTACGGGCTCTCGGAGAAT

  D   E   W   T   Q   D   R   A   K   P   V   T   Q   I   V   S   A   E   A   W
GACGAGTGGACCCAGGATAGGGCCAAACCTGTCACCCAGATCGTCAGCGCCGAGGCCTGG
```

<TCR beta linker c-fos>
```
  G   R   A   D   P   G   G   L   T   D   T   L   Q   A   E   T   D   Q   L   E
GGTAGAGCAGACcccgggGGTCTGACTGATACACTCCAAGCGGAGACAGATCAACTTGAA

  D   K   K   S   A   L   Q   T   E   I   A   N   L   L   K   E   K   E   K   L
GACAAGAAGTCTGCGTTGCAGACCGAGATTGCCAATCTACTGAAAGAGAAGGAAAAACTA

  E   F   I   L   A   A   Y   *
GAGTTCATCCTGGCAGCTTACTAG
```

Figure 12

TCR alfa>
```
  M   N   Y   S   P   A   L   V   T   V   M   L   F   V   F   G   R   T   H   G
ATGAACTATTCTCCAGCTTTAGTGACTGTGATGCTGTTTGTGTTTGGGAGGACCCATGGA

  D   S   V   T   Q   M   Q   G   Q   V   T   L   S   E   D   D   F   L   F   I
GACTCAGTAACCCAGATGCAAGGTCAAGTGACCCTCTCAGAAGACGACTTCCTATTTATA

  N   C   T   Y   S   T   T   W   Y   P   T   L   F   W   Y   V   Q   Y   P   G
AACTGTACTTATTCAACCACATGGTACCCGACTCTTTTCTGGTATGTCCAATATCCTGGA

  E   G   P   Q   L   L   L   K   V   T   T   A   N   N   K   G   I   S   R   G
GAAGGTCCACAGCTCCTTTTGAAAGTCACAACAGCCAACAACAAGGGAATCAGCAGAGGT

  F   E   A   T   Y   D   K   G   T   T   S   F   H   L   Q   K   A   S   V   Q
TTTGAAGCTACATATGATAAAGGAACAACGTCCTTCCACTTGCAGAAAGCCTCAGTGCAG

  E   S   D   S   A   V   Y   Y   C   V   L   G   D   R   Q   G   G   R   A   L
GAGTCAGACTCTGCTGTGTACTACTGTGTGCTGGGTGATCGACAGGGAGGCAGAGCTCTG

  I   F   G   T   G   T   T   V   S   V   S   P   N   I   Q   N   P   E   P   A
ATATTTGGAACAGGAACCACGGTATCAGTCAGCCCCAACATCCAGAACCCAGAACCTGCT

  V   Y   Q   L   K   D   P   R   S   Q   D   S   T   L   C   L   F   T   D   F
GTGTACCAGTTAAAAGATCCTCGGTCTCAGGACAGCACCCTCTGCCTGTTCACCGACTTT

  D   S   Q   I   N   V   P   K   T   M   E   S   G   T   F   I   T   D   K   T
GACTCCCAAATCAATGTGCCGAAAACCATGGAATCTGGAACGTTCATCACTGACAAAACT

  V   L   D   M   K   A   M   D   S   K   S   N   G   A   I   A   W   S   N   Q
GTGCTGGACATGAAAGCTATGGATTCCAAGAGCAATGGGGCCATTGCCTGGAGCAACCAG

  T   S   F   T   C   Q   D   I   S   K   E   T   N   A   T   Y   P   S   S   D
ACAAGCTTCACCTGCCAAGATATCTCCAAAGAGACCAACGCCACCTACCCCAGTTCAGAC
```

<TCR alfa linker c-jun>
```
  V   P   G   G   R   I   A   R   L   E   E   K   V   K   T   L   K   A   Q   N
GTTcccgggGGTAGAATCGCCCGGCTGGAGGAAAAAGTGAAAACCTTGAAAGCTCAGAAC

  S   E   L   A   S   T   A   N   M   L   R   E   Q   V   A   Q   L   K   Q   K
TCGGAGCTGGCGTCCACGGCCAACATGCTCAGGGAACAGGTGGCACAGCTTAAACAGAAA

  V   M   N   Y   *
GTCATGAACTACTAG
```

44

Figure 13

```
    TCR beta>
  M   K   A   G   V   T   Q   T   P   R   Y   L   I   K   T   R   G   Q   Q   V
ATGAAAGCTGGAGTTACTCAAACTCCAAGATATCTGATCAAAACGAGAGGACAGCAAGTG

  T   L   S   C   S   P   I   S   G   H   R   S   V   S   W   Y   Q   Q   T   P
ACACTGAGCTGCTCCCCTATCTCTGGGCATAGGAGTGTATCCTGGTACCAACAGACCCCA

  G   Q   G   L   Q   F   L   F   E   Y   F   S   E   T   Q   R   N   K   G   N
GGACAGGGCCTTCAGTTCCTCTTTGAATACTTCAGTGAGACACAGAGAAACAAAGGAAAC

  F   P   G   R   F   S   G   R   Q   F   S   N   S   R   S   E   M   N   V   S
TTCCCTGGTCGATTCTCAGGGCGCCAGTTCTCTAACTCTCGCTCTGAGATGAATGTGAGC

  T   L   E   L   G   D   S   A   L   Y   L   C   A   S   S   F   D   S   G   N
ACCTTGGAGCTGGGGGACTCGGCCCTTTATCTTTGCGCCAGCAGCTTCGACAGCGGGAAT

  S   P   L   H   F   G   N   G   T   R   L   T   V   T   E   D   L   N   K   V
TCACCCCTCCACTTTGGGAACGGGACCAGGCTCACTGTGACAGAGGACCTGAACAAGGTG

  F   P   P   E   V   A   V   F   E   P   S   E   A   E   I   S   H   T   Q   K
TTCCCACCCGAGGTCGCTGTGTTTGAGCCATCAGAAGCAGAGATCTCCCACACCCAAAAG

  A   T   L   V   C   L   A   T   G   F   F   P   D   H   V   E   L   S   W   W
GCCACACTGGTGTGCCTGGCCACAGGCTTCTTCCCTGACCACGTGGAGCTGAGCTGGTGG

  V   N   G   K   E   V   H   S   G   V   S   Q   D   P   Q   P   L   K   E   Q
GTGAATGGGAAGGAGGTGCACAGTGGGGTCAGCCAGGACCCGCAGCCCCTCAAGGAGCAG

  P   A   L   N   D   S   R   Y   S   L   S   S   R   L   R   V   S   A   T   F
CCCGCCCTCAATGACTCCAGATACAGCCTGAGCAGCCGCCTGAGGGTCTCGGCCACCTTC

  W   Q   N   P   R   N   H   F   R   C   Q   V   Q   F   Y   G   L   S   E   N
TGGCAGAACCCCCGCAACCACTTCCGCTGTCAAGTCCAGTTCTACGGGCTCTCGGAGAAT

  D   E   W   T   Q   D   R   A   K   P   V   T   Q   I   V   S   A   E   A   W
GACGAGTGGACCCAGGATAGGGCCAAACCTGTCACCCAGATCGTCAGCGCCGAGGCCTGG

    <TCR beta linker c-fos>
  G   R   A   D   P   G   G   L   T   D   T   L   Q   A   E   T   D   Q   L   E
GGTAGAGCAGACCCCGGGGGTCTGACTGATACACTCCAAGCGGAGACAGATCAACTTGAA

  D   K   K   S   A   L   Q   T   E   I   A   N   L   L   K   E   K   E   K   L
GACAAGAAGTCTGCGTTGCAGACCGAGATTGCCAATCTACTGAAAGAGAAGGAAAAACTA

  E   F   I   L   A   A   Y   *
GAGTTCATCCTGGCAGCTTACTAG
```

EP 1 066 380 B1

Figure 14

TCR alfa>

```
  M   K   Q   E   V   T   Q   I   P   A   A   L   S   V   P   E   G   E   N   L
ATGAAACAAGAAGTTACACAGATTCCTGCAGCTCTGAGTGTCCCAGAAGGAGAAAACTTG

  V   L   N   C   S   F   T   D   S   A   I   Y   N   L   Q   W   F   R   Q   D
GTTCTCAACTGCAGTTTCACTGATAGCGCTATTTACAACCTCCAGTGGTTTAGGCAGGAC

  P   G   K   G   L   T   S   L   L   L   I   Q   S   S   Q   R   E   Q   T   S
CCTGGGAAAGGTCTCACATCTCTGTTGCTTATTCAGTCAAGTCAGAGAGAGCAAACAAGT

  G   R   L   N   A   S   L   D   K   S   S   G   R   S   T   L   Y   I   A   A
GGAAGACTTAATGCCTCGCTGGATAAATCATCAGGACGTAGTACTTTATACATTGCAGCT

  S   Q   P   G   D   S   A   T   Y   L   C   A   V   T   N   F   N   K   F   Y
TCTCAGCCTGGTGACTCAGCCACCTACCTCTGTGCTGTGACCAACTTCAACAAATTTTAC

  F   G   S   G   T   K   L   N   V   K   P   N   I   Q   N   P   D   P   A   V
TTTGGATCTGGGACCAAACTCAATGTAAAACCAAATATCCAGAACCCTGACCCTGCCGTG

  Y   Q   L   R   D   S   K   S   S   D   K   S   V   C   L   F   T   D   F   D
TACCAGCTGAGAGACTCTAAATCCAGTGACAAGTCTGTCTGCCTATTCACCGATTTTGAT

  S   Q   T   N   V   S   Q   S   K   D   S   D   V   Y   I   T   D   K   T   V
TCTCAAACAAATGTGTCACAAAGTAAGGATTCTGATGTGTATATCACAGACAAAACTGTG

  L   D   M   R   S   M   D   F   K   S   N   S   A   V   A   W   S   N   K   S
CTAGACATGAGGTCTATGGACTTCAAGAGCAACAGTGCTGTGGCCTGGAGCAACAAATCT

  D   F   A   C   A   N   A   F   N   N   S   I   I   P   E   D   T   F   F   P
GACTTTGCATGTGCAAACGCCTTCAACAACAGCATTATTCCAGAAGACACCTTCTTCCCC
```

<TCR alfa linker c-jun>
```
  S   P   E   S   S   P   G   G   R   I   A   R   L   E   E   K   V   K   T   L
AGCCCAGAAAGTTCCcccgggGGTAGAATCGCCCGGCTGGAGGAAAAAGTGAAAACCTTG

  K   A   Q   N   S   E   L   A   S   T   A   N   M   L   R   E   Q   V   A   Q
AAAGCTCAGAACTCGGAGCTGGCGTCCACGGCCAACATGCTCAGGGAACAGGTGGCACAG

  L   K   Q   K   V   M   N   Y   *
CTTAAACAGAAAGTCATGAACTACTAG
```

46

Figure 15

TCR beta>

M K A G V T Q T P R Y L I K T R G Q Q V
ATGAAAGCTGGAGTTACTCAAACTCCAAGATATCTGATCAAAACGAGAGGACAGCAAGTG

T L S C S P I S G H R S V S W Y Q Q T P
ACACTGAGCTGCTCCCCTATCTCTGGGCATAGGAGTGTATCCTGGTACCAACAGACCCCA

G Q G L Q F L F E Y F S E T Q R N K G N
GGACAGGGCCTTCAGTTCCTCTTTGAATACTTCAGTGAGACACAGAGAAACAAAGGAAAC

F P G R F S G R Q F S N S R S E M N V S
TTCCCTGGTCGATTCTCAGGGCGCCAGTTCTCTAACTCTCGCTCTGAGATGAATGTGAGC

T L E L G D S A L Y L C A S S F D S G N
ACCTTGGAGCTGGGGGACTCGGCCCTTTATCTTTGCGCCAGCAGCTTCGACAGCGGGAAT

S P L H F G N G T R L T V T E D L N K V
TCACCCCTCCACTTTGGGAACGGGACCAGGCTCACTGTGACAGAGGACCTGAACAAGGTG

F P P E V A V F E P S E A E I S H T Q K
TTCCCACCCGAGGTCGCTGTGTTTGAGCCATCAGAAGCAGAGATCTCCCACACCCAAAAG

A T L V C L A T G F F P D H V E L S W W
GCCACACTGGTGTGCCTGGCCACAGGCTTCTTCCCTGACCACGTGGAGCTGAGCTGGTGG

V N G K E V H S G V S Q D P Q P L K E Q
GTGAATGGGAAGGAGGTGCACAGTGGGGTCAGCCAGGACCCGCAGCCCCTCAAGGAGCAG

P A L N D S R Y S L S S R L R V S A T F
CCCGCCCTCAATGACTCCAGATACAGCCTGAGCAGCCGCCTGAGGGTCTCGGCCACCTTC

W Q N P R N H F R C Q V Q F Y G L S E N
TGGCAGAACCCCCGCAACCACTTCCGCTGTCAAGTCCAGTTCTACGGGCTCTCGGAGAAT

D E W T Q D R A K P V T Q I V S A E A W
GACGAGTGGACCCAGGATAGGGCCAAACCTGTCACCCAGATCGTCAGCGCCGAGGCCTGG

<TCR beta linker c-fos>

G R A D P G G L T D T L Q A E T D Q L E
GGTAGAGCAGACcccgggGGTCTGACTGATACACTCCAAGCGGAGACAGATCAACTTGAA

D K K S A L Q T E I A N L L K E K E K L
GACAAGAAGTCTGCGTTGCAGACCGAGATTGCCAATCTACTGAAAGAGAAGGAAAAACTA

E F I L A A Y *
GAGTTCATCCTGGCAGCTTACTAG

47

Figure 16

TCR alfa>

```
 M   Q   K   E   V   E   Q   N   S   G   P   L   S.  V   P   E   G   A   I   A
atgCAGAAGGAAGTGGAGCAGAACTCTGGACCCCTCAGTGTTCCAGAGGGAGCCATTGCC

 S   L   N   C   T   Y   S   D   R   G   S   Q   S   F   F   W   Y   R   Q   Y
TCTCTCAACTGCACTTACAGTGACCGAGGTTCCCAGTCCTTCTTCTGGTACAGACAATAT

 S   G   K   S   P   E   L   I   M   S   I   Y   S   N   G   D   K   E   D   G
TCTGGGAAAAGCCCTGAGTTGATAATGTCCATATACTCCAATGGTGACAAAGAAGATGGA

 R   F   T   A   Q   L   N   K   A   S   Q   Y   V   S   L   L   I   R   D   S
AGGTTTACAGCACAGCTCAATAAAGCCAGCCAGTATGTTTCTCTGCTCATCAGAGACTCC

 Q   P   S   D   S   A   T   Y   L   C   A   V   T   T   D   S   W   G   K   L
CAGCCCAGTGATTCAGCCACCTACCTCTGTGCCGTTACAACTGACAGCTGGGGGAAATTG

 Q   F   G   A   G   T   Q   V   V   V   T   P   D   I   Q   N   P   D   P   A
CAGTTTGGAGCAGGGACCCAGGTTGTGGTCACCCCAGATATCCAGAACCCTGACCCTGCC

 V   Y   Q   L   R   D   S   K   S   S   D   K   S   V   C   L   F   T   D   F
GTGTACCAGCTGAGAGACTCTAAATCCAGTGACAAGTCTGTCTGCCTATTCACCGATTTT

 D   S   Q   T   N   V   S   Q   S   K   D   S   D   V   Y   I   T   D   K   T
GATTCTCAAACAAATGTGTCACAAAGTAAGGATTCTGATGTGTATATCACAGACAAAACT

 V   L   D   M   R   S   M   D   F   K   S   N   S   A   V   A   W   S   N   K
GTGCTAGACATGAGGTCTATGGACTTCAAGAGCAACAGTGCTGTGGCCTGGAGCAACAAA

 S   D   F   A   C   A   N   A   F   N   N   S   I   I   P   E   D   T   F   F
TCTGACTTTGCATGTGCAAACGCCTTCAACAACAGCATTATTCCAGAAGACACCTTCTTC
```

<TCR alfa linker c-jun>

```
 P   S   P   E   S   S   P   G   G   R   I   A   R   L   E   E   K   V   K   T
CCCAGCCCAGAAAGTTCCcccgggGGTAGAATCGCCCGGCTGGAGGAAAAAGTGAAAACC

 L   K   A   Q   N   S   E   L   A   S   T   A   N   M   L   R   E   Q   V   A
TTGAAAGCTCAGAACTCGGAGCTGGCGTCCACGGCCAACATGCTCAGGGAACAGGTGGCA

 Q   L   K   Q   K   V   M   N   Y   *
CAGCTTAAACAGAAAGTCATGAACTACTAG
```

Figure 17

```
    TCR beta>
    M   N   A   G   V   T   Q   T   P   K   F   Q   V   L   K   T   G   Q   S   M
    atgAACGCTGGTGTCACTCAGACCCCAAAATTCCAGGTCCTGAAGACAGGACAGAGCATG

    T   L   Q   C   A   Q   D   M   N   H   E   Y   M   S   W   Y   R   Q   D   P
    ACACTGCAGTGTGCCCAGGATATGAACCATGAATACATGTCCTGGTATCGACAAGACCCA

    G   M   G   L   R   L   I   H   Y   S   V   G   A   G   I   T   D   Q   G   E
    GGCATGGGGCTGAGGCTGATTCATTACTCAGTTGGTGCTGGTATCACTGACCAAGGAGAA

    V   P   N   G   Y   N   V   S   R   S   T   T   E   D   F   P   L   R   L   L
    GTCCCCAATGGCTACAATGTCTCCAGATCAACCACAGAGGATTTCCCGCTCAGGCTGCTG

    S   A   A   P   S   Q   T   S   V   Y   F   C   A   S   R   P   G   L   A   G
    TCGGCTGCTCCCTCCCAGACATCTGTGTACTTCTGTGCCAGCAGGCCGGGACTAGCGGGA

    G   R   P   E   Q   Y   F   G   P   G   T   R   L   T   V   T   E   D   L   K
    GGGCGACCAGAGCAGTACTTCGGGCCGGGCACCAGGCTCACGGTCACAGAGGACCTGAAA

    N   V   F   P   P   E   V   A   V   F   E   P   S   E   A   E   I   S   H   T
    AACGTGTTCCCACCCGAGGTCGCTGTGTTTGAGCCATCAGAAGCAGAGATCTCCCACACC

    Q   K   A   T   L   V   C   L   A   T   G   F   Y   P   D   H   V   E   L   S
    CAAAAGGCCACACTGGTGTGCCTGGCCACAGGCTTCTACCCCGACCACGTGGAGCTGAGC

    W   W   V   N   G   K   E   V   H   S   G   V   S   T   D   P   Q   P   L   K
    TGGTGGGTGAATGGGAAGGAGGTGCACAGTGGGGTCAGCACAGACCCGCAGCCCCTCAAG

    E   Q   P   A   L   N   D   S   R   Y   A   L   S   S   R   L   R   V   S   A
    GAGCAGCCCGCCCTCAATGACTCCAGATACgctCTGAGCAGCCGCCTGAGGGTCTCGGCC

    T   F   W   Q   N   P   R   N   H   F   R   C   Q   V   Q   F   Y   G   L   S
    ACCTTCTGGCAGAACCCCCGCAACCACTTCCGCTGTCAAGTCCAGTTCTACGGGCTCTCG

    E   N   D   E   W   T   Q   D   R   A   K   P   V   T   Q   I   V   S   A   E
    GAGAATGACGAGTGGACCCAGGATAGGGCCAAACCTGTCACCCAGATCGTCAGCGCCGAG

        <TCR beta linker c-fos>
    A   W   G   R   A   D   P   G   G   L   T   D   T   L   Q   A   E   T   D   Q
    GCCTGGGGTAGAGCAGACcccgggGGTCTGACTGATACACTCCAAGCCGAGACAGATCAA
```

Continued ........

EP 1 066 380 B1

Figure 17 (continued)

```
L   E   D   K   K   S   A   L   Q   T   E   I   A   N   L   L   K   E   K   E
CTTGAAGACAAGAAGTCTGCGTTGCAGACCGAGATTGCCAATCTACTGAAAGAGAAGGAA
```

```
                              linker  Biotinylation tag>
K   L   E   F   I   L   A   A   Y   G   S   G   G   G   L   N   D   I   F   E
AAACTAGAGTTCATCCTGGCAGCTTAOggatccGGTGGTGGTCTGAACGATATTTTTGAA
```

```
A   Q   K   I   E   W   H   *
GCTCAGAAAATCGAATGGCATTAAGCTT
```

Figure 18

TCR alfa>

M  Q  Q  K  N  D  D  Q  Q  V  K  Q  N  S  P  S  L  S  V  Q
atgCAACAGAAGAATGATGACCAGCAAGTTAAGCAAAATTCACCATCCCTGAGCGTCCAG

E  G  R  I  S  I  L  N  C  D  Y  T  N  S  M  F  D  Y  F  L
GAAGGAAGAATTTCTATTCTGAACTGTGACTATACTAACAGCATGTTTGATTATTTCCTA

W  Y  K  K  Y  P  A  E  G  P  T  F  L  I  S  I  S  S  I  K
TGGTACAAAAAATACCCTGCTGAAGGTCCTACATTCCTGATATCTATAAGTTCCATTAAG

D  K  N  E  D  G  R  F  T  V  F  L  N  K  S  A  K  H  L  S
GATAAAAATGAAGATGGAAGATTCACTGTCTTCTTAAACAAAAGTGCCAAGCACCTCTCT

L  H  I  V  P  S  Q  P  G  D  S  A  V  Y  F  C  A  A  M  E
CTGCACATTGTGCCCTCCCAGCCTGGAGACTCTGCAGTGTACTTCTGTGCAGCAATGGAG

G  A  Q  K  L  V  F  G  Q  G  T  R  L  T  I  N  P  N  I  Q
GGAGCCCAGAAGCTGGTATTTGGCCAAGGAACCAGGCTGACTATCAACCCAAATATCCAG

N  P  D  P  A  V  Y  Q  L  R  D  S  K  S  S  D  K  S  V  C
AACCCTGACCCTGCCGTGTACCAGCTGAGAGACTCTAAATCCAGTGACAAGTCTGTCTGC

L  F  T  D  F  D  S  Q  T  N  V  S  Q  S  K  D  S  D  V  Y
CTATTCACCGATTTTGATTCTCAAACAAATGTGTCACAAAGTAAGGATTCTGATGTGTAT

I  T  D  K  T  V  L  D  M  R  S  M  D  F  K  S  N  S  A  V
ATCACAGACAAAACTGTGCTAGACATGAGGTCTATGGACTTCAAGAGCAACAGTGCTGTG

A  W  S  N  K  S  D  F  A  C  A  N  A  F  N  N  S  I  I  P
GCCTGGAGCAACAAATCTGACTTTGCATGTGCAAACGCCTTCAACAACAGCATTATTCCA

<TCR alfa linker c-jun>

E  D  T  F  F  P  S  P  E  S  S  P  G  G  R  I  A  R  L  E
GAAGACACCTTCTTCCCCAGCCCAGAAAGTTCCcccgggGGTAGAATCGCCCGGCTGGAG

E  K  V  K  T  L  K  A  Q  N  S  E  L  A  S  T  A  N  M  L
GAAAAAGTGAAAACCTTGAAAGCTCAGAACTCGGAGCTGGCGTCCACGGCCAACATGCTC

R  E  Q  V  A  Q  L  K  Q  K  V  M  N  Y  *
AGGGAACAGGTGGCACAGCTTAAACAGAAAGTCATGAACTACTAG

Figure 19

TCR beta>

M N A G V T Q T P K F Q V L K T G Q S M
atgAACGCTGGTGTCACTCAGACCCCAAAAATTCCAGGTCCTGAAGACAGGACAGAGCATG

T L Q C A Q D M N H E Y M S W Y R Q D P
ACACTGCAGTGTGCCCAGGATATGAACCATGAATACATGTCCTGGTATCGACAAGACCCA

G M G L R L I H Y S V G A G I T D Q G E
GGCATGGGGCTGAGGCTGATTCATTACTCAGTTGGTGCTGGTATCACTGACCAAGGAGAA

V P N G Y N V S R S T T E D F P L R L L
GTCCCCAATGGCTACAATGTCTCCAGATCAACCACAGAGGATTTCCCGCTCAGGCTGCTG

S A A P S Q T S V Y F C A S S Y P G G G
TCGGCTGCTCCCTCCCAGACATCTGTGTACTTCTGTGCCAGCAGTTACCaGGaGGGGGGG

F Y E Q Y F G P G T R L T V T E D L K N
TTTTACGAGCAGTACTTCGGGCCCGGGCACCAGGCTCACGGTCACAGAGGACCTGAAAAAC

V F P P E V A V F E P S E A E I S H T Q
GTGTTCCCACCCGAGGTCGCTGTGTGTTTGAGCCATCAGAAGCAGAGATCTCCCACACCCAA

K A T L V C L A T G F Y P D H V E L S W
AAGGCCACACTGGTGTGCCTGGCCACAGGCTTCTACCCCGACCACGTGGAGCTGAGCTGG

W V N G K E V H S G V S T D P Q P L K E
TGGGTGAATGGGAAGGAGGTGCACAGTGGGGTCAGCACAGACCCCGCAGCCCCTCAAGGAG

Q P A L N D S R Y **A** L S S R L R V S A T
CAGCCCGCCCTCAATGACTCCAGATAC**gct**CTGAGCAGCCGCCTGAGGGTCTCGGCCACC

F W Q D P R N H F R C Q V Q F Y G L S E
TTCTGGCAGgACCCCCGCAACCACTTCCGCTGTCAAGTCCAGTTCTACGGGCTCTCGGAG

N D E W T Q D R A K P V T Q I V S A E A
AATGACGAGTGGACCCAGGATAGGGCCAAACCCGTCACCCAGATCGTCAGCGCCGAGGCC

Continued......

Figure 19 (continued)

<TCR beta linker c-fos>

W  G  R  A  D  P  G  G  L  T  D  T  L  Q  A  E  T  D  Q  L

TGGGGTAGAGCAGACcccgggGGTCTGACTGATACACTCCAAGCGGAGACAGATCAACTT


E  D  K  K  S  A  L  Q  T  E  I  A  N  L  L  K  E  K  E  K

GAAGACAAGAAGTCTGCGTTGCAGACCGAGATTGCCAATCTACTGAAAGAGAAGGAAAAA


linker Biotinylation tag>

L  E  F  I  L  A  A  Y  G  S  G  G  L  N  D  I  F  E  A

CTAGAGTTCATCCTGGCAGCTTACggatccGGTGGTGGTCTGAACGATATTTTTGAAGCT


Q  K  I  E  W  H  *

CAGAAAATCGAATGGCATTAAGCTT

53

Figure 20

```
TCR beta>
M N A G V T Q T P K F Q V L K T G Q S M
atgAACGCTGGTGTCACTCAGACCCCAAAATTCCAGGTCCTGAAGACAGGACAGAGCATG


T L Q C A Q D M N H E Y M S W Y R Q D P
ACACTGCAGTGTGCCCAGGATATGAACCATGAATACATGTCCTGGTATCGACAAGACCCA


G M G L R L I H Y S V G A G I T D Q G E
GGCATGGGGCTGAGGCTGATTCATTACTCAGTTGGTGCTGGTATCACTGACCAAGGAGAA


V P N G Y N V S R S T T E D F P L R L L
GTCCCCAATGGCTACAATGTCTCCAGATCAACCACAGAGGATTTCCCGCTCAGGCTGCTG


S A A P S Q T S V Y F C A S R P G L A G
TCGGCTGCTCCCTCCCAGACATCTGTGTACTTCTGTGCCAGCAGGCCCGGGACTAGCGGGA


G R P E Q Y F G P G T R L T V T E D L K
GGGCGACCAGAGCAGTACTTCGGGCCCGGCACCAGGCTCACGGTCACAGAGGACCTGAAA


N V F P P E V A V F E P S E A E I S H T
AACGTGTTCCCACCCGAGGTCGCTGTGTTTGAGCCATCAGAAGCAGAGATCTCCCACACC


Q K A T L V C L A T G F Y P D H V E L S
CAAAAGGCCACACTGGTGTGCCTGGCCACAGGCTTCTACCCCGACCACGTGGAGCTGAGC


W W V N G K E V H S G V S T D P Q P L K
TGGTGGGTGAATGGGAAGGAGGTGCACAGTGGGGTCAGCACAGACCCGCAGCCCCTCAAG


E Q P A L N D S R Y A L S S R L R V S A
GAGCAGCCCGCCCTCAATGACTCCAGATACgctCTGAGCAGCCGCCTGAGGGTCTCGGCC


T F W Q D P R N H F R C Q V Q F Y G L S
ACCTTCTGGCAGgACCCCCGCAACCACTTCCGCTGTCAAGTCCAGTTCTACGGGCTCTCG


E N D E W T Q D R A K P V T Q I V S A E
GAGAATGACGAGTGGACCCAGGATAGGGCCAAACCTGTCACCCAGATCGTCAGCGCCGAG
```

*Continued.....*

Figure 20 (continued)

<TCR beta linker c-fos>

A  W  G  R  A  D  P  G  G  L  T  D  T  L  Q  A  E  T  D  Q
GCCTGGGGTAGAGCAGACcccgggGGTCTGACTGATACACTCCAAGCCGAGACAGATCAA

L  E  D  K  K  S  A  L  Q  T  E  I  A  N  L  L  K  E  K  E
CTTGAAGACAAGAAGTCTGCGTTGCAGACCGAGATTGCCAATCTACTGAAAGAGAAGGAA

linker Biotinylation tag>

K  L  E  F  I  L  A  A  Y  G  S  G  G  G  L  N  D  I  F  E
AAACTAGAGTTCATCCTGGCAGCTTACggatccGGTGGTGGTCTGAACGATATTTTTGAA

A  Q  K  I  E  W  H  *
GCTCAGAAAATCGAATGGCATTAAGCTT

55

Figure 21

```
    Linker<-> fos
      P    G    G    L    T    D    T    L    Q    A    E    T    D    Q
5'- ccc ggg GGT CTG ACT GAT ACA CTC CAA GCG GAG ACA GAT CAA
    Xma I


    L    E    D    K    K    S    A    L    Q    T    E    I    A    N    L
  CTT GAA GAC AAG AAG TCT GCG TTG CAG ACC GAG ATT GCC AAT CTA


                                                      <-lin
    L    K    E    K    E    K    L    E    F    I    L    A    A    Y    G
  CTG AAA GAG AAG GAA AAA CTA GAG TTC ATC CTG GCA GCT TAC gga
                                                           Bam


Ker-> <- biotinylation tag
    S    G    G    G    L    N    D    I    F    E    A    Q    K    I    E
  tcc GGT GGT GGT CTG AAC GAT ATT TTT GAA GCT CAG AAA ATC GAA
  HI


    W    H    *
  TGG CAT TAA GCT T -3'
          Hind III
```

Figure 22

# A

**Reverse primer:**

5'-ACACAC <u>GGA TCC</u> GTA AGC TGC GAC GAT GAA CTC GAT TTT CTT-
3'

Bam HI

Figure 23

Figure 24.

Figure 25.

EP 1 066 380 B1

FIGURE 26 ai

EP 1 066 380 B1

60

Figure 26 b1

Figure 2b

Figure 27

20.65

15.67

a.

b.

Figure 28

Figure 29

FIGURE 30

Figure 31

FIGURE 32

TCR alfa>

M Q L L E Q S P Q F L S I Q E G E N L T
ATGCAaCTaCTaGAaCAaAGtCCTCAGTTTCTAAGCATCCAAGAGGGAGAAAATCTCACT

V Y C N S S S V F S S L Q W Y R Q E P G
GTGTACTGCAACTCCTCAAGTGTTTTTTCCAGCTTACAATGGTACAGACAGGAGCCTGGG

E G P V L L V T V V T G G E V K K L K R
GAAGGTCCTGTCCTCCTGGTGACAGTAGTTACGGGTGGAGAAGTGAAGAAGCTGAAGAGA

L T F Q F G D A R K D S S L H I T A A Q
CTAACCTTTCAGTTTGGTGATGCAAGAAAGGACAGTTCTCTCCACATCACTGCGGCCCAG

P G D T G L Y L C A G A G S Q G N L I F
CCTGGTGATACAGGCCTCTACCTCTGTGCAGGAGCGGGAAGCCAAGGAAATCTCATCTTT

G K G T K L S V K P N I Q N P D P A V Y
GGAAAAGGCACTAAACTCTCTGTTAAACCAAATATCCAGAACCCTGACCCTGCCGTGTAC

Q L R D S K S S D K S V C L F T D F D S
CAGCTGAGAGACTCTAAATCCAGTGACAAGTCTGTCTGCCTATTCACCGATTTTGATTCT

Q T N V S Q S K D S D V Y I T D K T V L
CAAACAAATGTGTCACAAAGTAAGGATTCTGATGTGTATATCACAGACAAAACTGTGCTA

D M R S M D F K S N S A V A W S N K S ·D
GACATGAGGTCTATGGACTTCAAGAGCAACAGTGCTGTGGCCTGGAGCAACAAATCTGAC

F A C A N A F N N S I I P E D T F F P S
TTTGCATGTGCAAACGCCTTCAACAACAGCATTATTCCAGAAGACACCTTCTTCCCCAGC

<TCR alfa linker c-jun>
P E S S P G G R I A R L E E K V K T L K
CCAGAAAGTTCCcccgggGGTAGAATCGCCCGGCTGGAGGAAAAAGTGAAAACCTTGAAA

A Q N S E L A S T A N M L R E Q V A Q L
GCTCAGAACTCGGAGCTGGCGTCCACGGCCAACATGCTCAGGGAACAGGTGGCACAGCTT

K Q K V M N Y *
AAACAGAAAGTCATGAACTACTAG

## FIGURE 33

```
TCR beta>
 M  V  D  G  G  I  T  Q  S  P  K  Y  L  F  R  K  E  G  Q  N
ATGGTGGATGGTGGAATCACTCAGTCCCCAAAGTACCTGTTCAGAAAGGAAGGACAGAAT

 V  T  L  S  C  E  Q  N  L  N  H  D  A  M  Y  W  Y  R  Q  D
GTGACCCTGAGTTGTGAACAGAATTTGAACCACGATGCCATGTACTGGTACCGACAGGAC

 P  G  Q  G  L  R  L  I  Y  Y  S  Q  I  V  N  D  F  Q  K  G
CCAGGGCAAGGGCTGAGATTGATCTACTACTCACAGATAGTAAATGACTTTCAGAAAGGA

 D  I  A  E  G  Y  S  V  S  R  E  K  K  E  S  F  P  L  T  V
GATATAGCTGAAGGGTACAGCGTCTCTCGGGAGAAGAAGGAATCCTTTCCTCTCACTGTG

 T  S  A  Q  K  N  P  T  A  F  Y  L  C  A  S  S  R  S  S
ACATCGGCCCAAAAGAACCCGACAGCTTTCTATCTCTGTGCCAGTAGTTCGAGGAGCTCC

 Y  E  Q  Y  F  G  P  G  T  R  L  T  V  T  E  D  L  K  N  V
TACGAGCAGTACTTCGGGCCGGGCACCAGGCTCACGGTCACAGAGGACCTGAAAAACGTT

 F  P  P  E  V  A  V  F  E  P  S  E  A  E  I  S  H  T  Q  K
TTCCCACCCGAGGTCGCTGTGTTTGAACCATCAGAAGCAGAGATCTCCCACACCCAAAAG

 A  T  L  V  C  L  A  T  G  F  Y  P  D  H  V  E  L  S  W  W
GCCACACTGGTGTGCCTGGCCACAGGCTTCTACCCCGACCACGTGGAGCTGAGCTGGTGG

 V  N  G  K  E  V  H  S  G  V  S  T  D  P  Q  P  L  K  E  Q
GTGAATGGGAAGGAGGTGCACAGTGGGGTCAGCACAGACCCGCAGCCCCTCAAGGAGCAG

 P  A  L  N  D  S  R  Y  S  L  S  S  R  L  R  V  S  A  T  F
CCCGCCCTCAATGACTCCAGATACTCCCTGAGCAGCCGCCTGAGGGTCTCGGCCACCTTC

 W  Q  N  P  R  N  H  F  R  C  Q  V  Q  F  Y  G  L  S  E  N
TGGCAGAACCCCCGCAACCACTTCCGCTGTCAAGTCCAGTTCTACGGGCTCTCGGAGAAT
```

```
  D   E   W   T   Q   D   R   A   K   P   V   T   Q   I   V   S   A   E   A   W
GACGAGTGGACCCAGGATAGGGCCAAACCTGTCACCCAGATCGTCAGCGCCGAGGCCTGG
<TCR beta linker c-fos>
  G   R   A   D   P   G   G   L   T   D   T   L   Q   A   E   T   D   Q   L   E
GGTAGAGCAGACcccgggGGTCTGACTGATACACTCCAAGCGGAGACAGATCAACTTGAA


  D   K   K   S   A   L   Q   T   E   I   A   N   L   L   K   E   K   E   K   L
GACAAGAAGTCTGCGTTGCAGACCGAGATTGCCAATCTACTGAAAGAGAAGGAAAAACTA


  E   F   I   L   A   A   Y   *
GAGTTCATCCTGGCAGCTTACTAG
```

## FIGURE 34

TCR beta>

```
   M   V   D   G   G   I   T   Q   S   P   K   Y   L   F   R   K   E   G   Q   N
ATGGTGGATGGTGGAATCACTCAGTCCCCAAAGTACCTGTTCAGAAAGGAAGGACAGAAT


   V   T   L   S   C   E   Q   N   L   N   H   D   A   M   Y   W   Y   R   Q   D
GTGACCCTGAGTTGTGAACAGAATTTGAACCACGATGCCATGTACTGGTACCGACAGGAC


   P   G   Q   G   L   R   L   I   Y   Y   S   Q   I · V   N   D   F   Q   K   G
CCAGGGCAAGGGCTGAGATTGATCTACTACTCACAGATAGTAAATGACTTTCAGAAAGGA


   D   I   A   E   G   Y   S   V   S   R   E   K   K   E   S   F   P   L   T   V
GATATAGCTGAAGGGTACAGCGTCTCTCGGGAGAAGAAGGAATCCTTTCCTCTCACTGTG


   T   S   A   Q   K   N   P   T   A   F   Y   L   C   A   S   S   S   R   S   S
ACATCGGCCCAAAAGAACCCGACAGCTTTCTATCTCTGTGCCAGTAGTTCGAGGAGCTCC


   Y   E · Q   Y   F   G   P   G   T   R   L   T   V   T   E   D   L   K   N   V
TACGAGCAGTACTTCGGGCCGGGCACCAGGCTCACGGTCACAGAGGACCTGAAAAACGTT


   F   P   P   E   V   A   V   F   E   P   S   E   A   E   I   S   H   T   Q   K
TTCCCACCCGAGGTCGCTGTGTTTGAACCATCAGAAGCAGAGATCTCCCACACCCAAAAG


   A   T   L   V   C   L   A   T   G   F   Y   P   D   H   V   E   L   S   W   W
GCCACACTGGTGTGCCTGGCCACAGGCTTCTACCCCGACCACGTGGAGCTGAGCTGGTGG


   V   N   G   K   E   V   H   S   G   V   S   T   D   P   Q   P   L   K   E   Q
GTGAATGGGAAGGAGGTGCACAGTGGGGTCAGCACAGACCCGCAGCCCCTCAAGGAGCAG


   P   A   L   N   D   S   R   Y   S̲   L   S   S   R   L   R   V   S   A   T   F
CCCGCCCTCAATGACTCCAGATACTC̲CCTGAGCAGCCGCCTGAGGGTCTCGGCCACCTTC


   W   Q   N   P   R   N   H   F   R   C   Q   V   Q   F   Y   G   L   S   E   N
TGGCAGAACCCCCGCAACCACTTCCGCTGTCAAGTCCAGTTCTACGGGCTCTCGGAGAAT
```

71

```
D   E   W   T   Q   D   R   A   K   P   V   T   Q   I   V   S   A   E   A   W
GACGAGTGGACCCAGGATAGGGCCAAACCTGTCACCCAGATCGTCAGCGCCGAGGCCTGG
```

<TCR beta <u>linker</u> c-fos>
```
G   R   A   D   P   G   G   L   T   D   T   L   Q   A   E   T   D   Q   L   E
GGTAGAGCAGACcccgggGGTCTGACTGATACACTCCAAGCGGAGACAGATCAACTTGAA
```

```
D   K   K   S   A   L   Q   T   E   I   A   N   L   L   K   E   K   E   K   L
GACAAGAAGTCTGCGTTGCAGACCGAGATTGCCAATCTACTGAAAGAGAAGGAAAAACTA
```

<u>linker</u> Biotinylation tag>
```
E   F   I   L   A   A   Y   G   S   G   G   G   L   N   D   I   F   E   A   Q
GAGTTCATCCTGGCAGCTTACggatccGGTGGTGGTCTGAACGATATTTTTGAAGCTCAG
```

```
K   I   E   W   H   *
AAAATCGAATGGCATTAA
```

FIGURE 35

*Vα*

*Vβ*

*TCR*

*Cα*

*Cβ*

*linker*

*G*  *G*

*G*  *G*

*jun L-zip*

*fos L-zip*

*G*

*G*

*Biotinylation tag*

*Biotin*

FIGURE 36

## FIGURE 38

```
  TCR alfa>
  M   Q   K   E   V   E   Q   N   S   G   P   L   S   V   P   E   G   A   I   A
  atgCAGAAGGAAGTGGAGCAGAACTCTGGACCCCTCAGTGTTCCAGAGGGAGCCATTGCC


  S   L   N   C   T   Y   S   D   R   G   S   Q   S   F   F   W   Y   R   Q   Y
  TCTCTCAACTGCACTTACAGTGACCGAGGTTCCCAGTCCTTCTTCTGGTACAGACAATAT


  S   G   K   S   P   E   L   I   M   S   I   Y   S   N   G   D   K   E   D   G
  TCTGGGAAAAGCCCTGAGTTGATAATGTCCATATACTCCAATGGTGACAAAGAAGATGGA


  R   F   T   A   Q   L   N   K   A   S   Q   Y   V   S   L   L   I   R   D   S
  AGGTTTACAGCACAGCTCAATAAAGCCAGCCAGTATGTTTCTCTGCTCATCAGAGACTCC


  Q   P   S   D   S   A   T   Y   L   C   A   V   T   T   D   S   W   G   K   L
  CAGCCCAGTGATTCAGCCACCTACCTCTGTGCCGTTACAACTGACAGCTGGGGGAAATTG


  Q   F   G   A   G   T   Q   V   V   V   T   P   D   I   Q   N   P   D   P   A
  CAGTTTGGAGCAGGGACCCAGGTTGTGGTCACCCCAGATATCCAGAACCCTGACCCTGCC


  V   Y   Q   L   R   D   S   K   S   S   D   K   S   V   C   L   F   T   D   F
  GTGTACCAGCTGAGAGACTCTAAATCCAGTGACAAGTCTGTCTGCCTATTCACCGATTTT


  D   S   Q   T   N   V   S   Q   S   K   D   S   D   V   Y   I   T   D   K   T
  GATTCTCAAACAAATGTGTCACAAAGTAAGGATTCTGATGTGTATATCACAGACAAAACT


  V   L   D   M   R   S   M   D   F   K   S   N   S   A   V   A   W   S   N   K
  GTGCTAGACATGAGGTCTATGGACTTCAAGAGCAACAGTGCTGTGGCCTGGAGCAACAAA


  S   D   F   A   C   A   N   A   F   N   N   S   I   I   P   E   D   T   F   F
  TCTGACTTTGCATGTGCAAACGCCTTCAACAACAGCATTATTCCAGAAGACACCTTCTTC

          <TCR alfa linker c-jun>
  P   S   P   E   S   S   P   G   G   R   I   A   R   L   E   E   K   V   K   T
  CCCAGCCCAGAAAGTTCCcccgggGGTAGAATCGCCCGGCTGGAGGAAAAAGTGAAAACC


  L   K   A   Q   N   S   E   L   A   S   T   A   N   M   L   R   E   Q   V   A
  TTGAAAGCTCAGAACTCGGAGCTGGCGTCCACGGCCAACATGCTCAGGGAACAGGTGGCA


  Q   L   K   Q   K   V   M   N   Y   *
  CAGCTTAAACAGAAAGTCATGAACTACTAG
```

76

## FIGURE 39

```
TCR beta>
M   N   A   G   V   T   Q   T   P   K   F   Q   V   L   K   T   G   Q   S   M
atgAACGCTGGTGTCACTCAGACCCCAAAATTCCAGGTCCTGAAGACAGGACAGAGCATG


T   L   Q   C   A   Q   D   M   N   H   E   Y   M   S   W   Y   R   Q   D   P
ACACTGCAGTGTGCCCAGGATATGAACCATGAATACATGTCCTGGTATCGACAAGACCCA


G   M   G   L   R   L   I   H   Y   S   V   G   A   G   I   T   D   Q   G   E
GGCATGGGGCTGAGGCTGATTCATTACTCAGTTGGTGCTGGTATCACTGACCAAGGAGAA


V   P   N   G   Y   N   V   S   R   S   T   T   E   D   F   P   L   R   L   L
GTCCCCAATGGCTACAATGTCTCCAGATCAACCACAGAGGATTTCCCGCTCAGGCTGCTG


S   A   A   P   S   Q   T   S   V   Y   F   C   A   S   R   P   G   L   A   G
TCGGCTGCTCCCTCCCAGACATCTGTGTACTTCTGTGCCAGCAGGCCGGGACTAGCGGGA


G   R   P   E   Q   Y   F   G   P   G   T   R   L   T   V   T   E   D   L   K
GGGCGACCAGAGCAGTACTTCGGGCCGGGCACCAGGCTCACGGTCACAGAGGACCTGAAA


N   V   F   P   P   E   V   A   V   F   E   P   S   E   A   E   I   S   H   T
AACGTGTTCCCACCCGAGGTCGCTGTGTTTGAGCCATCAGAAGCAGAGATCTCCCACACC


Q   K   A   T   L   V   C   L   A   T   G   F   Y   P   D   H   V   E   L   S
CAAAAGGCCACACTGGTGTGCCTGGCCACAGGCTTCTACCCCGACCACGTGGAGCTGAGC


W   W   V   N   G   K   E   V   H   S   G   V   S   T   D   P   Q   P   L   K
TGGTGGGTGAATGGGAAGGAGGTGCACAGTGGGGTCAGCACAGACCCGCAGCCCCTCAAG


E   Q   P   A   L   N   D   S   R   Y   A   L   S   S   R   L   R   V   S   A
GAGCAGCCCGCCCTCAATGACTCCAGATACgctCTGAGCAGCCGCCTGAGGGTCTCGGCC


T   F   W   Q   N   P   R   N   H   F   R   C   Q   V   Q   F   Y   G   L   S
ACCTTCTGGCAGAACCCCCGCAACCACTTCCGCTGTCAAGTCCAGTTCTACGGGCTCTCG


E   N   D   E   W   T   Q   D   R   A   K   P   V   T   Q   I   V   S   A   E
GAGAATGACGAGTGGACCCAGGATAGGGCCAAACCTGTCACCCAGATCGTCAGCGCCGAG

        <TCR beta linker c-fos>
A   W   G   R   A   D   P   G   G   L   T   D   T   L   Q   A   E   T   D   Q
GCCTGGGGGTAGAGCAGACcccgggGGTCTGACTGATACACTCCAAGCGGAGACAGATCAA
```

```
L   E   D   K   K   S   A   L   Q   T   E   I   A   N   L   L   K   E   K   E
CTTGAAGACAAGAAGTCTGCGTTGCAGACCGAGATTGCCAATCTACTGAAAGAGAAGGAA
```

```
                                    linker Biotinylation tag>
K   L   E   F   I   L   A   A   Y   G   S   G   G   G   L   N   D   I   F   E
AAACTAGAGTTCATCCTGGCAGCTTACggatccGGTGGTGGTCTGAACGATATTTTTGAA
```

```
A   Q   K   I   E   W   H   *
GCTCAGAAAATCGAATGGCATTAAGCTT
```

## FIGURE 40

```
  TCR alfa>
  M   Q   Q   K   N   D   D   Q   Q   V   K   Q   N   S   P   S   L   S   V   Q
  atgCAACAGAAGAATGATGACCAGCAAGTTAAGCAAAATTCACCATCCCTGAGCGTCCAG


  E   G   R   I   S   I   L   N   C   D   Y   T   N   S   M   F   D   Y   F   L
  GAAGGAAGAATTTCTATTCTGAACTGTGACTATACTAACAGCATGTTTGATTATTTCCTA


  W   Y   K   K   Y   P   A   E   G   P   T   F   L   I   S   I   S   S   I   K
  TGGTACAAAAAATACCCTGCTGAAGGTCCTACATTCCTGATATCTATAAGTTCCATTAAG


  D   K   N   E   D   G   R   F   T   V   F   L   N   K   S   A   K   H   L   S
  GATAAAAATGAAGATGGAAGATTCACTGTCTTCTTAAACAAAAGTGCCAAGCACCTCTCT


  L   H   I   V   P   S   Q   P   G   D   S   A   V   Y   F   C   A   A   M   E
  CTGCACATTGTGCCCTCCCAGCCTGGAGACTCTGCAGTGTACTTCTGTGCAGCAATGGAG


  G   A   Q   K   L   V   F   G   Q   G   T   R   L   T   I   N   P   N   I   Q
  GGAGCCCAGAAGCTGGTATTTGGCCAAGGAACCAGGCTGACTATCAACCCAAATATCCAG


  N   P   D   P   A   V   Y   Q   L   R   D   S   K   S   S   D   K   S   V   C
  AACCCTGACCCTGCCGTGTACCAGCTGAGAGACTCTAAATCCAGTGACAAGTCTGTCTGC


  L   F   T   D   F   D   S   Q   T   N   V   S   Q   S   K   D   S   D   V   Y
  CTATTCACCGATTTTGATTCTCAAACAAATGTGTCACAAAGTAAGGATTCTGATGTGTAT


  I   T   D   K   T   V   L   D   M   R   S   M   D   F   K   S   N   S   A   V
  ATCACAGACAAAACTGTGCTAGACATGAGGTCTATGGACTTCAAGAGCAACAGTGCTGTG


  A   W   S   N   K   S   D   F   A   C   A   N   A   F   N   N   S   I   I   P
  GCCTGGAGCAACAAATCTGACTTTGCATGTGCAAACGCCTTCAACAACAGCATTATTCCA

                          <TCR alfa linker c-jun>
  E   D   T   F   F   P   S   P   E   S   S   P   G   G   R   I   A   R   L   E
  GAAGACACCTTCTTCCCCAGCCCAGAAAGTTCCcccgggGGTAGAATCGCCCGGCTGGAG


  E   K   V   K   T   L   K   A   Q   N   S   E   L   A   S   T   A   N   M   L
  GAAAAAGTGAAAACCTTGAAAGCTCAGAACTCGGAGCTGGCGTCCACGGCCAACATGCTC


  R   E   Q   V   A   Q   L   K   Q   K   V   M   N   Y   *
  AGGGAACAGGTGGCACAGCTTAAACAGAAAGTCATGAACTACTAG
```

## FIGURE 4J

```
    TCR beta>
    M   N   A   G   V   T   Q   T   P   K   F   Q   V   L   K   T   G   Q   S   M
    atgAACGCTGGTGTCACTCAGACCCCAAAATTCCAGGTCCTGAAGACAGGACAGAGCATG


    T   L   Q   C   A   Q   D   M   N   H   E   Y   M   S   W   Y   R   Q   D   P
    ACACTGCAGTGTGCCCAGGATATGAACCATGAATACATGTCCTGGTATCGACAAGACCCA


    G   M   G   L   R   L   I   H   Y   S   V   G   A   G   I   T   D   Q   G   E
    GGCATGGGGCTGAGGCTGATTCATTACTCAGTTGGTGCTGGTATCACTGACCAAGGAGAA


    V   P   N   G   Y   N   V   S   R   S   T   T   E   D   F   P   L   R   L   L
    GTCCCCAATGGCTACAATGTCTCCAGATCAACCACAGAGGATTTCCCGCTCAGGCTGCTG


    S   A   A   P   S   Q   T   S   V   Y   F   C   A   S   S   Y   P   G   G   G
    TCGGCTGCTCCCTCCCAGACATCTGTGTACTTCTGTGCCAGCAGTTACCaGGaGGGGGGG


    F   Y   E   Q   Y   F   G   P   G   T   R   L   T   V   T   E   D   L   K   N
    TTTTACGAGCAGTACTTCGGGCCGGGCACCAGGCTCACGGTCACAGAGGACCTGAAAAAC


    V   F   P   P   E   V   A   V   F   E   P   S   E   A   E   I   S   H   T   Q
    GTGTTCCCACCCGAGGTCGCTGTGTTTGAGCCATCAGAAGCAGAGATCTCCCACACCCAA


    K   A   T   L   V   C   L   A   T   G   F   Y   P   D   H   V   E   L   S   W
    AAGGCCACACTGGTGTGCCTGGCCACAGGCTTCTACCCCGACCACGTGGAGCTGAGCTGG


    W   V   N   G   K   E   V   H   S   G   V   S   T   D   P   Q   P   L   K   E
    TGGGTGAATGGGAAGGAGGTGCACAGTGGGGTCAGCACAGACCCGCAGCCCCTCAAGGAG


    Q   P   A   L   N   D   S   R   Y   A   L   S   S   R   L   R   V   S   A   T
    CAGCCCGCCCTCAATGACTCCAGATACgctCTGAGCAGCCGCCTGAGGGTCTCGGCCACC


    F   W   Q   D   P   R   N   H   F   R   C   Q   V   Q   F   Y   G   L   S   E
    TTCTGGCAGgACCCCCGCAACCACTTCCGCTGTCAAGTCCAGTTCTACGGGCTCTCGGAG


    N   D   E   W   T   Q   D   R   A   K   P   V   T   Q   I   V   S   A   E   A
    AATGACGAGTGGACCCAGGATAGGGCCAAACCCGTCACCCAGATCGTCAGCGCCGAGGCC

    <TCR beta linker c-fos>
    W   G   R   A   D   P   G   G   L   T   D   T   L   Q   A   E   T   D   Q   L
    TGGGGTAGAGCAGACcccgggGGTCTGACTGATACACTCCAAGCGGAGACAGATCAACTT
```

```
     E   D   K   K   S   A   L   Q   T   E   I   A   N   L   L   K   E   K   E   K
GAAGACAAGAAGTCTGCGTTGCAGACCGAGATTGCCAATCTACTGAAAGAGAAGGAAAAA

                                          linker Biotinylation tag>
     L   E   F   I   L   A   A   Y   G   S   G   G   G   L   N   D   I   F   E   A
CTAGAGTTCATCCTGGCAGCTTACggatccGGTGGTGGTCTGAACGATATTTTTGAAGCT


     Q   K   I   E   W   H   *
CAGAAAATCGAATGGCATTAAGCTT
```